(19) Europäisches Patentamt
European Patent Office
Office européen des brevets

(11) EP 3 960 201 A1

(12) EUROPEAN PATENT APPLICATION
published in accordance with Art. 153(4) EPC

(43) Date of publication:
**02.03.2022 Bulletin 2022/09**

(21) Application number: **20794938.9**

(22) Date of filing: **22.04.2020**

(51) International Patent Classification (IPC):
***A61K 45/00*** *(2006.01)* ***A61P 3/04*** *(2006.01)*
***A61P 3/10*** *(2006.01)* ***A61P 43/00*** *(2006.01)*
***A61K 9/10*** *(2006.01)* ***A23L 33/10*** *(2016.01)*
***A61K 47/14*** *(2017.01)* ***A61K 47/24*** *(2006.01)*
***A61K 47/26*** *(2006.01)* ***A61K 47/34*** *(2017.01)*
***A23L 2/52*** *(2006.01)* ***A61K 31/704*** *(2006.01)*

(52) Cooperative Patent Classification (CPC):
**A23L 2/52; A23L 33/10; A61K 9/10; A61K 31/704; A61K 45/00; A61K 47/14; A61K 47/24; A61K 47/26; A61K 47/34; A61P 3/04; A61P 3/10; A61P 43/00**

(86) International application number:
**PCT/JP2020/017399**

(87) International publication number:
**WO 2020/218379 (29.10.2020 Gazette 2020/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.04.2019 JP 2019086867**

(71) Applicant: **Suntory Holdings Limited**
**Osaka 530-8203 (JP)**

(72) Inventors:
- **URAI Soichiro**
  **Kawasaki-shi, Kanagawa 211-0067 (JP)**
- **NAGAO Koji**
  **Kawasaki-shi, Kanagawa 211-0067 (JP)**
- **YOKOO Yoshiaki**
  **Kawasaki-shi, Kanagawa 211-0067 (JP)**

(74) Representative: **Mewburn Ellis LLP**
**Aurora Building**
**Counterslip**
**Bristol BS1 6BX (GB)**

# (54) COMPOSITION FOR PROMOTING GLP-1 SECRETION

(57) It has been expected to develop a better GLP-1 secretagogue component. The present invention provides a composition for promoting GLP-1 secretion that contains emulsified particles comprising an amphiphilic GLP-1 secretagogue component and an emulsifier, wherein the emulsifier is an oil-in-water type emulsifier and the GLP-1 secretagogue component is incorporated in the emulsified particles.

EP 3 960 201 A1

## Description

### Technical Field

The present invention relates to a composition for promoting GLP-1 secretion comprising an emulsified particle comprising an amphiphilic GLP-1 secretion-promoting component and an emulsifier.

### Background Art

Glucagon-like peptide-1 (GLP-1) is a hormone secreted from the gastrointestinal mucosal epithelium and the like. GLP-1 is known to have effects such as stimulation of insulin synthesis and secretion, inhibition of glucagon secretion, inhibition of food intake, and reduction of hyperglycemia. Activation of GLP-1 secretion can be expected to improve these effects.

Patent Literature 1 describes eleven kinds of cucurbitane-type triterpenes contained in bitter melon as novel GLP-1 secretion-promoting components. Patent Literature 2 describes that stevioside, rebaudioside A, rebaudioside B, and rebaudioside D have properties that promote GLP-1 secretion.

### Citation List

#### Patent Literature

Patent Literature 1: WO 2017/159725
Patent Literature 2: WO 2017/018404

### Summary of Invention

#### Problem to be Solved by the Invention

Developments of better GLP-1 secretion-promoting components are expected.

#### Means for Solving the Problems

In one embodiment, the present invention provides the followings.

[1] A composition for promoting GLP-1 secretion, comprising an emulsified particle comprising an amphiphilic GLP-1 secretion-promoting component and an emulsifier, wherein

the emulsifier is an oil-in-water emulsifier; and
the GLP-1 secretion-promoting component is incorporated into the emulsified particle.

[2] The composition according to [1], wherein the GLP-1 secretion-promoting component is rebaudioside A.
[3] The composition according to [1] or [2], wherein an HLB value of the emulsifier is 7 to 18.
[4] The composition according to any one of [1] to [3], wherein the emulsifier comprises at least one selected from the group consisting of a polyglycerol fatty acid ester, a propylene glycol fatty acid ester, an enzyme-treated lecithin, a sucrose fatty acid ester, and an organic acid monoglyceride.
[5] The composition according to any one of the [1] to [4], wherein a hydrophilic group of the emulsifier is derived from a sweetness component.
[6] The composition according to any one of [1] to [5], wherein the emulsifier comprises at least one emulsifier selected from the group consisting of a polyglycerol fatty acid ester and a sucrose fatty acid ester.
[7] The composition according to any one of [1] to [6], wherein a hydrophilic group of the emulsifier comprises a phosphate group.
[8] The composition according to any one of [1] to [7], wherein a proportion of the emulsifier based on the GLP-1 secretion-promoting component is 0.02 to 50% by mass.
[9] The composition according to any one of [1] to [8], wherein the composition comprises the emulsifier in a proportion of 0.1 to 5% by mass.
[10] The composition according to any one of [1] to [9], wherein the composition comprises the GLP-1 secretion-promoting component in a proportion of 1 to 20% by mass.

[11] The composition according to any one of [1] to [10], wherein the composition is for use in improving glycometabolism, in suppressing appetite, or in preventing or ameliorating diabetes or obesity.

[12] A beverage comprising the composition according to any one of [1] to [11].

[13] A food comprising the composition according to any one of [1] to [11].

[14] A pharmaceutical composition comprising the composition according to any one of [1] to [11].

[15] A method of producing an emulsified particle into which a GLP-1 secretion-promoting component is incorporated, comprising the steps of:

mixing an oil-in-water emulsifier, an amphiphilic GLP-1 secretion-promoting component, and an aqueous medium to prepare a mixture; and
adding a fat and oil to the mixture and stirring.

[16] A composition comprising an emulsified particle comprising rebaudioside A and an oil-in-water emulsifier, wherein rebaudioside A is incorporated into the emulsified particle.

Advantageous Effects of Invention

In the present invention, by using an amphiphilic GLP-1 secretion-promoting component and an oil-in-water emulsifier, and incorporating the GLP-1 secretion-promoting component into an emulsified particle, GLP-1 secretion-promoting effect of the GLP-1 secretion-promoting component can be increased.

Brief Description of Drawings

Fig. 1 is a graph showing GLP-1 secretion amounts of various sweeteners in Experimental Example 1.

Fig. 2 is a graph showing a relationship between the concentration of rebaudioside C (hereinafter, rebaudioside is sometimes abbreviated as Reb)-containing sample obtained in Experimental Example 2 and the GLP-1 secretion amount.

Fig. 3 is a graph showing a relationship between the concentration of stevioside-containing sample obtained in Experimental Example 3 and the GLP-1 secretion amount.

Fig. 4 is a graph showing a relationship between the concentration of rebaudioside A-containing sample obtained in Experimental Example 4 and the GLP-1 secretion amount.

Fig. 5 is a graph showing a relationship between the concentration of emulsified rebaudioside A-containing sample obtained in Experimental Example 5 and the GLP-1 secretion amount.

Fig. 6 is a graph showing the surface tension of various sweeteners in Experimental Example 6.

Fig. 7 is a graph showing the flavor characteristics of RebA in Experimental Example 7.

Fig. 8 is a graph showing a relationship between the concentration of various samples obtained in Experimental Examples 8 to 13 and the GLP-1 secretion amount.

Fig. 9 is a graph showing a relationship between the concentration of rebaudioside A-containing sample obtained in Experimental Example 8 and the GLP-1 secretion amount.

Fig. 10 is a graph showing a relationship between the concentration of emulsified rebaudioside A-containing sample obtained in Experimental Example 9 and the GLP-1 secretion amount.

Fig. 11 is a graph showing a relationship between the concentration of emulsified particle-containing sample obtained in Experimental Example 10 and the GLP-1 secretion amount.

Fig. 12 is a graph showing a relationship between the concentration of the mixed solution of RebA-containing sample and emulsified particle-containing sample obtained in Experimental Example 11 and the GLP-1 secretion amount.

Fig. 13 is a graph showing a relationship between the concentration of glycerol-containing sample obtained in Experimental Example 12 and the GLP-1 secretion amount.

Fig. 14 is a graph showing a relationship between the concentration of MCT oil-containing sample obtained in Experimental Example 13 and the GLP-1 secretion amount.

Fig. 15 is a graph showing the cytotoxicity (using dead cells as an indicator) of various samples obtained in Experimental Examples 15 to 20. The result after 2 hours from the administration is shown in a, and the result after 24 hours from the administration is shown in b.

Fig. 16 is a graph showing the GLP-1 secretion amount of various samples obtained in Experimental Examples 21 to 31. The result after 2 hours from the administration is shown in a, and the result after 24 hours from the administration is shown in b.

Fig. 17 shows the results of distribution ratio and the photos showing status during the test of each steviol glycoside obtained in Experimental Example 32.
Fig. 18 is a photo showing the status of the RebA-containing solution after the test in Experimental Example 32.
Fig. 19 is a diagram showing the effect of storage temperature on flavor characteristics in Experimental Example 33.
Fig. 20 is a diagram showing the stability under vibration environments in Experimental Example 33.
Fig. 21 is a diagram showing the degree of reduction in sweetness intensity due to emulsification in Experimental Example 33.
Fig. 22 is a graph showing the GLP-1 secretion amount in Experimental Example 34.

Description of Embodiments

Through the study of the present inventors, it has been found that better GLP-1 secretion-promoting ability can be achieved by incorporating a GLP-1 secretion-promoting component such as RebA into emulsified particles such as micelles.
It has been also found that the composition comprising an emulsified particle into which a GLP-1 secretion-promoting component is incorporated maintains the emulsified state after storage, especially after storage under harsh environments such as high-temperature conditions, acidic conditions, and vibration environments, and tends to have little taste deterioration after storage at high temperatures and excellent storage stability.

<GLP-1 secretion-promoting component>

A GLP-1 secretion-promoting component is a substance or composition having a property that the substance or composition itself acts on cells of human or non-human animals having GLP-1 secretion ability to increase the amount of GLP-1 secretion (hereinafter referred to as GLP-1 secretion-promoting ability).
When a substance or composition having GLP-1 secretion-promoting ability is administered to GLP-1 secreting cells, the amount of GLP-1 secretion is increased. The increase or decrease of the GLP-1 secretion amount is confirmed in the same manner as in the experimental examples. That is, a sample containing a test substance or test composition and a non-stimulant negative comparison sample of the same composition except that the sample does not contain the test substance or test composition are prepared, and both samples are added to human colon-derived cells having GLP-1 secretion ability (e.g., H716) under the same conditions, then the amount of GLP-1 secretion tested with the sample is compared to that with the non-stimulant negative comparison sample to confirm an increase or decrease of the amount. Note that the negative comparison is sometimes referred to herein as a "negative control".
The GLP-1 secretion-promoting component used in the present invention is amphiphilic. The term "amphiphilic" means that "a molecule or a group of atoms has both a part of high affinity with water (a hydrophilic group) and a part of low affinity with water (a hydrophobic group)" (see, Digital Dai-ji-sen Japanese Dictionary).
The amphiphilic GLP-1 secretion-promoting component tends to have a smaller surface tension than water.
The amphiphilic GLP-1 secretion-promoting component preferably includes a glycoside sweetener having GLP-1 secretion-promoting ability. Preferred examples of the glycoside sweetener include a steviol glycoside or mogroside (hereinafter, mogroside is sometimes abbreviated as Mog) having GLP-1 secretion-promoting ability. More preferred examples include rebaudioside A, rebaudioside B, a stevia extract, a steviol glycoside having a structure to which rhamnose is attached, stevioside, and mogroside V, and even more preferred examples include rebaudioside A, rebaudioside B, rebaudioside C, stevioside, and mogroside V.
The glycoside sweetener described above preferably has surface tension Q (mN/m) of any of the following:
Q = 40-70, 45-65, 40-60, 50-70, 58-63, 59-62, 60-61, 50-63, 51-63, 52-63, 53-63, 54-63, 55-63, 56-63, 57-63, 59-63, 60-63, 58-62, 58-61, 58-60.
Surface tension Q is measured by a plate method. For example, a glycoside sweetener, which is a test substance, is added in an amount to have Brix 10 on a sucrose basis to prepare an aqueous solution. The surface tension of the prepared aqueous solution is measured by a plate method using an automatic surface tension meter (CBVP-Z type, manufactured by Kyowa Interface Science Co., Ltd).
In view of application to foods and beverages, a steviol glycoside is preferred as the glycoside sweetener having GLP-1 secretion-promoting ability.
Examples of the amphiphilic GLP-1 secretion-promoting component other than the glycoside sweetener include a protein and a glycoprotein. Examples thereof include a milk protein including a casein, a casein salt or a whey protein; an egg yolk protein, an egg white protein, a soy protein, a wheat protein, a pea protein, a mucin, a proteoglycan, and a processed product thereof.
In the present invention, rebaudioside A is most preferred as the amphiphilic GLP-1 secretion-promoting component. This is because the GLP-1 secretion-promoting ability of rebaudioside A itself can be effectively enhanced by incorporation into emulsified particles. The reason for this is not known, but the present inventors presume as follows.

Rebaudioside A has a structure in which four glucoses are attached to the aglycone steviol. There are various types of steviol glycosides other than rebaudioside A. Herein, by way of example, the structures of the steviol glycosides used in Experimental Example 32 (for the distribution ratio) are shown.

Formula 1

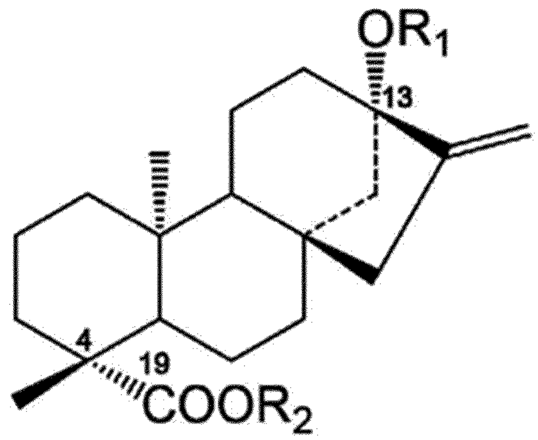

| Compound Name | $R_1$ | $R_2$ |
|---|---|---|
| Rebaudioside A (rebA) | Glcβ1,2(Gluβ1,3)Gluβ1- | Glcβ1- |
| Stevioside (stv) | Glcβ1,2Gluβ1- | Glcβ1- |
| Rebaudioside D (rebD) | Glcβ1,2(Gluβ1,3)Gluβ1- | Glcβ1,2Glcβ1- |
| Rebaudioside M (rebM) | Glcβ1,2(Gluβ1,3)Gluβ1- | Glcβ1,2(Gluβ1,3)Gluβ1- |

When the present inventors measured the distribution ratio of rebaudioside A and other stevioside glycosides, it was surprisingly found that only systems using rebaudioside A had an emulsification phenomenon in which the organic solvent was dispersed into the aqueous medium and resulted in that the organic layer and the aqueous layer could not be separated, despite the fact that rebaudioside A and other stevioside glycosides were only slightly different in the position and the number of glucose attached. That is, rebaudioside A does not only exhibit amphiphilicity, but also exhibits much better emulsification ability than other steviol glycosides.

The surface tension was also studied, and it was found that the surfactant effect of RebA was higher than that of other glycosides. This low surface tension of RebA and the structural characteristic of RebA that glucoses are attached at positions 13 and 19 of the steviol backbone are believed to make RebA relatively tend to be amphiphilic by having hydrophobic and hydrophilic moieties. Accordingly, it is presumed that rebaudioside A tends to be incorporated into the shell present outside of the core of the emulsified particles, which results in a great enhancement of GLP-1 secretion-promoting ability.

<Emulsifier>

The emulsifier used in the present invention is an oil-in-water emulsifier.

It is preferred that the oil-in-water emulsifier has one of the following HLB values: 7 to 18, 9 to 18, 11 to 18, 13 to 18, 15 to 18, 7 to 17, 7 to 15, 7 to 13, 7 to 11, 7 to 9, 9 to 16, or 11 to 14.

In the present invention, the HLB value is calculated by Griffin's method.

In the present invention, the oil-in-water emulsifier may be, but is not limited to, a known oil-in-water emulsifier. Examples of the oil-in-water emulsifier under the requirement that the HLB value is any one of the above values include a glycerol fatty acid ester such as a monoglyceride, an organic acid monoglyceride, and a polyglycerol fatty acid ester; a sorbitan fatty acid ester; a sucrose fatty acid ester; a propylene glycol fatty acid ester; a stearoyl lactate; a polysorbate; a lecithin such as a plant lecithin, an egg yolk lecithin, a fractionated lecithin, or an enzyme-treated lecithin; a kiraya extract, a yucca foam extract; a plant sterol, a sphingolipid; a bile powder, and an animal sterol.

Preferred examples of the oil-in-water emulsifier under the requirement that the HLB value is any one of the above values include at least one selected from the group consisting of a polyglycerol fatty acid ester, a propylene glycol fatty acid ester, an enzyme-treated lecithin, a sucrose fatty acid ester, and an organic acid monoglyceride.
Alternatively, it is preferred that the emulsifier used includes an emulsifier in which the hydrophilic group is derived from a sweetness component under the requirement that the HLB value is any one of the above values, and it is particularly preferred that the emulsifier used includes at least one emulsifier selected from the group consisting of a polyglycerol fatty acid ester and a sucrose fatty acid ester.
Furthermore, under the requirement that the HLB value is any one of the above values, it is preferred that the emulsifier includes an emulsifier having a hydrophilic group comprising a phosphate group, and is particularly preferred that the emulsifier includes a lecithin.

<Composition>

In the composition of the present invention, the GLP-1 secretion-promoting component is incorporated into an emulsified particle.
In the present invention, the "emulsified particle" means a colloidal particle in which molecules or ions derived from an emulsifier gather to form a colloidal particle. In the field of emulsifiers, colloidal particles are sometimes differently referred to as micelles, nanoemulsions, emulsions, or the like, depending on the size (see, Toshiyuki Suzuki, J. Soc. Cosmet. Jpn., Vol. 44, No. 2, 2010). The "emulsified particle" in the present invention includes all of these.
The phrase "GLP-1 secretion-promoting component is incorporated into an emulsified particle" means that a molecule or ion derived from at least a portion of the GLP-1 secretion-promoting component is incorporated into at least a portion of the emulsified particle comprised in the composition. The term "incorporated" means that at least a portion of the molecule or ion derived from the GLP-1 secretion-promoting component is present at an interior of or in contact with the emulsified particle. The "interior of an emulsified particle" means a core of the emulsified particle, or a shell located at or outside of the core of the emulsified particle and mainly composed a part of the hydrophilic group to the hydrophobic group of the emulsifier. Given that the GLP-1 secretion-promoting component is amphiphilic, it is presumed that the GLP-1 secretion-promoting component is mainly present in or tends to contact with the shell of the emulsified particle. When the GLP-1 secretion-promoting component is incorporated into an emulsified particle, the GLP-1 secretion-promoting component tends to be comprised in the fraction on the emulsified particle side upon fractionation between the emulsified particle and an aqueous medium.
It is preferred that the incorporation ratio of the GLP-1 secretion-promoting component to the emulsified particle is 10 to 90%. The incorporation ratio is expressed by the following formula:

Incorporation ratio (%) = (Amount of GLP-1 secretion-promoting component incorporated into emulsified particle/Amount of total GLP-1 secretion-promoting component in composition) × 100
In the calculation of the incorporation ratio, the GLP-1 secretion-promoting component that are incorporated into the emulsified particle and the GLP-1 secretion-promoting component that are not incorporated into the emulsified particle may be separated from the emulsified composition. Specifically, the emulsified composition is subjected to a separation operation such as filter filtration, centrifugal filtration, column separation, or the like.

Preferably, filter filtration is performed as a separation operation.
The average particle size of the emulsified particles is preferably 0.05 to 0.5, 0.07 to 0.5, 0.05 to 0.4, 0.05 to 0.3, 0.05 to 0.2, 0.05 to 0.1, 0.1 to 0.4, 0.2 to 0.3, 0.07 to 0.15, 0.07 to 0.12 or 0.07 to 0.09 μm at a stage before storage (e.g., within half a day from the production).
The average particle size of the emulsified particles tends not to change much after long-term storage. For example, when the GLP-1 secretion-promoting composition of the present invention is stored at 4°C for 2 months after preparation, the rate R of change in the average particle size before and after storage (R = Average particle size after storage/Average particle size before storage × 100 - 100) is -40% to +40%, -37% to +37%, -35% to +35%, -20% to +20%, or -12% to +12%.
The average particle size of the emulsified particles can be measured by a laser diffraction scattering method. For example, as shown in the Examples described later, the average particle size of the emulsified particles can be measured by appropriately diluting a sample with ion exchange water so that the laser scattering intensity is about 1%, and then measuring the diluted sample with a laser scattering type particle size distribution meter (Spectris Co., Ltd., Malvern Panalytical).
The proportion (% by mass) of the emulsifier based on the GLP-1 secretion-promoting component is preferably 0.02 to 50, 0.07 to 50, 0.1 to 50, 0.4 to 50, 0.7 to 50, 1 to 50, 5 to 50, 10 to 50, 20 to 50, 30 to 50, 40 to 50, 0.02 to 40, 0.02 to 30, 0.02 to 20, 0.02 to 10, 0.02 to 5, 0.02 to 1, 0.07 to 40, 0.1 to 30, 0.4 to 20, 0.7 to 10, 1 to 5, 1 to 100, 5 to 100, 9 to 100, 1 to 100, 1 to 90, 1 to 80, 1 to 70, 1 to 60, 1 to 50, 1 to 40, 1 to 30, 1 to 20, 1 to 10, 5 to 99, 5 to 90, 5 to

80, 5 to 70, 5 to 60, 5 to 50, 5 to 40, 5 to 30, 5 to 20, 5 to 10, 10 to 50, 20 to 50, 30 to 50, 40 to 50, 5 to 40, 10 to 30% by mass.

Alternatively, the proportion (% by mass) of the emulsifier based on the GLP-1 secretion-promoting component is preferably 5 to 200, 7 to 200, 9 to 200, 10 to 200, 30 to 200, 50 to 200, 70 to 200, 90 to 200, 110 to 200, 130 to 200, 150 to 200, 170 to 200, 5 to 180, 5 to 160, 5 to 140, 5 to 120, 5 to 100, 5 to 80, 5 to 60, 5 to 40, 5 to 30, 5 to 20, 7 to 180, 9 to 160, 10 to 140, 30 to 120, 50 to 100, 10.0 to 180, 10.0 to 160, 10.0 to 140, 10.0 to 120, 10.0 to 100, 10.0 to 80 or 10.0 to 60% by mass.

The content (% by mass) of the emulsifier in the composition of the present invention is preferably 0.1 to 5, 0.1 to 4, 0.1 to 3, 0.1 to 2, 0.1 to 1, 0.3 to 5, 0.5 to 5, 0.7 to 5, 0.9 to 5, 1.1 to 5, 2.1 to 5, 3.1 to 5, 4.1 to 5, 0.3 to 4, 0.5 to 3, 0.7 to 2, 0.9 to 1, 0.3 to 5, 0.5 to 5.0, 0.7 to 5.0, 0.9 to 5.0, 1 to 5.0, 1.5 to 5.0, 2 to 5.0, 2.5 to 5.0, 3 to 5.0, 3.5 to 5.0, 4 to 5.0, 4.5 to 5.0, 0.1 to 4, 0.1 to 3, 0.1 to 2, 0.1 to 1, 0.1 to 0.8, 0.1 to 0.6, 0.1 to 0.4, 0.1 to 0.2, 0.2 to 4.5, 0.3 to 4.0, 0.4 to 3.5, 0.5 to 3.0, 0.6 to 2.5, 0.7 to 2.0, 0.8 to 1.5, or 0.9 to 1.0% by mass, on a mass basis.

Alternatively, the content (% by mass) of the emulsifier in the composition of the present invention is preferably 0.001 to 1, 0.001 to 0.5, 0.001 to 0.25, 0.001 to 0.2, 0.01 to 1, 0.01 to 0.5, 0.01 to 0.25, 0.01 to 0.2, 0.1 to 1,0.1 to 10, 0.4 to 10, 0.7 to 10, 1 to 10, 3 to 10, 5 to 10, 7 to 10, 0.1 to 8, 0.1 to 6, 0.1 to 4, 0.1 to 2, 1 to 8, 1 to 7, 1 to 6, 3 to 10, 3 to 7, 3 to 6, or 5 to 7% by mass, on a mass basis.

The content (% by mass) of the GLP-1 secretion-promoting component in the composition of the present invention is preferably 0.1 to 50, 1 to 20, 0.1 to 40, 0.1 to 30, 0.1 to 20, 0.1 to 10, 0.1 to 5, 0.1 to 1, 0.4 to 50, 0.7 to 50, 1 to 50, 5 to 50, 10 to 50, 20 to 50, 30 to 50, 40 to 50, 0.4 to 40, 0.7 to 30, 1 to 20, 5 to 10, 2 to 15, 2 to 10, 2 to 5, 5 to 20, 8 to 20, 11 to 20, 14 to 20, 17 to 20, 5 to 15, or 8 to 10% by mass.

Alternatively, the content (% by mass) of the GLP-1 secretion-promoting component in the composition of the present invention is preferably 1 to 500,000, 1 to 400,000, 1 to 300,000, 1 to 200,000, 1 to 100,000, 1 to 80,000, 1 to 60,000, 1 to 40,000, 1 to 20,000, 1 to 10,000, 1 to 8,000, 1 to 6,000, 1 to 4,000, 1 to 2,000, 1 to 1,000, 1 to 500, 50 to 500,000, 100 to 500,000, 500 to 500,000, 1,000 to 500,000, 1,500 to 500,000, 3,000 to 500,000, 4,500 to 500,000, 6,000 to 500,000, 7,500 to 500,000, 9,000 to 500,000, 10,000 to 500,000, 50,000 to 500,000, 100,000 to 500,000, 200,000 to 500,000, 300,000 to 500,000, 400,000 to 500,000, 50 to 400,000, 100 to 300,000, 500 to 200,000, 1,000 to 100,000, 1,500 to 80,000, 3,000 to 60,000, 4,500 to 40,000, 6,000 to 20,000, or 7,500 to 10,000 ppm, on a mass basis. Alternatively, it is preferably 25 to 550, 30 to 550, 35 to 550, 40 to 550, 45 to 550, 50 to 550, 55 to 550, 20 to 540, 25 to 540, 30 to 540, 35 to 540, 40 to 540, 45 to 540, 50 to 540, 55 to 540, 20 to 530, 25 to 530, 30 to 530, 35 to 530, 40 to 530, 45 to 530, 50 to 530, 55 to 530, 20 to 520, 25 to 520, 30 to 520, 35 to 520, 40 to 520, 45 to 520, 50 to 520, 55 to 520, 20 to 510, 25 to 510, 30 to 510, 35 to 510, 40 to 510, 45 to 510, 50 to 510, 55 to 510, 20 to 505, 25 to 505, 30 to 505, 35 to 505, 40 to 505, 45 to 505, 50 to 505, 55 to 505, 20 to 500, 25 to 500, 30 to 500, 35 to 500, 40 to 500, 45 to 500, 50 to 500, 55 to 500, 20 to 495, 25 to 495, 30 to 495, 35 to 495, 40 to 495, 45 to 495, 50 to 495, 55 to 495, 20 to 490, 25 to 490, 30 to 490, 35 to 490, 40 to 490, 45 to 490, 50 to 490, or 55 to 490 ppm, on a mass basis. Alternatively, it is preferably 1 to 1500, 1 to 1200, 5 to 1200, 1 to 1000, 5 to 1000, 10 to 1000, 1 to 900, 5 to 900, 10 to 900, 15 to 900, 20 to 900, 25 to 900, 30 to 900, 35 to 900, 40 to 900, 45 to 900, 50 to 900, 55 to 900, 1 to 800, 5 to 800, 10 to 800, 15 to 800, 20 to 800, 25 to 800, 30 to 800, 35 to 800, 40 to 800, 45 to 800, 50 to 800, 55 to 800, 1 to 700, 5 to 700, 10 to 700, 15 to 700, 20 to 700, 25 to 700, 30 to 700, 35 to 700, 40 to 700, 45 to 700, 50 to 700, 55 to 700, 1 to 600, 5 to 600, 10 to 600, 15 to 600, 20 to 600, 25 to 600, 30 to 600, 35 to 600, 40 to 600, 45 to 600, 50 to 600, 55 to 600, 1 to 550, 1 to 540, 1 to 530, 1 to 520, 1 to 510, 1 to 505, 1 to 500, 1 to 495, 1 to 490, 5 to 550, 5 to 540, 5 to 530, 5 to 520, 5 to 510, 5 to 505, 5 to 500, 5 to 495, 5 to 490, 10 to 550, 10 to 540, 10 to 530, 10 to 520, 10 to 510, 10 to 505, 10 to 500, 10 to 495, 10 to 490, 15 to 550, 15 to 540, 15 to 530, 15 to 520, 15 to 510, 15 to 505, 15 to 500, 15 to 495, or 15 to 490 ppm, on a mass basis.

Alternatively, the content of the GLP-1 secretion-promoting component in the composition of the present invention is preferably 0.1 to 25,000, 1 to 25000, 10 to 25000, 0.1 to 2500, 1 to 2500, or 10 to 2500 ppm, on a mass basis.

The GLP-1 secretion-promoting ability of the composition of the present invention is increased compared to that of the GLP-1 secretion-promoting component alone. Whether or not the GLP-1 secretion-promoting ability of the composition of the present invention is increased compared to that of the GLP-1 secretion-promoting component alone is confirmed by the following method. That is, the composition of the present invention and a composition to be compared that is the same except that only the GLP-1 secretion-promoting component is contained as the active ingredient are prepared. The composition of the present invention and the composition to be compared are administered to cells having GLP-1 secretion ability, and the amount of GLP-1 secretion is measured. When the amount of GLP-1 secretion with the administration of the composition of the present invention is greater than that with the administration of the composition to be compared, it is determined that the GLP-1 secretion-promoting ability is improved. Preferably, the GLP-1 secretion-promoting ability of the composition of the present invention is increased by 1.05 times or more, more preferably 1.1 times or more, particularly preferably 1.2 to 100 times, and most preferably 2 to 50 times compared to that of the GLP-1 secretion-promoting component alone.

The composition of the present invention may comprise a further component other than the GLP-1 secretion-promoting component and the emulsifier.

The further component is appropriately determined depending on the use, etc., of the composition of the present

invention. Examples of the further component include a sweetener, an excipient, a binder, a disintegrant, a coating agent, a lubricant, a colorant, a taste and flavor modifier, a stabilizer, an absorption enhancer, a pH adjusting agent, a preservative, an anti-oxidant, a fragrance, a vitamin, a trace metal ingredient, and the like.

The sweetener of the further component may be a natural sweetener, a sugar alcohol, an artificial sweetener, or the like. Examples include glucose; fructose; maltose; sucrose; lactose; a rare sugar; a peptide-based sweetener such as aspartame, neotame, alitame; a sucrose derivative such as sucralose; a synthetic sweetener such as acesulfame K, saccharin, advantame, sodium cyclohexylsulfamate, and dulcin; a plant protein-based sweetener such as monellin, curculin, brazzein, and thaumatin; taumarin; neohesperidin dihydrochalcone; a sugar alcohol such as erythritol, xylitol, sorbitol, maltitol, and mannitol; a high fructose liquor; a fructose-glucose liquor; a glucose-fructose liquor; an oligosaccharide; honey; a sugar cane juice (brown sugar syrup); a sugar (white sugar, soft brown sugar, brown sugar, refined Japanese sugar, and the like); a maple syrup; molasses; and a starch syrup.

As the further component, a component that may be selectively present in the center (core) of the emulsified particle, such as a fat and oil, are suitably used. As the fat and oil, an MCT oil composed of a fatty acid of C6 to C12 is preferably used.

The component that can be selectively present in the center (core) of the emulsified particle is preferably comprised in an amount of 0.5 to 500% by mass based on the GLP-1 secretion-promoting component.

Alternatively, the component that can be selectively present in the center (core) of the emulsified particle is preferably comprised in an amount of 100 to 400, 150 to 400, 200 to 400, 250 to 400, 300 to 400, 350 to 400, 100 to 350, 100 to 300, 100 to 250, 150 to 250, 100 to 200, 100 to 150, 190 to 300, or 240 to 300% by mass based on the GLP-1 secretion-promoting component.

Furthermore, it is preferred that an aqueous base such as glycerol is used as the further component.

The aqueous base is preferably comprised in an amount of 2 to 600% by mass based on the GLP-1 secretion-promoting component.

Alternatively, the aqueous base is preferably comprised in an amount of 2 to 2100, 100 to 2100, 200 to 2100, 300 to 2100, 400 to 2100, 500 to 2100, 600 to 2100, 700 to 2100, 800 to 2100, 900 to 2100, 1000 to 2100, 100 to 2000, 100 to 1800, 100 to 1600, 100 to 1400, 100 to 1200, 100 to 1000, 100 to 800, 100 to 700, 100 to 600, 100 to 500, 200 to 1800, 300 to 1600, 400 to 1400, 500 to 1200, 600 to 1000, 490 to 2100, 490 to 1900, 490 to 1700, 490 to 1500, 490 to 1300, 490 to 1100, 490 to 900, 490 to 700, 490 to 600, 490 to 560, 490 to 550, 490 to 540, or 490 to 530% by mass based on the GLP-1 secretion-promoting component.

As will be described in detail later, when the composition of the present invention is used as a food and beverage, a food additive may be contained as the further component. Examples of the food additive include an excipient (such as a wheat starch, a corn starch, a cellulose, lactose, sucrose, mannitol, sorbitol, xylitol, a pregelatinized starch, a casein, a magnesium aluminate silicate, and a calcium silicate); a binder (such as a pregelatinized starch, a hydroxypropyl methyl cellulose, and a polyvinylpyrrolidone; a disintegrant such as a cellulose, a hydroxypropyl methyl cellulose, and a corn starch); a fluidizer (such as a light anhydrous silicic acid); a fat and oil (for example, a vegetable oil such as a soybean oil, a sesame oil, an olive oil, a linseed oil, a perilla oil, a rapeseed oil, a coconut oil, and a corn oil, or an oil derived from animals or fish); a nutrient (such as various minerals various kind of vitamins, and an amino acid); a flavor; a sweetener; a taste modifier; a colorant; a solvent (such as ethanol); salts; a pH adjusting agent; a buffer; an anti-oxidant; a stabilizer, a gelling agent; a thickener; a lubricant; an encapsulant; a suspending agent; a coating agent; a preservative; and an emulsifier. The food additive may be used singly or in combination of two or more.

Examples of the composition of the present invention include a composition comprising: RebA as the GLP-1 secretion-promoting component; at least one selected from the group consisting of a polyglycerol fatty acid ester, a propylene glycol fatty acid ester, an enzyme-treated lecithin, a sucrose fatty acid ester, and an organic acid monoglyceride as the oil-in-water emulsifier; an MCT oil composed of a fatty acid of C6 to C12 as the component that can be selectively present in the center (core) of the emulsified particle; glycerol as the aqueous base; and an aqueous medium, particularly water, as a dispersion medium. Specific examples include a composition comprising: RebA as the GLP-1 secretion-promoting component; a polyglycerol fatty acid ester as the oil-in-water emulsifier, an MCT oil composed of a fatty acid of C6 to C12 as the component that can be selectively present in the center (core) of the emulsified particle; glycerol as the aqueous base; and water as the dispersion medium.

Further examples of the composition of the present invention include a composition comprising: RebA as the GLP-1 secretion-promoting component; at least one selected from the group consisting of a polyglycerol fatty acid ester, a propylene glycol fatty acid ester, an enzyme-treated lecithin, a sucrose fatty acid ester, and an organic acid monoglyceride as the oil-in-water emulsifier; an MCT oil composed of a fatty acid of C6 to C12 as the component that can be selectively present in the center (core) of the emulsified particle; glycerol as the aqueous base; and an aqueous medium, particularly water, as the dispersion medium, in which the content of the oil-in-water emulsifier is 0.02 to 50% by mass based on the GLP-1 secretion-promoting component; the content of the component that can be selectively present in the center (core) of the emulsified particle is 0.5 to 500% by mass based on the GLP-1 secretion-promoting component; and the content of the aqueous base is 2 to 600% by mass based on the GLP-1 secretion-promoting component. Specific

examples include a composition comprising: RebA as the GLP-1 secretion-promoting component; a polyglycerol fatty acid ester as the oil-in-water emulsifier, an MCT oil composed of a fatty acid of C6 to C12 as the component that can be selectively present in the center (core) of the emulsified particle; glycerol as the aqueous base; and water as the dispersion medium, in which the content of the oil-in-water emulsifier is 0.02 to 50% by mass based on the GLP-1 secretion-promoting component; the content of the component that can be selectively present in the center (core) of the emulsified particle is 2 to 600% by mass based on the GLP-1 secretion-promoting component; and the content of the aqueous base is 2 to 600% by mass based on the GLP-1 secretion-promoting component.

Another example of the composition of the present invention includes a composition comprising: RebA as the GLP-1 secretion-promoting component; at least one selected from the group consisting of a polyglycerol fatty acid ester, a propylene glycol fatty acid ester, an enzyme-treated lecithin, a sucrose fatty acid ester, and an organic acid monoglyceride as the oil-in-water emulsifier; an MCT oil composed of a fatty acid of C6 to C12 as the component that can be selectively present in the center (core) of the emulsified particle; glycerol as the aqueous base; and an aqueous medium, particularly water, as the dispersion medium, in which the content of the oil-in-water emulsifier is 5 to 200% by mass based on the GLP-1 secretion-promoting component; the content of the component that can be selectively present in the center (core) of the emulsified particle is 100 to 400% by mass based on the GLP-1 secretion-promoting component; and the content of the aqueous base is 2 to 2,100% by mass based on the GLP-1 secretion-promoting component. Specific examples include a composition comprising: RebA as the GLP-1 secretion-promoting component; a polyglycerol fatty acid ester as the oil-in-water emulsifier, an MCT oil composed of a fatty acid of C6 to C12 as the component that can be selectively present in the center (core) of the emulsified particle; glycerol as the aqueous base; and water as the dispersion medium, in which the content of the oil-in-water emulsifier is 5 to 200% by mass based on the GLP-1 secretion-promoting component; the content of the component that can be selectively present in the center (core) of the emulsified particle is 100 to 400% by mass based on the GLP-1 secretion-promoting component; and the content of the aqueous base is 2 to 2,100% by mass based on the GLP-1 secretion-promoting component.

The composition of the present invention may be a composition of any form, and may be, for example, a solid (e.g., powdery), gel-like, or liquid composition.

The composition of the present invention can be applied for use in GLP-1 secretion promotion.

For example, the composition of the present invention can be used for at least one use selected from suppressing elevated blood glucose levels, suppressing appetite, suppressing overeating, improving glycometabolism, preventing or treating diabetes, preventing or treating obesity, reducing body weight, and reducing body fat percentage. Preferably, the composition of the present invention can be used for at least one use selected from improving glycometabolism, suppressing appetite, and preventing or ameliorating diabetes or obesity.

The composition of the present invention can also be used for promoting gastric emptying, suppressing gastric acid secretion, suppressing liver glucose release, myocardial protection, improving learning memory, preventing or treating postprandial hyperglycemia (hidden diabetes), preventing or treating ulcerative colitis, and the like.

The composition of the present invention can also be used for preventing or treating vascular diseases such as atherosclerosis, neurodegenerative diseases, non-alcoholic hepatitis, chloasma, and asthma (see, Young-Sun Lee et al., "Anti-Inflammatory Effects of GLP-1-Based Therapies beyond Glucose Control", Mediators of Inflammation Volume 2016, Article ID 3094642, 11 pages).

Alternatively, the composition of the present invention can be a composition for use in reducing bitter taste or for use in reducing sweetness fold. For example, when the GLP-1 secretion-promoting component comprises a glycoside sweetener, the composition of the present invention can be used for the purpose of reducing bitterness or sweetness fold of the glycoside sweetener by incorporation of the GLP-1 secretion-promoting component into the emulsified particle.

The composition of the present invention can itself be directly used for (taken by, received by, or administered to) a human or a non-human animal having GLP-1 secretion ability.

Examples of an embodiment of the composition of the present invention used directly for human or non-human animals include a food, for example, a noodle (such as a soba noodle, an udon, a Chinese noodle, an instant noodle); tofu; a confectionery (such as a candy, a gum, a chocolate, a snack, a biscuit, a cookie, and a gummy); a bread; a marine or livestock processed food (such as a kamaboko, a ham, and a sausage); a dairy product (such as a fermented milk); a fat and oil, and an fat-and-oil processed food (such as a salad oil, a tempura oil, a margarine, and a mayonnaise, a shortening, a whipped cream, a dressing, and a fat spread); a condiment (such as a sauce and a baste); a cooked or semi-cooked product (such as a chanpuru); a retorted food (such as a curry, a stew, a bowl, a porridge, and a rice porridge); a chilled sweet (such as an ice cream, a sorbet, and a shaved ice); a powdered food (such as a powdered beverage and a powdered soup); and an enteric fluid food for use in suppressing elevated blood glucose levels, suppressing appetite, suppressing overeating, improving glycometabolism, preventing or treating diabetes, preventing or treating obesity, reducing body weight, reducing body fat percentage, promoting gastric emptying, suppressing gastric acid secretion, suppressing liver glucose release, protecting myocardium, improving learning memory, preventing or treating vascular diseases, preventing or treating neurodegenerative diseases, preventing or treating non-alcoholic hepatitis, preventing or treating chloasma, or preventing or treating asthma. The foods for use in the above uses include,

besides the so-called health foods, a food with health claims such as a food for specified health uses and a food with nutrient function claims, supplements (nutritional supplements), and feeds.

Further examples include a beverage such as a tea beverage, a soft drink, a carbonated beverage including a non-alcoholic beer, a nutritional beverage, a fruit beverage, a lactic acid beverage, a juice, an energy drink, an alcoholic beverage, a processed milk, and a prepared soy milk for use in suppressing elevated blood glucose levels, suppressing appetite, suppressing overeating, improving glycometabolism, preventing or treating diabetes, preventing or treating obesity, reducing body weight, reducing body fat percentage, promoting gastric emptying, suppressing gastric acid secretion, suppressing liver glucose release, protecting myocardium, improving learning memory, preventing or treating vascular diseases, preventing or treating neurodegenerative diseases, preventing or treating non-alcoholic hepatitis, preventing or treating chloasma, or preventing or treating asthma.

When the composition of the present invention is formulated to be used, examples of the dosage form include a tablet, a powder, a fine granule, a granule, a dry syrup, a coated tablet, an intra-oral disintegrating tablet, a chewable tablet, a capsule, a soft capsule, a syrup, and an enteral nutritional supplement.

<Production Method>

The composition of the present invention is produced by a known method depending on its use, composition, or the like.

Preferably, the composition of the present invention is produced by the steps of: mixing an oil-in-water emulsifier, an amphiphilic GLP-1 secretion-promoting component, and an aqueous medium to prepare a mixture; and adding a component that can be comprised in a core of an emulsified particle, such as a fat and oil, to the mixture, and stirring. The stirring is preferably carried out at a higher speed using a homo-mixer or the like and under more intense conditions than mixing so that the fat and oil or the like is contained in the core of the emulsified particle.

For stirring at a higher speed in more intense conditions than mixing, the stirring speed is preferably 5,000 to 10,000, 5,000 to 8,000, or 8,000 to 10,000 rpm.

Accordingly, one aspect of the present invention relates to a method for producing a composition for use in promoting GLP-1 secretion, comprising the steps of: mixing an oil-in-water emulsifier, an amphiphilic GLP-1 secretion-promoting component, and an aqueous medium to prepare a mixture; and adding a fat and oil to the obtained mixture and stirring, and a composition produced by the method, which comprises an emulsified particle comprising an amphiphilic GLP-1 secretion-promoting component and an oil-in-water emulsifier, in which the amphiphilic GLP-1 secretion-promoting component is incorporated into the emulsified particle.

As another aspect, the composition of the present invention can also be produced through the steps of: mixing an oil-in-water emulsifier and an aqueous medium to prepare a mixture; and adding an amphiphilic GLP-1 secretion-promoting component and a component that can be comprised in a core of an emulsified particle such as a fat and oil to the prepared mixture and stirring. The stirring is preferably carried out at a higher speed using a homo-mixer or the like and under more intense conditions than mixing so that the fat and oil or the like is contained in the core of the emulsified particle. For stirring at a higher speed in more intense conditions than mixing, the stirring speed is preferably 5,000 to 10,000, 5,000 to 8,000, or 8,000 to 10,000 rpm.

<Application>

The composition of the present invention can also be used in a state contained in a beverage, a food, a pharmaceutical composition, or the like, by a route such as an oral, nasal, or enteral route, or a route with a tube.

When the composition of the present invention is added to (or contained in) a food and beverage, the type of the food and beverage is not particularly limited. The food includes, for example, a noodle (such as a soba noodle, an udon, a Chinese noodle, an instant noodle); tofu; a confectionery (such as a candy, a gum, a chocolate, a snack, a biscuit, a cookie, and a gummy); a bread; a marine or livestock processed food (such as a kamaboko, a ham, and a sausage); a dairy product (such as a fermented milk); a fat and oil, and an fat-and-oil processed food (such as a salad oil, a tempura oil, a margarine, and a mayonnaise, a shortening, a whipped cream, a dressing, and a fat spread); a condiment (such as a sauce and a baste); a cooked or semi-cooked product (such as a chanpuru); a retorted food (such as a curry, a stew, a bowl, a porridge, and a rice porridge); a chilled sweet (such as an ice cream, a sorbet, and a shaved ice); a powdered food (such as a powdered beverage and a powdered soup); and an enteric fluid food. The food also includes, besides the so-called health foods, a food with health claims such as a food for specified health uses and a food with nutrient function claims, supplements (nutritional supplements), feeds, and the like.

The beverage includes, for example, a tea beverage, a soft drink, a carbonated beverage including a non-alcoholic beer, a nutritional beverage, a fruit beverage, a lactic acid beverage, a juice, an energy drink, an alcoholic beverage, a processed milk, a prepared soy milk, and the like.

The food and beverage may comprise a further component as long as the food and beverage comprises the

composition of the present invention. Examples of the further component in this case include the same as those in the examples of the further component in the <Composition> section.

In the present invention, the intake amount (dose or dosage) of the GLP-1 secretion-promoting component is not particularly limited, and can be appropriately selected according to the age, weight, and health condition of the subject, and the like.

The present invention can be used or applied to any therapeutic application (medical application) or non-therapeutic application. Specifically, whether it is classified as a pharmaceutical product, a quasi-pharmaceutical product, a cosmetic, or the like, the present invention can be used or applied as any composition or food and beverage that explicitly or implicitly pursues the function of promoting GLP-1 secretion or the function resulting from the promotion of GLP-1 secretion. Furthermore, the product using the present invention may indicate a function that promotes GLP-1 secretion and a function resulting from GLP-1 secretion promotion. Examples of such an indication include, but are not particularly limited to, a function that promotes GLP-1 secretion and a function resulting from GLP-1 secretion promotion, such as a blood glucose-level elevation suppression function, an appetite suppression function, an overeating suppression function, a glycometabolism improvement function, a diabetes prevention or treatment function, an obesity prevention or treatment function, a weight reduction function, a body fat percentage reduction function, a gastric emptying promotion function, a gastric acid secretion suppression function, a liver glucose release suppression function, a myocardial protective function, a learning memory improvement function, a vascular disease prevention or treatment function, a neurodegenerative disease prevention or treatment function, a non-alcoholic hepatitis prevention or treatment function, a chloasma prevention or treatment function, an asthma prevention or treatment function, or an indication considered to be equivalent to these.

The timing of use of the compositions of the present invention is not particularly limited, and may be, for example, before, during, between, or after meals, or before bedtime.

One aspect of the present invention relates to a composition comprising an emulsified particle comprising an amphiphilic GLP-1 secretion-promoting component and an emulsifier, in which the emulsifier is an oil-in-water emulsifier, the GLP-1 secretion-promoting component is incorporated into the emulsified particle, and the composition is used for treating a disease that can be improved by promoting GLP-1 secretion. One aspect of the present invention relates to a method for promoting GLP-1 secretion in a subject or treating a disease that can be ameliorated by promoting GLP-1 secretion in a subject, comprising administering to the subject in need thereof a composition comprising an emulsified particle comprising an amphiphilic GLP-1 secretion-promoting component and an emulsifier, wherein the emulsifier is an oil-in-water emulsifier, and wherein the GLP-1 secretion-promoting component is incorporated into the emulsified particle. Furthermore, one aspect of the present invention relates to a use of a composition comprising an emulsified particle comprising an amphiphilic GLP-1 secretion-promoting component and an emulsifier for the manufacture of a medicament for treating a disease that can be ameliorated by promoting GLP-1 secretion, wherein the emulsifier is an oil-in-water emulsifier, and wherein the GLP-1 secretion-promoting component is incorporated into the emulsified particle. It should be noted that each of the aspects above may have various features described herein.

Examples of the disease include hyperglycemia, bulimia, gastric acidosis, diabetes, obesity, vascular disease, neurodegenerative disease, non-alcoholic hepatitis, chloasma, and asthma.

Herein, the term "subject" means any individual organism, preferably animal, more preferably mammalian, further preferably human individual. The subject may be healthy (e.g., have no particular or any disease) or may be affected by some disease. When the treatment of a disease that can be ameliorated by promoting GLP-1 secretion is intended, for example, the subject typically means a subject affected by the disease or having a risk of being affected by the disease.

Herein, the term "treatment" includes every type of medically acceptable prophylactic and/or therapeutic intervention aimed at cure, transient remission or prevention, etc. of a disease. The "treatment" includes medically acceptable intervention for various purposes including, for example, the delay or arrest of progression of a disease, the regression or disappearance of a lesion, the prevention of onset of the disease or the prevention of recurrence of the disease. Thus, the composition of the present invention can be used in the treatment and/or the prevention of a disease.

One aspect of the present invention also relates to a method for increasing the GLP-1 secretion-promoting ability of a GLP-1 secretion-promoting component, comprising incorporating (emulsifying) a GLP-1 secretion-promoting component into an emulsified particle.

## Examples

Hereinafter, the present invention is described in more detail by way of Examples. Note that the number of Experimental Example 14 is unused.

<Experimental Example 1>

RebA, RebB, RebM, RebN, RebD, RebC, MogV, and stevioside were examined for their GLP-1 secretion-promoting effects. Specifically, the amount of GLP-1 secretion was measured by the following procedures. The substance that had a larger amount of GLP-1 secretion than that in control, no sample, was determined to have a GLP-1 secretion-promoting effect.

(Preparation of each test substance)

Each test substance B, C, D, E, F, G, I, or J was dissolved in PBS (Thermo Fisher Scientific Inc.) (Cat. No. 14190250) to obtain a stock solution. The concentration of the stock solution is shown below:
B (RebA); 5mM, C (RebB); 1M, D (RebM); 2.5mM, E (RebN); 1M, F (RebD); 2.5mM, G (RebC); 2.5mM, I (MogV); 1M, J(stevioside);1M

(Quantitation of GLP-1)

H716 (ATCC) (Cat. No. ATCC® CCL-251) was cultured in RPMI 1640 (10% FBS (Thermo Fisher Scientific Inc.) (Cat. No. 10439024), 1 mM Sodium Pyruvate) in an incubator at 37°C, 5%$CO_2$.
The preculture was performed for 2 weeks, and the cells were sufficiently recovered, and then cell stocks were prepared.
Cells were seeded in 96-well plates at 2 x $10^5$ cells/90 μL (high) per well (N = 4). At this time, the content of FBS in the medium was lowered to 0.5% to suppress cell proliferation.
After 24 hours, the sample at the highest concentration (i.e., stock solution) was added by 10 μL each.
After further 24 hours, quantitation of GLP-1 was performed. Specific procedures were followed the user guide of Human GLP-1 (7-36 amide) Immunoassay kit (Cat. No. AL359) (Lot number 2361115) provided by PerkinElmer, Inc. PBS was used as a negative control (no sample).
The results are shown in Fig. 1. As can be seen from Fig. 1, RebA, RebB, RebC, MogV, and stevioside had significantly larger amounts of GLP-1 secretion than no sample.

<Experimental Example 2>

To 20 mg of RebC, 21 μL of DMSO (manufactured by FUJIFILM Wako Pure Chemical Corporation, model number: 037-24053) was added. The mixture was then stirred by vortex and allowed to stand for several hours to obtain a stock solution having a RebC concentration of 1,000 mM. The obtained stock solution and PBS were used to prepare a 10 mM solution.
The 10 mM solution was diluted as needed with 1% DMSO-containing PBS to obtain Sample G-1 mM to Sample G-10 mM. The concentrations of RebC are described in Table 1.

<Experimental Example 3>

To 15 mg of stevioside, 12.4 μL of DMSO (manufactured by FUJIFILM Wako Pure Chemical Corporation, model number: 037-24053) was added. The mixture was then stirred by vortex to obtain a stock solution having a stevioside concentration of 150 mM. The obtained stock solution and PBS were used to prepare a 15 mM solution.
The 15 mM solution was diluted as needed with 1% DMSO-containing PBS to obtain Sample J-0.1 mM to Sample J-15 mM. The concentrations of stevioside are described in Table 1.

<Experimental Example 4>

To 20 mg of RebA, 8.27 μL of DMSO (manufactured by FUJIFILM Wako Pure Chemical Corporation, model number: 037-24053) was added followed by an addition of 74.5 μL of PBS.
The mixture was then stirred by vortex to obtain a stock solution having a RebA concentration of 250 mM. The obtained stock solution and PBS were used to prepare a 25 mM solution.
The 25 mM solution was diluted as needed with 1% DMSO-containing PBS to obtain Sample B-1 mM to Sample B-25 mM. The concentrations of RebA are described in Table 1.

<Experimental Example 5>

An aqueous mixed solution was prepared by mixing 490 g of glycerol, 100 g of RebA, and 10 g of polyglycerol

fatty acid ester, and heating and dissolving the mixture at 70°C. To the aqueous mixed solution, 300 g of MCT oil was added as a fat and oil, and the mixture was emulsified at a rotation rate of 8000 rpm with a homo-mixer (manufactured by Tokushu Kika Kogyo Co., Ltd.). After completion of the stirring, the mixture was cooled to 40°C, and 100 g of water was added to obtain a processed RebA solution.

To 241.8 μL of the obtained processed RebA solution, 10 μL of DMSO (manufactured by FUJIFILM Wako Pure Chemical Corporation, model number: 037-24053) and 748.2 μL of PBS were added. The mixture was stirred by vortex to obtain a stock solution having a RebA concentration of 25 mM.

The stock solution was diluted as needed with 1% DMSO-containing PBS to obtain Sample B processed-0.25 mM to Sample B processed-25 mM. The concentrations of RebA are described in Table 1.

<Measurement of amount of GLP-1 secretion>

Under the conditions shown in Table 1, each of the samples obtained in Experimental Examples 2 to 5 was added to a medium. In the obtained medium, cells were cultured, and the amount of GLP-1 secretion after 2 hours was measured. The specific procedures are as follows.

H716 (human-colon-derived cell) was cultured in RPMI 1640 (Thermo Fisher Scientific Inc., model number: 61870-036) (10% FBS (Hyclone, model number: SH30396.03), 1 mM Sodium Pyruvate) in an incubator at 37°C, 5%$CO_2$.

The preculture was performed for 2 weeks, and the cells were sufficiently recovered, and then cell stocks were prepared.

Cells were seeded in 96-well plates at 2 x $10^5$ cells/90 μL per well. At this time, the content of FBS in the medium was lowered to 0.5% to suppress cell proliferation.

After 24 hours, 10 μL of each sample was added (n = 3 to 4 for Sample B, and n = 4 for Sample B processed, Sample G, and Sample J). The final concentrations in the medium are shown in the "Sample F.C. (mM)" column of Table 1.

After a predetermined time (2 hours) from the addition, the 96-well plates were collected.

The collected 96-well plates were centrifuged at 300 g x 5 min, and the supernatant was collected.

GLP-1 in the collected supernatant was quantified with Human GLP-1 (7-36 amide) Immunoassay kit (manufactured by PerkinElmer, Inc., model number: AL359C). The results are shown in Figs. 2 to 5. The amount of GLP-1 secretion in a 1% DMSO-containing solution as a non-stimulant control (negative comparison), and the amount of GLP-1 secretion in an aqueous solution of PMA (manufactured by FUJIFILM Wako Pure Chemical Corporation, model number: 162-23591) as a positive control are also shown in Figs. 2 to 5. The aqueous solution of PMA was obtained by adding 16.21 μL of DMSO to 1 mg of PMA to prepare a stock solution; adding PBS to the stock solution to prepare a 1 mM solution; and diluting it with 1% DMSO-containing PBS.

Table 1 is shown below. The positive comparison in Table 1 is also referred to as positive control. The negative comparison is also referred to as negative control.

Table 1

| | # | Sample | Addition concentration (mM) | ppm equivalent | Addition amount ($\mu L$) | Medium ($\mu L$) | Total ($\mu L$) | Sample F.C. (mM) | ppm equivalent |
|---|---|---|---|---|---|---|---|---|---|
| Non-stimulantnegative comparison | 1 | 1% DMSO-containing PBS | - | - | 10 | 90 | 100 | - | - |
| Positive comparison | 2 | PMA 1%DMSO | 0.01 | 6.2 | 10 | 90 | 100 | 0.001 | 0.6 |
| | 3 | | 1 | 616.8 | 10 | 90 | 100 | 0.1 | 61.7 |
| Test substance | 4 | B 1%DMSO | 1 | 967.0 | 10 | 90 | 100 | 0.1 | 96.7 |
| | 5 | | 2.5 | 2417.5 | 10 | 90 | 100 | 0.25 | 241.8 |
| | 6 | | 5 | 4835.1 | 10 | 90 | 100 | 0.5 | 483.5 |
| | 7 | | 10 | 9670.1 | 10 | 90 | 100 | 1 | 967.0 |
| | 8 | | 15 | 14505.2 | 10 | 90 | 100 | 1.5 | 1450.5 |
| | 9 | | 20 | 19340.2 | 10 | 90 | 100 | 2 | 1934.0 |
| | 10 | | 25 | 24175.3 | 10 | 90 | 100 | 2.5 | 2417.5 |
| | 11 | B processed 1%DMSO | 0.25 | 241.8 | 10 | 90 | 100 | 0.025 | 24.2 |
| | 12 | | 0.5 | 483.5 | 10 | 90 | 100 | 0.05 | 48.4 |
| | 13 | | 1 | 967.0 | 10 | 90 | 100 | 0.1 | 96.7 |
| | 14 | | 2.5 | 2417.5 | 10 | 90 | 100 | 0.25 | 241.8 |
| | 15 | | 5 | 4835.0 | 10 | 90 | 100 | 0.5 | 483.5 |
| | 16 | | 10 | 9670.0 | 10 | 90 | 100 | 1 | 967.0 |
| | 17 | | 15 | 14505.0 | 10 | 90 | 100 | 1.5 | 1450.5 |
| | 18 | | 20 | 19340.0 | 10 | 90 | 100 | 2 | 1934.0 |

(continued)

| | # | Sample | Addition concentration (mM) | ppm equivalent | Addition amount (μL) | Medium (μL) | Total (μL) | Sample F.C. (mM) | ppm equivalent |
|---|---|---|---|---|---|---|---|---|---|
| | 19 | | 25 | 24175.0 | 10 | 90 | 100 | 2.5 | 2417.5 |
| | 20 | G 1%DMSO | 1 | 951.01 | 10 | 90 | 100 | 0.1 | 95.1 |
| | 21 | | 2.5 | 2377.5 | 10 | 90 | 100 | 0.25 | 237.8 |
| | 22 | | 5 | 4755.1 | 10 | 90 | 100 | 0.5 | 475.5 |
| | 23 | | 10 | 9510.1 | 10 | 90 | 100 | 1 | 951.0 |
| | 25 | J 1%DMSO | 0.1 | 80.5 | 10 | 90 | 100 | 0.01 | 8.0 |
| | 26 | | 1 | 804.9 | 10 | 90 | 100 | 0.1 | 80.5 |
| | 27 | | 2.5 | 2012.2 | 10 | 90 | 100 | 0.25 | 201.2 |
| | 28 | | 5 | 4024.4 | 10 | 90 | 100 | 0.5 | 402.4 |
| | 29 | | 10 | 8048.7 | 10 | 90 | 100 | 1 | 804.9 |
| | 30 | | 15 | 12073.1 | 10 | 90 | 100 | 1.5 | 1207.3 |

- For Sample B processed, final concentrations of 0.025 mM (#11) and 0.05 mM (#12) were added to the concentrations to be tested (0.1 to 2.5 mM).
- For Sample G, evaluation was performed at equal to or lower than the final concentration of 1 mM since the sample was not able to be dissolved at the final concentration of 1.5 mM (#24).

<Experimental Example 6: Measurement of surface tension>

To investigate whether there was a correlation between the GLP-1 secretion-promoting effect and surface tension of the emulsifier, experiments were performed in accordance with the following procedures.

Aqueous solutions containing RebA, RebD, and RebM (commercially available products) each were prepared. The contents of RebA, RebD, and RebM were aligned to Brix 10 on a sucrose basis. That is, the contents of RebA, RebD, and RebM were 333 ppm, 351 ppm, and 351 ppm, respectively. The surface tension of the obtained aqueous solution was measured by a plate method using an automatic surface tension meter (CBVP-Z type, manufactured by Kyowa Interface Science Co., Ltd). Water was used as a control and tested in the same manner. The results are shown in Fig. 6.

<Experimental Example 7: Evaluation of flavor characteristics>

The flavor characteristics of an emulsified composition of RebA were evaluated by the following procedure.

RebA was dissolved in pure water to prepare 100 ppm and 300 ppm aqueous solutions, which were used as controls. Next, the processed RebA solution prepared in Experimental Example 5 was added to pure water, and 100 ppm and 300 ppm aqueous solutions were prepared in the same manner. Flavor evaluation of the processed RebA solution-added aqueous solution was performed by seven well-trained sensory panelists by 0.5 points taking the control as 3 points. The average values are shown in the Figure. The flavor was evaluated by sweetness intensity, sweetness lingering, and bitterness intensity.

The results are shown in Fig. 7.

<Experimental Example 8>

To 20 mg of RebA, 8.27 μL of DMSO (manufactured by FUJIFILM Wako Pure Chemical Corporation, model number: 037-24053) was added followed by an addition of 74.43 μL of PBS. The mixture was then stirred by vortex to obtain a stock solution having a RebA concentration of 250 mM. The obtained stock solution and PBS were used to prepare a 50 mM solution.

The 50 mM solution was diluted as needed with 1% DMSO-containing PBS to obtain Sample B-1 mM to Sample B-25 mM. The concentrations of RebA are described in Table 2.

<Experimental Example 9>

An aqueous mixed solution was prepared by mixing 490 g of glycerol, 100 g of RebA, and 10 g of polyglycerol fatty acid ester, and heating and dissolving the mixture at 70°C. To the aqueous mixed solution, 300 g of MCT oil was added as a fat and oil, and the mixture was emulsified at a rotation rate of 8000 rpm with a homo-mixer (manufactured by Tokushu Kika Kogyo Co., Ltd.). After completion of the stirring, the mixture was cooled to 40°C, and 100 g of water was added to obtain a processed RebA solution.

To 241.8 μL of the obtained processed RebA solution, 10 μL of DMSO (manufactured by FUJIFILM Wako Pure Chemical Corporation, model number: 037-24053) and 748.2 μL of PBS were added. The mixture was stirred by vortex to obtain a stock solution having a RebA concentration of 25 mM.

The stock solution was diluted as needed with 1% DMSO-containing PBS to obtain Sample B processed-1 mM to Sample B processed-25 mM. The concentrations of RebA are described in Table 2.

<Experimental Example 10>

The solution C was obtained in the same manner as the preparation of the processed RebA solution in Experimental Example 9 except that 100 g of RebA was not used and 490 g of glycerol, 10 g of polyglycerol fatty acid ester, 300 g of MCT oil, and 200 g of water were used.

To 483.5 μL of the obtained solution C, 10 μL of DMSO (manufactured by FUJIFILM Wako Pure Chemical Corporation, model number: 037-24053) and 506.5 μL of PBS were added. The mixture was stirred by vortex to obtain a stock solution having a RebA equivalent concentration of 50 mM.

The obtained stock solution was diluted with 1% DMSO-containing PBS to obtain Sample C-1 mM to Sample C-25 mM. Table 2 describes RebA equivalent concentrations. The RebA equivalent concentration refers to a concentration of RebA in a given Sample B processed of Experimental Example 9 when concentrations of glycerol, polyglycerol fatty acid ester and MCT oil in a given Sample C and concentrations of glycerol, polyglycerol fatty acid ester and MCT oil in the given Sample B processed are the same.

<Experimental Example 11>

In the same manner as in Experimental Example 8, Sample B containing RebA at twice the concentration in Sample B + C was obtained. That is, Sample B-2 mM, Sample B-5 mM, Sample B-10 mM, Sample B-20 mM, Sample B-30 mM, Sample B-40 mM, and Sample B-50 mM were obtained. The concentration behind the hyphens indicates the concentration of RebA.

In the same manner as in Experimental Example 10, Sample C containing polyglycerol fatty acid ester, etc., at twice the concentration in Sample B + C was obtained. That is, Sample C-2 mM, Sample C-5 mM, Sample C-10 mM, Sample C-20 mM, Sample C-30 mM, Sample C-40 mM, and Sample C-50 mM were obtained. The concentration behind the hyphens indicates the RebA equivalent concentration.

Equal amounts of Sample B and Sample C at the same concentration (e.g., Sample B-2 mM and Sample C-2 mM) were mixed to obtain Sample B + C-1 mM to Sample B + C-25mM. The concentrations of RebA are described in Table 2.

<Experimental Example 12>

To 118.5 μL of glycerol, 10 μL of DMSO (manufactured by FUJIFILM Wako Pure Chemical Corporation, model number: 037-24053) and 871.5 μL of PBS were added. The mixture was stirred by vortex to obtain a stock solution having a RebA equivalent concentration of 25 mM.

The obtained stock solution was diluted as needed with 1% DMSO-containing PBS to obtain Sample D-1 mM to Sample D-25 mM. Table 2 describes the RebA equivalent concentrations. The RebA equivalent concentration refers to a concentration of RebA in a given Sample B processed of Experimental Example 9 when the concentration of glycerol in a given Sample D and the concentrations of glycerol in the given Sample B processed are the same.

<Experimental Example 13>

To 72.5 μL of MCT oil, 10 μL of DMSO (manufactured by FUJIFILM Wako Pure Chemical Corporation, model number: 037-24053) and 917.5 μL of PBS were added. The mixture was stirred by vortex to obtain a stock solution having a RebA equivalent concentration of 25 mM.

The obtained stock solution was diluted as needed with 1% DMSO-containing PBS to obtain Sample E-1 mM to Sample E-25 mM. Table 2 describes the RebA equivalent concentrations. The RebA equivalent concentration refers to a concentration of RebA in a given Sample B processed of Experimental Example 9 when the concentration of MCT oil in a given Sample E and the concentrations of MCT oil in the given Sample B processed are the same.

<Measurement of amount of GLP-1 secretion>

Under the conditions shown in Table 2, each of the samples obtained in Experimental Examples 8 to 13 above was added to a medium. In the obtained medium, cells were cultured, and the amount of GLP-1 secretion after 2 hours was measured. The specific procedures are as follows.

H716 (human-colon-derived cell) was cultured in RPMI 1640 (Thermo Fisher Scientific Inc., model number: 61870-036) (10% FBS (Hyclone, model number: SH30396.03), 1 mM Sodium Pyruvate) in an incubator at 37°C, 5%$CO_2$.

The preculture was performed for 2 weeks, and the cells were sufficiently recovered, and then cell stocks were prepared.

Cells were seeded in 96-well plates at 2 x $10^5$ cells/90 μL per well. At this time, the content of FBS (Hyclone, model number: SH30396.03) in the medium was lowered to 0.5% to suppress cell proliferation.

After 24 hours, 10 μL of each sample was added (n = 3 to 4). The final concentrations in the medium are shown in the "Sample F.C." column of Table 2.

After a predetermined time (2 hours) from the addition, the 96-well plates were collected.

The collected 96-well plates were centrifuged at 300 g x 5 min, and the supernatant was collected.

GLP-1 in the collected supernatant was quantified with Human GLP-1 (7-36 amide) Immunoassay kit manufactured by PerkinElmer, Inc. At this time, the range of the standard in the calibration curve was 30 to 100,000 pg/mL. There was a value that caused errors with a value greater than 100,000 pg/mL, thus the value supposed to be 100,000 pg/mL or greater was calculated as 100,000 pg/mL.

Outliers ($p < 0.01$) were excluded by the Smirnov-Grubbs test.

The results are shown in Fig. 8. The amount of GLP-1 secretion in a 1% DMSO-containing solution as a non-stimulant control, and the amount of GLP-1 secretion in an aqueous solution of PMA (manufactured by FUJIFILM Wako Pure Chemical Corporation, model number: 162-23591) as a positive control are also shown in Fig. 8. The aqueous solution of PMA was obtained by adding 16.21 μL of DMSO to 1 mg of PMA to prepare a stock solution; adding PBS to

the stock solution to prepare a 1 mM solution; and diluting it with 1% DMSO-containing PBS.

Next, for confirmation, the amount of GLP-1 secretion was also measured with other plates. Specifically, the same culture supernatant as in Fig. 8 was used, and the measurement of GLP-1 amount was performed in one assay plate for Sample B to Sample D and another assay plate for Sample E. The results obtained by measuring the culture supernatant after 2 hours in the same assay plate are shown in Figs. 9 to 14. The concentrations of each sample used are shown in Figs. 9 to 14. Note that the concentrations of C, D, and E shown in Figs. 11, 13, and 14 are RebA equivalent concentrations.

The range of the calibration curve was 30 to 100,000 pg/mL, and this time there was no numerical value that would cause an error with a value greater than 100,000 pg/mL, thus values greater than 100,000 pg/mL were calculated as the theoretical value.

Table 2 is shown below. The positive comparison in Table 2 is also referred to as positive control. The negative comparison is also referred to as negative control.

Table 2

| | # | | Addition concentration | | ppm equivalent | Addition amount (μL) | PBS | Medium (μL) | Total (μL) | Sample F.C. | | ppm equivalent |
|---|---|---|---|---|---|---|---|---|---|---|---|---|
| Non-stimulant negative comparison | 1 | 1% DMSO-containing PBS | - | | - | 10 | 0 | 90 | 100 | - | | - |
| Positive comparison | 2 | PMA 1% DMSO | 0.01 | mM | - | 10 | 0 | 90 | 100 | 0.001 | mM | - |
| | 3 | | 1 | mM | - | 10 | 0 | 90 | 100 | 0.1 | mM | - |
| Test substance | 4 | B 1% DMSO | 1 | mM | 10.0 | 10 | 0 | 90 | 100 | 0.10 | mM | 1.0 |
| | 5 | | 2.5 | mM | 25.0 | 10 | 0 | 90 | 100 | 0.25 | mM | 2.5 |
| | 6 | | 5 | mM | 50.0 | 10 | 0 | 90 | 100 | 0.50 | mM | 5.0 |
| | 7 | | 10 | mM | 100.0 | 10 | 0 | 90 | 100 | 1.00 | mM | 10.0 |
| | 8 | | 15 | mM | 150.0 | 10 | 0 | 90 | 100 | 1.50 | mM | 15.0 |
| | 9 | | 20 | mM | 200.0 | 10 | 0 | 90 | 100 | 2.00 | mM | 20.0 |
| | 10 | | 25 | mM | 250.0 | 10 | 0 | 90 | 100 | 2.50 | mM | 25.0 |
| | 11 | B processed 1% DMSO | 1 | mM | 10.0 | 10 | 0 | 90 | 100 | 0.10 | mM | 1.0 |
| | 12 | | 2.5 | mM | 25.0 | 10 | 0 | 90 | 100 | 0.25 | mM | 2.5 |
| | 13 | | 5 | mM | 50.0 | 10 | 0 | 90 | 100 | 0.50 | mM | 5.0 |
| | 14 | | 10 | mM | 100.0 | 10 | 0 | 90 | 100 | 1.00 | mM | 10.0 |
| | 15 | | 15 | mM | 150.0 | 10 | 0 | 90 | 100 | 1.50 | mM | 15.0 |
| | 16 | | 20 | mM | 200.0 | 10 | 0 | 90 | 100 | 2.00 | mM | 20.0 |
| | 17 | | 25 | mM | 250.0 | 10 | 0 | 90 | 100 | 2.50 | mM | 25.0 |
| | 18 | C 1% DMSO | 1 | mM | - | 10 | 0 | 90 | 100 | 0.10 | mM | - |
| | 19 | | 2.5 | mM | - | 10 | 0 | 90 | 100 | 0.25 | mM | - |
| | 20 | | 5 | mM | - | 10 | 0 | 90 | 100 | 0.50 | mM | - |
| | 21 | | 10 | mM | - | 10 | 0 | 90 | 100 | 1.00 | mM | - |
| | 22 | | 15 | mM | - | 10 | 0 | 90 | 100 | 1.50 | mM | - |
| | 23 | | 20 | mM | - | 10 | 0 | 90 | 100 | 2.00 | mM | - |
| | 24 | | 25 | mM | - | 10 | 0 | 90 | 100 | 2.50 | mM | - |

(continued)

| | # | | Addition concentration | | ppm equivalent | Addition amount (μL) | PBS | Medium (μL) | Total (μL) | Sample F.C. | | ppm equivalent |
|---|---|---|---|---|---|---|---|---|---|---|---|---|
| | 25 | B+C 1% DMSO | 1 | mM | 10.0 | 10 | 0 | 90 | 100 | 0.10 | mM | 1.0 |
| | 26 | | 2.5 | mM | 25.0 | 10 | 0 | 90 | 100 | 0.25 | mM | 2.5 |
| | 27 | | 5 | mM | 50.0 | 10 | 0 | 90 | 100 | 0.50 | mM | 5.0 |
| | 28 | | 10 | mM | 100.0 | 10 | 0 | 90 | 100 | 1.00 | mM | 10.0 |
| | 29 | | 15 | mM | 150.0 | 10 | 0 | 90 | 100 | 1.50 | mM | 15.0 |
| | 30 | | 20 | mM | 200.0 | 10 | 0 | 90 | 100 | 2.00 | mM | 20.0 |
| | 31 | | 25 | mM | 250.0 | 10 | 0 | 90 | 100 | 2.50 | mM | 25.0 |
| | 32 | D 1% DMSO | 1 | mM | - | 10 | 0 | 90 | 100 | 0.10 | mM | - |
| | 33 | | 2.5 | mM | - | 10 | 0 | 90 | 100 | 0.25 | mM | - |
| | 34 | | 5 | mM | - | 10 | 0 | 90 | 100 | 0.50 | mM | - |
| | 35 | | 10 | mM | - | 10 | 0 | 90 | 100 | 1.00 | mM | - |
| | 36 | | 15 | mM | - | 10 | 0 | 90 | 100 | 1.50 | mM | - |
| | 37 | | 20 | mM | - | 10 | 0 | 90 | 100 | 2.00 | mM | - |
| | 38 | | 25 | mM | - | 10 | 0 | 90 | 100 | 2.50 | mM | - |
| | 39 | E 1% DMSO | 1 | mM | - | 10 | 0 | 90 | 100 | 0.10 | mM | - |
| | 40 | | 2.5 | mM | - | 10 | 0 | 90 | 100 | 0.25 | mM | - |
| | 41 | | 5 | mM | - | 10 | 0 | 90 | 100 | 0.50 | mM | - |
| | 42 | | 10 | mM | - | 10 | 0 | 90 | 100 | 1.00 | mM | - |
| | 43 | | 15 | mM | - | 10 | 0 | 90 | 100 | 1.50 | mM | - |
| | 44 | | 20 | mM | - | 10 | 0 | 90 | 100 | 2.00 | mM | - |
| | 45 | | 25 | mM | - | 10 | 0 | 90 | 100 | 2.50 | mM | - |

<Experimental Example 15>

To 36.3 mg of RebA, 9.4 μL of DMSO (manufactured by FUJIFILM Wako Pure Chemical Corporation, model number: 037-24053) was added followed by an addition of 929.0 μL of PBS. The mixture was then stirred by vortex to obtain a stock solution having a RebA concentration of 40 mM.

The obtained stock solution and PBS were diluted with 1% DMSO-containing PBS to obtain Sample B-0.31 mM to Sample B-20 mM. The concentrations of RebA are described in Table 3.

<Experimental Example 16>

An aqueous mixed solution was prepared by mixing 490 g of glycerol, 100 g of RebA, and 10 g of polyglycerol fatty acid ester, and heating and dissolving the mixture at 70°C. To the aqueous mixed solution, 300 g of MCT oil was added as a fat and oil, and the mixture was emulsified at a rotation rate of 8000 rpm with a homo-mixer (manufactured by Tokushu Kika Kogyo Co., Ltd.). After completion of the stirring, the mixture was cooled to 40°C, and 100 g of water was added to obtain a processed RebA solution.

To 1.4 μL of the obtained processed RebA solution, 10 μL of DMSO (manufactured by FUJIFILM Wako Pure Chemical Corporation, model number: 037-24053) and 796.6 μL of PBS were added. The mixture was stirred by vortex to obtain a stock solution having a RebA concentration of 20 mM.

The stock solution was diluted as needed with 1% DMSO-containing PBS to obtain Sample B processed-0.31 mM to Sample B processed-20 mM. The concentrations of RebA are described in Table 3.

<Experimental Example 17>

The solution C was obtained in the same manner as the preparation of the processed RebA solution in Experimental Example 16 except that 100 g of RebA was not used and 490 g of glycerol, 10 g of polyglycerol fatty acid ester, 300 g of MCT oil, and 200 g of water were used.

To 386.8 μL of solution C, 10 μL of DMSO (manufactured by FUJIFILM Wako Pure Chemical Corporation, model number: 037-24053) and 603.2 μL of PBS were added. The mixture was stirred by vortex to obtain a stock solution having a RebA equivalent concentration of 40 mM.

The stock solution was diluted with 1% DMSO-containing PBS to obtain Sample C-0.31 mM to Sample C-20 mM. Table 3 describes RebA equivalent concentrations. The RebA equivalent concentration refers to a concentration of RebA in a given Sample B processed of Experimental Example 16 when concentrations of polyglycerol fatty acid ester in a given Sample C and concentrations of polyglycerol fatty acid ester in the given Sample B processed are the same.

<Experimental Example 18>

In the same manner as in Experimental Example 15, Sample B containing RebA at twice the concentration in Sample B + C was obtained. That is, Sample B-0.62 mM, Sample B-2.5 mM, Sample B-10 mM, and Sample B-40 mM were obtained. The concentration behind the hyphens indicates the concentration of RebA.

In the same manner as in Experimental Example 17, Sample C containing polyglycerol fatty acid ester at twice the concentration in Sample B + C was obtained. That is, Sample C-0.62 mM, Sample C-2.5 mM, Sample C-10 mM, and Sample C-40 mM were obtained. The concentration behind the hyphens indicates the RebA equivalent concentration.

Equal amounts of Sample B and Sample C at the same concentration (e.g., Sample B-0.62 mM and Sample C-0.62 mM) were mixed to obtain Sample B + C-0.31 mM to Sample B + C-20mM. The concentrations of RebA are described in Table 3.

<Experimental Example 19>

To 94.8 μL of glycerol, 10 μL of DMSO (manufactured by FUJIFILM Wako Pure Chemical Corporation, model number: 037-24053) and 895 μL of PBS were added. The mixture was stirred by vortex to obtain a stock solution having a RebA equivalent concentration of 20 mM.

The obtained stock solution was diluted as needed with 1% DMSO-containing PBS to obtain Sample D-0.31 mM to Sample D-20 mM. Table 3 describes the RebA equivalent concentrations. The RebA equivalent concentration refers to a concentration of RebA in a given Sample B processed of Experimental Example 16 when the concentration of glycerol in a given Sample D and the concentrations of glycerol in the given Sample B processed are the same.

<Experimental Example 20>

To 58.0 μL of MCT oil, 10 μL of DMSO (manufactured by FUJIFILM Wako Pure Chemical Corporation, model number: 037-24053) and 932.0 μL of PBS were added. The mixture was stirred by vortex to obtain a stock solution having a RebA equivalent concentration of 20 mM.

The obtained stock solution was diluted as needed with 1% DMSO-containing PBS to obtain Sample E-0.31 mM to Sample E-20 mM. Table 3 describes the RebA equivalent concentrations. The RebA equivalent concentration refers to a concentration of RebA in a given Sample B processed of Experimental Example 16 when the concentration of MCT oil in a given Sample E and the concentrations of MCT oil in the given Sample B processed are the same.

<Cytotoxicity>

Under the conditions shown in Table 3, the samples obtained in Experimental Examples 15 to 20 above were added to a medium. In the obtained medium, cells were cultured, and the cytotoxicity after 2 hours and 24 hours was measured. Specific procedures were as follows.

H716 cells were cultured in RPMI 1640 (manufactured by Thermo Fisher Scientific Inc., model number 61870-036) (10% FBS (manufactured by Gibco, model number SH102770), 1 mM sodium pyruvate) in an incubator at 37°C, 5%$CO_2$. H716 cells were seeded in 96-well plates at 2 x $10^5$ cells/90 μL/well (for dead cell evaluation) and in 48-well plates at 4 x $10^5$ cells/180 μL/well (for morphological observation). The content of FBS in the medium when the cells were seeded in the plate was 0.5%. After 24 hours from the cell seeding, the sample at each concentration was added at 10 μL/well (96 well plates) or 20 μL/well (48 well plates). The final concentrations in the medium are shown in the "Sample F.C." column of Table 3. After two hours from the sample addition, each sample at the highest concentration (#1, #6, #10, #14, #18, #22, #26, #30 in Table 3) was evaluated.

(Evaluation using dead cells as indicator)

After 2 hours and 24 hours from the sample addition, the cells were centrifuged at 100 x g for 5 minutes, and the supernatant was removed. Propidium iodide solution (0.66 μg/mL, 1:1500 dilution in PBS) was added by 50 μL/well, and the cells were allowed to stand at room temperature for 15 minutes to stain dead cells. The cells were suspended by pipetting, and then phase-contrast images and fluorescence images were taken under a microscope. The total number of cells was counted based on the phase-contrast images with ImageJ, and the number of dead cells was counted based on the fluorescence images. The dead cell ratio was calculated with the counted number of cells.

$$\text{Dead cell ratio (\%)} = \text{number of dead cells/total number of cells} \times 100$$

The dead cell ratio at the time of sample addition each was corrected by taking the dead cell ratio after 24 hours from the cell seeding without no sample addition as 0%, and the dead cell ratio when the cells were allowed to stand on ice for 30 minutes or more under 70% ethanol addition as 100%. The results are shown in Fig. 15. Fig. 15a shows the results after 2 hours, and Fig. 15b shows the results after 24 hours.

It can be seen from Fig. 15a showing the results after 2 hours that no cell death was induced by all samples.

Also, as can be seen from Fig. 15b showing the results after 24 hours, the dead cell ratio was the highest when Sample B + C (RebA concentration 2 mM) was added, but it was the same level as the lowest concentration of PMA. Samples other than Sample B + C had the dead cell ratio equal to or lower than the negative comparison (-) (also referred to as a negative control). Thus, it was unlikely that cell death was induced by Sample B + C, and no cell death was induced by other samples.

(Evaluation using cellular morphology as indicator)

After 2 hours and 24 hours from the sample addition, the cells were photographed under a microscope, and the presence or absence of morphological change was examined. Since Sample B processed, Sample C and Sample B + C were turbid, the determination under a microscope at their 0.5 mM and 2 mM additions was difficult. However, when other samples were added to H716 cells, no morphological changes such as cell contraction or rupture were seen.

Table 3 is shown below. The positive comparison in Table 3 is also referred to as positive control. The negative comparison is also referred to as negative control.

Table 3

| | # | Test substance | Addition concentration | | ppm equivalent | Sample F.C. | | ppm equivalent |
|---|---|---|---|---|---|---|---|---|
| Non-stimulant negative comparison | 1 | 1% DMSO-containing solution | - | | - | - | | - |
| Positive comparison | 2 | PMA 1%DMSO | 0.004 | mM | - | 0.0004 | mM | - |
| | 3 | | 0.016 | mM | - | 0.0016 | mM | - |
| | 4 | | 0.063 | mM | - | 0.0063 | mM | - |
| | 5 | | 0.25 | mM | - | 0.025 | mM | - |
| | 6 | | 1 | mM | - | 0.1 | mM | - |
| Test substance | 7 | B 1%DMSO | 0.31 | mM | 3.1 | 0.03 | mM | 0.3 |
| | 8 | | 1.25 | mM | 12.5 | 0.13 | mM | 1.3 |
| | 9 | | 5 | mM | 50.0 | 0.50 | mM | 5.0 |
| | 10 | | 20 | mM | 200.0 | 2.00 | mM | 20.0 |
| | 11 | B processed 1%DMSO | 0.31 | mM | 3.1 | 0.03 | mM | 0.3 |
| | 12 | | 1.25 | mM | 12.5 | 0.13 | mM | 1.3 |
| | 13 | | 5 | mM | 50.0 | 0.50 | mM | 5.0 |
| | 14 | | 20 | mM | 200.0 | 2.00 | mM | 20.0 |
| | 15 | C 1%DMSO | 0.31 | mM | - | 0.03 | mM | - |
| | 16 | | 1.25 | mM | - | 0.13 | mM | - |
| | 17 | | 5 | mM | - | 0.50 | mM | - |
| | 18 | | 20 | mM | - | 2.00 | mM | - |
| | 19 | B + C 1%DMSO | 0.31 | mM | 3.1 | 0.03 | mM | 0.3 |
| | 20 | | 1.25 | mM | 12.5 | 0.13 | mM | 1.3 |
| | 21 | | 5 | mM | 50.0 | 0.50 | mM | 5.0 |
| | 22 | | 20 | mM | 200.0 | 2.00 | mM | 20.0 |
| | 23 | D 1%DMSO | 0.31 | mM | - | 0.03 | mM | - |
| | 24 | | 1.25 | mM | - | 0.13 | mM | - |
| | 25 | | 5 | mM | - | 0.50 | mM | - |
| | 26 | | 20 | mM | - | 2.00 | mM | - |
| | 27 | E 1%DMSO | 0.31 | mM | - | 0.03 | mM | - |
| | 28 | | 1.25 | mM | - | 0.13 | mM | - |
| | 29 | | 5 | mM | - | 0.50 | mM | - |
| | 30 | | 20 | mM | - | 2.00 | mM | - |

<Experimental Example 21>

To 134.3 mg of RebA, 34.7 μL of DMSO (manufactured by FUJIFILM Wako Pure Chemical Corporation, model number: 037-24053) was added followed by an addition of 3438 μL of PBS. The mixture was then stirred by vortex to obtain a stock solution having a RebA concentration of 40 mM.

The obtained stock solution are diluted with 1% DMSO-containing PBS to obtain Sample B-1 mM to Sample B-20 mM. The concentrations of RebA are described in Table 4.

<Experimental Example 22>

The C-1 solution was obtained in the same manner as the preparation of the processed RebA solution in Experimental Example 16 except that 100 g of RebA was replaced with 100 g of water, and 10 g of sucrose fatty acid ester was used instead of 10 g of polyglycerol fatty acid ester as the emulsifier. Specifically, an aqueous mixed solution was prepared by mixing 490 g of glycerol, and 10 g of sucrose fatty acid ester (manufactured by Mitsubishi-Chemical Foods Corporation, HLB = 16), and heating and dissolving the mixture at 70°C. To the aqueous mixed solution, 300 g of MCT oil was added as a fat and oil, and the mixture was emulsified at a rotation rate of 8000 rpm with a homo-mixer (manufactured by Tokushu Kika Kogyo Co., Ltd.). After completion of the stirring, the mixture was cooled to 40°C, and 200 g of water was added to obtain the solution C-1.

To 580.2 μL of the obtained solution C-1, 15 μL of DMSO (manufactured by FUJIFILM Wako Pure Chemical Corporation, model number: 037-24053) and 904.8 μL of PBS were added. The mixture was stirred by vortex to obtain a stock solution having a RebA equivalent concentration of 40 mM.

The obtained stock solution was diluted with 1% DMSO-containing PBS to obtain Sample C-1-1 mM to Sample C-1-20 mM. Table 4 describes RebA equivalent concentrations. The RebA equivalent concentration refers to a concentration of RebA in a given Sample B+C-1 of Experimental Example 25 when concentrations of glycerol and emulsifier in a given Sample C-1 and concentrations of glycerol and emulsifier in the given Sample B+C-1 are the same.

<Experimental Example 23>

Sample C-2-1 mM to Sample C-2-20 mM were obtained in the same manner as in Experimental Example 22 except that 10 g of enzymatically digested lecithin (manufactured by Taiyo Kagaku Co., Ltd., HLB = 12.0) was used instead of 10 g of sucrose fatty acid ester (manufactured by Mitsubishi-Chemical Foods Corporation, HLB = 16). Table 4 describes RebA equivalent concentrations. The RebA equivalent concentration refers to a concentration of RebA in a given Sample B+C-2 of Experimental Example 26 when concentrations of glycerol and emulsifier in a given Sample C-2 and concentrations of glycerol and emulsifier in the given Sample B+C-2 are the same.

<Experimental Example 24>

Sample C-3-1 mM to Sample C-3-20 mM were obtained in the same manner as in Experimental Example 22 except that 10 g of organic acid monoglyceride (manufactured by Taiyo Kagaku Co., Ltd., HLB = 9.0) was used instead of 10 g of sucrose fatty acid ester (manufactured by Mitsubishi-Chemical Foods Corporation, HLB = 16). Table 4 describes RebA equivalent concentrations. The RebA equivalent concentration refers to a concentration of RebA in a given Sample B+C-3 of Experimental Example 27 when concentrations of glycerol and emulsifier in a given Sample C-3 and concentrations of glycerol and emulsifier in the given Sample B+C-3 are the same.

<Experimental Example 25>

In the same manner as in Experimental Example 21, Sample B containing RebA at twice the concentration in Sample B + C-1 was obtained. That is, Sample B-2 mM, Sample B-5 mM, Sample B-10 mM, Sample B-20 mM, Sample B-30 mM, and Sample B-40 mM were obtained. The concentration behind the hyphens indicates the concentration of RebA.

In the same manner as in Experimental Example 22, Sample C-1 containing sucrose fatty acid ester at twice the concentration in Sample B + C-1 was obtained. That is, Sample C-1-2 mM, Sample C-1-5 mM, Sample C-1-10 mM, Sample C-1-20 mM, Sample C-1-30 mM, and Sample C-1-40 mM were obtained. The concentration behind the hyphens indicates the RebA equivalent concentration.

Equal amounts of Sample B and Sample C at the same concentration (e.g., Sample B-1-2 mM and Sample C-1-2 mM) were mixed to obtain Sample B + C-1-1 mM to Sample B + C-1-20mM. The concentrations of RebA are described in Table 4.

<Experimental Example 26>

Sample B+C-2-1 mM to Sample B+C-2-20 mM were obtained in the same manner as in Experimental Example 25 except that 10 g of enzymatically digested lecithin (manufactured by Taiyo Kagaku Co., Ltd., HLB = 12.0) was used instead of 10 g of sucrose fatty acid ester (manufactured by Mitsubishi-Chemical Foods Corporation, HLB = 16). The concentrations of RebA are described in Table 4.

<Experimental Example 27>

Sample B+C-3-1 mM to Sample B+C-3-20 mM were obtained in the same manner as in Experimental Example 25 except that 10 g of organic acid monoglyceride (manufactured by Taiyo Kagaku Co., Ltd., HLB = 9.0) was used instead of 10 g of sucrose fatty acid ester (manufactured by Mitsubishi-Chemical Foods Corporation, HLB = 16). The concentrations of RebA are described in Table 4.

<Experimental Example 28>

To 13.1 mg of sucrose fatty acid ester, 339 μL of DMSO (manufactured by FUJIFILM Wako Pure Chemical Corporation, model number: 037-24053) was added. The mixture was stirred by vortex and diluted 20 times with DMSO to obtain a stock solution having a RebA equivalent concentration of 2,000 mM. To the obtained stock solution, PBS was added to obtain a 20 mM solution.

The obtained 20 mM solution was diluted as needed with 1% DMSO-containing PBS to obtain Sample G-1-1 mM to Sample G-1-20 mM. Table 4 described the RebA equivalent concentrations. The RebA equivalent concentration refers to a concentration of RebA in a given Sample B + C-1 of Experimental Example 25 when the concentration of sucrose fatty acid ester in a given Sample G-1 and the concentration of sucrose fatty acid ester in the given Sample B + C-1 are the same.

<Experimental Example 29>

To 20.9 mg of enzymatically digested lecithin, 540 μL of PBS was added. The mixture was stirred by vortex and diluted 20 times with DMSO (manufactured by FUJIFILM Wako Pure Chemical Corporation, model number: 037-24053) to obtain a stock solution having a RebA equivalent concentration of 2,000 mM. To the obtained stock solution, PBS and DMSO were added, and a 20 mM solution (1% DMSO) was prepared.

The obtained 20 mM solution was diluted as needed with 1% DMSO-containing PBS to obtain Sample G-2-1 mM to Sample G-2-20 mM. Table 4 described the RebA equivalent concentrations. The RebA equivalent concentration refers to a concentration of RebA in a given Sample B + C-2 of Experimental Example 26 when the concentration of enzymatically digested lecithin in a given Sample G-2 and the concentration of enzymatically digested lecithin in the given Sample B + C-2 are the same.

<Experimental Example 30>

Organic acid monoglyceride was dissolved at 60°C, and 905 μL of DMSO was added to 35.0 mg of the dissolved organic acid monoglyceride. The mixture was stirred by vortex and diluted 20 times with DMSO to obtain a stock solution having a RebA equivalent concentration of 2,000 mM. To the obtained stock solution, PBS was added to obtain a 20 mM solution.

The obtained 20 mM solution was diluted as needed with 1% DMSO-containing PBS to obtain Sample G-3-1 mM to Sample G-3-20 mM. Table 4 described the RebA equivalent concentrations. The RebA equivalent concentration refers to a concentration of RebA in a given Sample B + C-3 of Experimental Example 27 when the concentration of organic acid monoglyceride in a given Sample G-3 and the concentration of organic acid monoglyceride in the given Sample B + C-3 are the same.

<Experimental Example 31>

Polyglycerol fatty acid ester was dissolved at 60°C, and 259 μL of DMSO (manufactured by FUJIFILM Wako Pure Chemical Corporation, model number: 037-24053) was added to 50 μL of the dissolved polyglycerol fatty acid ester followed by an addition of 984 μL of PBS. The mixture was stirred by vortex and diluted 20 times with DMSO to obtain a stock solution having a RebA equivalent concentration of 400 mM.

The obtained stock solution and PBS were used to prepare a 20 mM solution. The obtained 20 mM solution was diluted as needed with 1% DMSO-containing PBS to obtain Sample G-4-1 mM to Sample G-4-20 mM. Table 4 described the RebA equivalent concentrations. The RebA equivalent concentration refers to a concentration of RebA in a given Sample B + C of Experimental Examples 25-27 when the concentration of polyglycerol fatty acid ester in a given Sample G-4 and the concentration of emulsifier in the given Sample B + C are the same.

<Amount of GLP-1 secretion>

Each of the samples obtained in Experimental Examples 21 to 31 above was administered to a cell, and the

amount of GLP-1 secretion after 2 hours and 24 hours was measured. The specific procedures are as follows.

H716 cells were cultured in RPMI 1640 (Thermo Fisher Scientific Inc., model number: 61870-036) (10% FBS (manufactured by Gibco, model number SH102770), 1 mM Sodium Pyruvate) in an incubator at 37°C, 5%$CO_2$. H716 cells were seeded in 96-well plates at 2 x $10^5$ cells/90 μL/well. The content of FBS in the medium when the cells were seeded in the plate was 0.5%.

After 24 hours from the cell seeding, the samples obtained in Experimental Examples 21 to 31 as shown in Table 4 were added to the medium by 10 μL/well. The final concentrations in the medium are shown in the "Sample F.C." column of Table 4.

After 2 hours and 24 hours from the sample addition, the 96-well plate was centrifuged at 300 x g for 5 minutes, and the culture supernatant was collected.

GLP-1 in the collected culture supernatant was quantified with Human GLP-1 (7-36 amide) Immunoassay kit (manufactured by PerkinElmer, Inc., model number: AL359C). For quantitative values, a value that was beyond the range of the calibration curve and caused an error was excluded. Furthermore, a box plot was created with a statistical analysis software R, and when a value less than the first quartile or greater than the third quartile was at or beyond the 1.5 quartile range, the value was excluded as an outlier.

The results are shown in Fig. 16. The amount of GLP-1 secretion in a 1% DMSO-containing solution as a non-stimulant control, and the amount of GLP-1 secretion in an aqueous solution of PMA (manufactured by FUJIFILM Wako Pure Chemical Corporation, 162-23591) as a positive control are also shown in Fig.16. The aqueous solution of PMA was obtained by adding 16.21 μL of DMSO to 1 mg of PMA to prepare a stock solution; adding PBS to the stock solution to prepare a 1 mM solution; and diluting it with 1% DMSO-containing PBS.

Table 4 is shown below. The positive comparison in Table 4 is also referred to as positive control. The negative comparison is also referred to as negative control.

Table 4

| | # | Addition concentration | | | ppm equivalent | Addition amount (μL) | Medium (μL) | Total (μL) | Sample F.C. | |
|---|---|---|---|---|---|---|---|---|---|---|
| Non-stimulant negative comparison | 1 | 1%DMSO-containing PBS | - | | - | 10 | 90 | 100 | - | |
| Positive comparison | 2 | PMA (1%DMSO) | 0.01 | mM | - | 10 | 90 | 100 | 0.001 | mM |
| | 3 | | 1 | mM | - | 10 | 90 | 100 | 0.1 | mM |
| Test substance | 4 | B (1%DMSO) | 1 | mM | 967.0 | 10 | 90 | 100 | 0.10 | mM |
| | 5 | | 2.5 | mM | 2417.5 | 10 | 90 | 100 | 0.25 | mM |
| | 6 | | 5 | mM | 4835.1 | 10 | 90 | 100 | 0.50 | mM |
| | 7 | | 10 | mM | 9670.1 | 10 | 90 | 100 | 1.00 | mM |
| | 8 | | 15 | mM | 14505.2 | 10 | 90 | 100 | 1.50 | mM |
| | 9 | | 20 | mM | 19340.2 | 10 | 90 | 100 | 2.00 | mM |
| | 10 | C-1 (1%DMSO) | 1 | mM | 967.0 | 10 | 90 | 100 | 0.10 | mM |
| | 11 | | 2.5 | mM | 2417.5 | 10 | 90 | 100 | 0.25 | mM |
| | 12 | | 5 | mM | 4835.1 | 10 | 90 | 100 | 0.50 | mM |
| | 13 | | 10 | mM | 9670.1 | 10 | 90 | 100 | 1.00 | mM |
| | 14 | | 15 | mM | 14505.2 | 10 | 90 | 100 | 1.50 | mM |
| | 15 | | 20 | mM | 19340.2 | 10 | 90 | 100 | 2.00 | mM |
| | 16 | C-2 (1%DMSO) | 1 | mM | 967.0 | 10 | 90 | 100 | 0.10 | mM |
| | 17 | | 2.5 | mM | 2417.5 | 10 | 90 | 100 | 0.25 | mM |
| | 18 | | 5 | mM | 4835.1 | 10 | 90 | 100 | 0.50 | mM |
| | 19 | | 10 | mM | 9670.1 | 10 | 90 | 100 | 1.00 | mM |
| | 20 | | 15 | mM | 14505.2 | 10 | 90 | 100 | 1.50 | mM |
| | 21 | | 20 | mM | 19340.2 | 10 | 90 | 100 | 2.00 | mM |
| | 22 | | 1 | mM | 967.0 | 10 | 90 | 100 | 0.10 | mM |

(continued)

| | # | Addition concentration | | | ppm equivalent | Addition amount (μL) | Medium (μL) | Total (μL) | Sample F.C. | |
|---|---|---|---|---|---|---|---|---|---|---|
| | 23 | C-3 (1%DMSO) | 2.5 | mM | 2417.5 | 10 | 90 | 100 | 0.25 | mM |
| | 24 | | 5 | mM | 4835.1 | 10 | 90 | 100 | 0.50 | mM |
| | 25 | | 10 | mM | 9670.1 | 10 | 90 | 100 | 1.00 | mM |
| | 26 | | 15 | mM | 14505.2 | 10 | 90 | 100 | 1.50 | mM |
| | 27 | | 20 | mM | 19340.2 | 10 | 90 | 100 | 2.00 | mM |
| | 28 | B + C-1 (1%DMSO) | 1 | mM | 967.0 | 10 | 90 | 100 | 0.10 | mM |
| | 29 | | 2.5 | mM | 2417.5 | 10 | 90 | 100 | 0.25 | mM |
| | 30 | | 5 | mM | 4835.1 | 10 | 90 | 100 | 0.50 | mM |
| | 31 | | 10 | mM | 9670.1 | 10 | 90 | 100 | 1.00 | mM |
| | 32 | | 15 | mM | 14505.2 | 10 | 90 | 100 | 1.50 | mM |
| | 33 | | 20 | mM | 19340.2 | 10 | 90 | 100 | 2.00 | mM |
| | 34 | B + C-2 (1%DMSO) | 1 | mM | 967.0 | 10 | 90 | 100 | 0.10 | mM |
| | 35 | | 2.5 | mM | 2417.5 | 10 | 90 | 100 | 0.25 | mM |
| | 36 | | 5 | mM | 4835.1 | 10 | 90 | 100 | 0.50 | mM |
| | 37 | | 10 | mM | 9670.1 | 10 | 90 | 100 | 1.00 | mM |
| | 38 | | 15 | mM | 14505.2 | 10 | 90 | 100 | 1.50 | mM |
| | 39 | | 20 | mM | 19340.2 | 10 | 90 | 100 | 2.00 | mM |
| | 40 | B + C-3 (1%DMSO) | 1 | mM | 967.0 | 10 | 90 | 100 | 0.10 | mM |
| | 41 | | 2.5 | mM | 2417.5 | 10 | 90 | 100 | 0.25 | mM |
| | 42 | | 5 | mM | 4835.1 | 10 | 90 | 100 | 0.50 | mM |
| | 43 | | 10 | mM | 9670.1 | 10 | 90 | 100 | 1.00 | mM |
| | 44 | | 15 | mM | 14505.2 | 10 | 90 | 100 | 1.50 | mM |
| | 45 | | 20 | mM | 19340.2 | 10 | 90 | 100 | 2.00 | mM |
| | 46 | G-1 (1%DMSO) | 1 | mM | 967.0 | 10 | 90 | 100 | 0.10 | mM |
| | 47 | | 2.5 | mM | 2417.5 | 10 | 90 | 100 | 0.25 | mM |
| | 48 | | 5 | mM | 4835.1 | 10 | 90 | 100 | 0.50 | mM |
| | 49 | | 10 | mM | 9670.1 | 10 | 90 | 100 | 1.00 | mM |
| | 50 | | 15 | mM | 14505.2 | 10 | 90 | 100 | 1.50 | mM |
| | 51 | | 20 | mM | 19340.2 | 10 | 90 | 100 | 2.00 | mM |
| | 52 | G-2 (1%DMSO) | 1 | mM | 967.0 | 10 | 90 | 100 | 0.10 | mM |
| | 53 | | 2.5 | mM | 2417.5 | 10 | 90 | 100 | 0.25 | mM |
| | 54 | | 5 | mM | 4835.1 | 10 | 90 | 100 | 0.50 | mM |
| | 55 | | 10 | mM | 9670.1 | 10 | 90 | 100 | 1.00 | mM |
| | 56 | | 15 | mM | 14505.2 | 10 | 90 | 100 | 1.50 | mM |
| | 57 | | 20 | mM | 19340.2 | 10 | 90 | 100 | 2.00 | mM |
| | 58 | G-3 (1%DMSO) | 1 | mM | 967.0 | 10 | 90 | 100 | 0.10 | mM |
| | 59 | | 2.5 | mM | 2417.5 | 10 | 90 | 100 | 0.25 | mM |
| | 60 | | 5 | mM | 4835.1 | 10 | 90 | 100 | 0.50 | mM |
| | 61 | | 10 | mM | 9670.1 | 10 | 90 | 100 | 1.00 | mM |
| | 62 | | 15 | mM | 14505.2 | 10 | 90 | 100 | 1.50 | mM |
| | 63 | | 20 | mM | 19340.2 | 10 | 90 | 100 | 2.00 | mM |
| | 64 | G-4 (1%DMSO) | 1 | mM | 967.0 | 10 | 90 | 100 | 0.10 | mM |
| | 65 | | 2.5 | mM | 2417.5 | 10 | 90 | 100 | 0.25 | mM |
| | 66 | | 5 | mM | 4835.1 | 10 | 90 | 100 | 0.50 | mM |
| | 67 | | 10 | mM | 9670.1 | 10 | 90 | 100 | 1.00 | mM |
| | 68 | | 15 | mM | 14505.2 | 10 | 90 | 100 | 1.50 | mM |

(continued)

| | # | Addition concentration | | | ppm equivalent | Addition amount (μL) | Medium (μL) | Total (μL) | Sample F.C. | |
|---|---|---|---|---|---|---|---|---|---|---|
| | 69 | | 20 | mM | 19340.2 | 10 | 90 | 100 | 2.00 | mM |
| * The concentration of #10 to #69 is indicated as the concentration of Sample B processed. | | | | | | | | | | |

<Experimental Example 32>

The distribution ratios of stevioside, RebA, RebD, and RebM were measured. For reference, structural formulae of stevioside, RebA, RebD, and RebM are shown below.

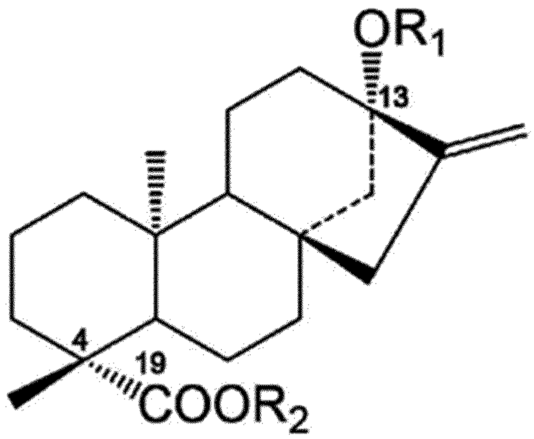

| Compound Name | $R_1$ | $R_2$ |
|---|---|---|
| Rebaudioside A (rebA) | Glcβ1,2(Gluβ1,3)Gluβ1- | Glcβ1- |
| Stevioside (stv) | Glcβ1,2Gluβ1- | Glcβ1- |
| Rebaudioside D (rebD) | Glcβ1,2(Gluβ1,3)Gluβ1- | Glcβ1,2Glcβ1- |
| Rebaudioside M (rebM) | Glcβ1,2(Gluβ1,3)Gluβ1- | Glcβ1,2(Gluβ1,3)Gluβ1- |

Specific procedures of the measurement were as follows.

After each about 100 mL of pure water and toluene was charged into a separatory funnel, 30 mg of each steviol glycoside was added. The separatory funnel was sealed to avoid liquid leakage, and stirred with shaking using a shaker for about 30 minutes, and then allowed to stand. When the liquid layer was separated into two layers, the distribution ratio was measured by LCMS after sampling was performed from each layer.

The measurement results and photos during the test are shown in Fig. 17. The photos of the RebA-containing solution after the test are shown in Fig. 18.

<Experimental Example 33>

1. Sample Preparation

Samples SA-70A, SA-70B, SA-200A, SA-200B, SA-206A, SA-206B, SA-207A and SA-207B were prepared by the following procedures. Note that the sample having a name ending with A is a sample prepared by dissolving RebA in an aqueous system. The sample having a name ending with B is a sample prepared by dispersing RebA into an oil system.

(SA-70A)

An aqueous mixed solution was obtained by mixing 487 g of glycerol, 103 g of RebA, and 10 g of polyglycerol fatty acid ester (manufactured by Taiyo Kagaku Co., Ltd., HLB = 16.7), and heating and dissolving the mixture at 70°C. To the aqueous mixed solution, 300 g of MCT was added as a fat and oil, and the mixture was emulsified at a rotation rate of 8000 rpm with a homo-mixer (manufactured by Tokushu Kika Kogyo Co., Ltd.). After completion of the stirring, the mixture was cooled to 40°C, and 100 g of water was added to obtain the emulsified composition SA-70A.

(SA-70B)

An aqueous mixed solution was obtained by mixing 487 g of glycerol and 10 g of polyglycerol fatty acid ester (manufactured by Taiyo Kagaku Co., Ltd., HLB = 16.7), and heating and dissolving the mixture at 70°C. An oily mixed solution was obtained by mixing 300 g of MCT and 103 g of RebA at room temperature. To the aqueous mixed solution, the oily mixed solution was added, and the mixture was emulsified at a rotation rate of 8000 rpm with a homo-mixer (manufactured by Tokushu Kika Kogyo Co., Ltd.). After completion of the stirring, the mixture was cooled to 40°C, and 100 g of water was added to obtain the emulsified composition SA-70B.

(SA-200A)

An aqueous mixed solution was prepared by mixing 567 g of glycerol, 103 g of RebA, and 30 g of polyglycerol fatty acid ester (manufactured by Taiyo Kagaku Co., Ltd., HLB = 16.7), and heating and dissolving the mixture at 70°C. To the aqueous mixed solution, 200 g of MCT was added as a fat and oil, and the mixture was emulsified at a rotation rate of 8000 rpm with a homo-mixer (manufactured by Tokushu Kika Kogyo Co., Ltd.). After completion of the stirring, the mixture was cooled to 40°C, and 100 g of water was added to obtain the emulsified composition SA-200A.

(SA-200B)

An aqueous mixed solution was prepared by mixing 567 g of glycerol and 30 g of polyglycerol fatty acid ester (manufactured by Taiyo Kagaku Co., Ltd., HLB = 16.7), and heating and dissolving the mixture at 70°C. An oily mixed solution was obtained by mixing 200 g of MCT and 103 g of RebA at room temperature. To the aqueous mixed solution, the oily mixed solution was added, and the mixture was emulsified at a rotation rate of 8000 rpm with a homo-mixer (manufactured by Tokushu Kika Kogyo Co., Ltd.). After completion of the stirring, the mixture was cooled to 40°C, and 100 g of water was added to obtain the emulsified composition SA-200B.

(SA-206A)

An aqueous mixed solution was prepared by mixing 537 g of glycerol, 103 g of RebA, and 60 g of polyglycerol fatty acid ester (manufactured by Taiyo Kagaku Co., Ltd., HLB = 16.7), and heating and dissolving the mixture at 70°C. To the aqueous mixed solution, 200 g of MCT was added as a fat and oil, and the mixture was emulsified at a rotation rate of 8000 rpm with a homo-mixer (manufactured by Tokushu Kika Kogyo Co., Ltd.). After completion of the stirring, the mixture was cooled to 40°C, and 100 g of water was added to obtain a pre-emulsified composition. The pre-emulsified composition was then subjected to fine emulsification at a pressure of 150 MPa with a wet-type atomizer to obtain the emulsified composition SA-206A.

(SA-206B)

An aqueous mixed solution was prepared by mixing 578 g of glycerol and 55 g of polyglycerol fatty acid ester (manufactured by Taiyo Kagaku Co., Ltd., HLB = 16.7), and heating and dissolving the mixture at 70°C. An oily mixed solution was obtained by mixing 182 g of MCT and 94 g of RebA at room temperature. To the aqueous mixed solution, the oily mixed solution was added, and the mixture was emulsified at a rotation rate of 8000 rpm with a homo-mixer (manufactured by Tokushu Kika Kogyo Co., Ltd.). After completion of the stirring, the mixture was cooled to 40°C, and 91 g of water was added to obtain a pre-emulsified composition. The pre-emulsified composition was then subjected to fine emulsification at a pressure of 150 MPa with a wet-type atomizer to obtain the emulsified composition SA-206B.

(SA-207A)

An aqueous mixed solution was prepared by mixing 706 g of glycerol, 34 g of RebA, and 60 g of polyglycerol fatty acid ester (manufactured by Taiyo Kagaku Co., Ltd., HLB = 16.7), and heating and dissolving the mixture at 70°C.

To the aqueous mixed solution, 100 g of MCT was added as a fat and oil, and the mixture was emulsified at a rotation rate of 8000 rpm with a homo-mixer (manufactured by Tokushu Kika Kogyo Co., Ltd.). After completion of the stirring, the mixture was cooled to 40°C, and 100 g of water was added to obtain a pre-emulsified composition. The pre-emulsified composition was then subjected to fine emulsification at a pressure of 150 MPa with a wet-type atomizer to obtain the emulsified composition SA-207A.

(SA-207B)

An aqueous mixed solution was prepared by mixing 706 g of glycerol and 60 g of polyglycerol fatty acid ester (manufactured by Taiyo Kagaku Co., Ltd., HLB = 16.7), and heating and dissolving the mixture at 70°C. An oily mixed solution was obtained by mixing 100 g of MCT and 34 g of RebA at room temperature. To the aqueous mixed solution, the oily mixed solution was added, and the mixture was emulsified at a rotation rate of 8000 rpm with a homo-mixer (manufactured by Tokushu Kika Kogyo Co., Ltd.). After completion of the stirring, the mixture was cooled to 40°C, and 100 g of water was added to obtain a pre-emulsified composition. The pre-emulsified composition was then subjected to fine emulsification at a pressure of 150 MPa with a wet-type atomizer to obtain the emulsified composition SA-207B.

2. Evaluation of emulsion stability

The stability was evaluated using the samples prepared in 1. above. Specific procedures of the evaluation were as follows.

2.1 Measurement of particle size of emulsified particles immediately after preparation

The average particle size of the emulsified particles in each sample immediately after preparation was measured. Specifically, each sample immediately after preparation was appropriately diluted with ion exchange water so that the laser scattering intensity was around 1%, and then the particle size was measured with a laser diffraction/scattering type particle size distribution meter (Spectris Co., Ltd., Malvern Panalytical).

2.2 Measurement of particle size of emulsified particles after refrigerated storage

Each sample was allowed to stand in a refrigerator at 4°C for 2 months. Then, the particle size of the emulsified particles was measured in the same manner as in 2.1.

The results are shown in the table below.

Table 5

| (%) | SA-70 | | SA-200 | | SA-206 | | SA-207 | |
|---|---|---|---|---|---|---|---|---|
| | A | B | A | B | A | B | A | B |
| **RebA** | 10.3 | | 10.3 | | 10.3 | 9.4 | 3.4 | |
| **MCT** | 30.0 | | 20.0 | | 20.0 | 18.2 | 10.0 | |
| **Emulsifier** | 1.0 | | 3.0 | | 6.0 | 5.5 | 6.0 | |
| **Glycerol** | 48.7 | | 56.7 | | 53.7 | 57.8 | 70.6 | |
| **Water** | 10.0 | | 10.0 | | 10.0 | 9.1 | 10.0 | |
| **Total** | 100.0 | | 100.0 | | 100.0 | 100.0 | 100.0 | |
| **Particle size of emulsified particles (µm) At preparation** | 0.40 | 0.27 | 0.20 | 0.17 | 0.07 | 0.08 | 0.06 | 0.06 |
| **Particle size of emulsified particles (µm) After refrigerated storage for 2 months** | 0.36 | 0.29 | 0.20 | 0.18 | 0.11 | 0.10 | 0.09 | 0.10 |

3. Evaluation of flavor characteristics

The flavor characteristics were evaluated using the sample prepared in 1. above. Specific procedures of the evaluation were as follows.

RebA was dissolved in pure water to prepare an aqueous solution having a RebA concentration of 467 ppm, which was used as a control. Next, pure water was added to each sample prepared in 1. above to prepare an aqueous solution having a RebA concentration of 467 ppm. Flavor evaluation of each aqueous solution was performed by seven well-trained sensory panelists by 0.5 points taking the control as 3 points. The average values were calculated, and they were ranked. The flavor was evaluated by sweetness intensity, sweetness lingering, and bitterness intensity. Those having the same score were set in the same rank. The ranks are shown in each evaluation item column of the table below. The sum of ranks is shown in the column of total of the table below.

Table 6

| | 70A | 200A | 206A | 207A |
|---|---|---|---|---|
| Sweetness intensity | 2 | 3 | 1 | 3 |
| Lingering | 3 | 1 | 2 | 1 |
| Bitterness | 1 | 4 | 2 | 3 |
| total | 6 | 8 | 5 | 7 |
| | 70B | 200B | 206B | 207B |
| Sweetness intensity | 4 | 2 | 3 | 1 |
| Lingering | 3 | 2 | 1 | 2 |
| Bitterness | 4 | 2 | 1 | 3 |
| total | 11 | 6 | 5 | 6 |

4. Evaluation of various characteristics after storage

Emulsion stability, flavor characteristics, and vibration stability after storage were evaluated using the sample prepared in 1. above. Specific procedures of the evaluation were as follows.

To 16990.3 mg of Sample SA-70A, 3,483 ml of pure water was added to obtain evaluation solution 70A. Evaluation solution 206A was also prepared in the same manner except that Sample SA-206A was used instead of Sample SA-70A. The composition of each evaluation solution was as follows.

Table 7

| | 70A | 206A |
|---|---|---|
| Sample amount (mg) | 16,990.3 | 16,990.3 |
| RebA ratio in sample (%) | 10.3 | 10.3 |
| RebA equivalent (mg) | 1,750.0 | 1,750.0 |
| Pure water addition amount (ml) | 3,483.0 | 3,483.0 |
| Total amount (ml) | 3,500 | 3,500 |
| RebA concentration (ppm) | 500.0 | 500.0 |

The obtained evaluation solution 70A and evaluation solution 206A were allowed to stand in a refrigerator having the inside temperature of 5°C for 4 weeks. Another evaluation solution 70A and another evaluation solution 206A were allowed to stand in a thermostatic cabinet at 55°C for 4 weeks.

In addition, evaluation solution 70A and evaluation solution 206A after adjusted their pH to 2.5 using citric acid

anhydride were allowed to stand in a refrigerator having the inside temperature of 5°C for 4 weeks. Another evaluation solution 70A and another evaluation solution 206A after adjusted their pH to 2.5 using citric acid anhydride were allowed to stand in a thermostatic cabinet at 55°C for 4 weeks.

4-1. Emulsion stability after storage

The appearance of liquid surface of the evaluation solutions after 4 weeks of standing was observed. The results are shown in the table below.

Table 8

| | | Standing temperature | |
|---|---|---|---|
| | | 5°C | 55°C |
| Without pH adjustment | 70A | No oil float | No oil float |
| | 206A | No oil float | No oil float |
| pH 2.5 | 70A | Oil float | Oil float |
| | 206A | No oil float | No oil float |

4-2. Effect of storage temperature on flavor characteristics

The effect of the temperature of standing on the flavor was examined. Specific procedures were as follows.

Evaluation solution 70A with a temperature of standing of 5°C and without pH adjustment was used as a control. The flavor of evaluation solution 70A with a temperature of standing of 55°C and without pH adjustment was evaluated by 0.5 points taking the evaluation of the control as 5 points. The panelists were four well-trained sensory panelists. The average value of the evaluations of the panelists was calculated.

In the same way, evaluation solution 206A with a temperature of standing of 5°C and without pH adjustment was used as a control, and the flavor of evaluation solution 206A with a temperature of standing of 5°C and without pH adjustment was evaluated by 0.5 points.

In the same way, evaluation solution 70A of pH 2.5 with a temperature of standing of 5°C was used as a control, and the flavor of evaluation solution 70A of pH 2.5 with a temperature of standing of 55°C was evaluated by 0.5 points.

In the same way, evaluation solution 260A of pH 2.5 with a temperature of standing of 5°C was used as a control, and the flavor of evaluation solution 260A of pH 2.5 with a temperature of standing of 55°C was evaluated by 0.5 points.

The results are shown in Fig. 19. In addition, comments of the panelists are described below.

70A, without pH adjustment;
Mild taste with no sting of bitterness, milky and slightly crisp. Milder and less bitter. Increased milkiness. Easier to drink. Slightly less freshness, but no critical deterioration.
260A, without pH adjustment;
Mild taste with no sting of bitterness, milky and slightly crisp. Milder and less bitter. Increased milkiness. No change in sweetness. No change. Slightly less freshness, but no critical deterioration.
70A, pH 2.5
Suppressed sourness. Increased sourness. Taste with some spikes. Milder sourness. Good. Slightly milder and less spikes of sourness.
206A, pH 2.5;
Suppressed sourness. Increased sourness. But easier to drink. No change. Slightly less bitterness. Good. Slightly milder and less spikes of sourness.

4-3. Vibration acceleration test

For evaluation solutions after 4 week-standing at 5°C, absorbance at a wavelength of 680 nm was measured

with a spectrophotometer (UV1800 UV spectrophotometer, manufactured by SHIMADZU CORPORATION).

The evaluation solutions were then centrifuged at 3000 rpm for 30 minutes, and absorbance at a wavelength of 680 nm was measured for the evaluation solutions after centrifugation. The difference in the absorbance between before and after centrifugation Δabs was calculated. Those having Δabs of 0.02 or less were evaluated as "good" as having excellent emulsion stability during vibration.

The results are shown in Fig. 20.

5. Degree of reduction in sweetness intensity

The degree of reduction in sweetness intensity due to emulsification was evaluated using Sample SA-70A and Sample SA-206A prepared in 1. above. Specific procedures for evaluation were as follows.

RebA was dissolved in pure water to prepare a 467 ppm aqueous solution, which was used as a control. Next, each sample prepared in 1. was added to pure water to prepare an aqueous solution having a RebA concentration of 467 ppm. Then, four well-trained sensory panelists determined which sweetness intensity of Brix 2, 5, 8, 11, and 14 the sweetness intensity of each aqueous solution was close to. The average value of the evaluation results of the panelists was calculated. The results are shown in Fig. 21.

<Experimental Example 34>

In the same manner as in "4. Evaluation of various characteristics after storage" in Experimental Example 33, Evaluation solution 70A and Evaluation solution 206A were obtained.

Evaluation solution 70A and Evaluation solution 206A were diluted with DMSO-containing PBS to prepare diluted solutions having RebA concentrations shown in the addition concentration column of the table below.

Another Evaluation solution 70A and another Evaluation solution 206A were allowed to stand in a thermostatic cabinet at 55°C for 4 weeks. Evaluation solution 70A and Evaluation solution 206A after standing were diluted with DMSO-containing PBS to prepare diluted solutions having RebA concentrations shown in the addition concentration column of the table below.

As a non-stimulant control (negative comparison), 1% DMSO-containing solution was prepared. In addition, as a positive control, a solution was prepared by diluting PMA (manufactured by FUJIFILM Wako Pure Chemical Corporation, model number: 162-23591) with 1% DMSO to have the concentration shown in the addition concentration column of the table below.

Table 9

| Addition amount and final concentration of each test substance | | | | | | |
|---|---|---|---|---|---|---|
| **#** | | **Addition concentration (mM)** | **Addition amount (μL)** | **Medium (μL)** | **Total (μL)** | **Final conc. (mM)** |
| 1 | 1% DMSO-containing solution | - | 10 | 90 | 100 | - |
| 2 | PMA (1%DMSO) | 0.01 | 10 | 90 | 100 | 0.001 |
| 3 | | 1 | 10 | 90 | 100 | 0.100 |
| 4 | 70A | 0.25 | 10 | 90 | 100 | 0.025 |
| 5 | | 0.5 | 10 | 90 | 100 | 0.050 |
| 6 | | 1 | 10 | 90 | 100 | 0.100 |
| 7 | | 2.5 | 10 | 90 | 100 | 0.250 |
| 8 | | 5 | 10 | 90 | 100 | 0.500 |
| 9 | | 10 | 10 | 90 | 100 | 1.000 |

(continued)

| Addition amount and final concentration of each test substance | | | | | | |
|---|---|---|---|---|---|---|
| # | | Addition concentration (mM) | Addition amount (μL) | Medium (μL) | Total (μL) | Final conc. (mM) |
| 10 | 70A4W | 0.25 | 10 | 90 | 100 | 0.025 |
| 11 | | 0.5 | 10 | 90 | 100 | 0.050 |
| 12 | | 1 | 10 | 90 | 100 | 0.100 |
| 13 | | 2.5 | 10 | 90 | 100 | 0.250 |
| 14 | | 5 | 10 | 90 | 100 | 0.500 |
| 15 | | 10 | 10 | 90 | 100 | 1.000 |
| 16 | 206A | 0.25 | 10 | 90 | 100 | 0.025 |
| 17 | | 0.5 | 10 | 90 | 100 | 0.050 |
| 18 | | 1 | 10 | 90 | 100 | 0.100 |
| 19 | | 2.5 | 10 | 90 | 100 | 0.250 |
| 20 | | 5 | 10 | 90 | 100 | 0.500 |
| 21 | | 10 | 10 | 90 | 100 | 1.000 |
| 22 | 206A4W | 0.25 | 10 | 90 | 100 | 0.025 |
| 23 | | 0.5 | 10 | 90 | 100 | 0.050 |
| 24 | | 1 | 10 | 90 | 100 | 0.100 |
| 25 | | 2.5 | 10 | 90 | 100 | 0.250 |
| 26 | | 5 | 10 | 90 | 100 | 0.500 |
| 27 | | 10 | 10 | 90 | 100 | 1.000 |

Under the conditions shown in the table above, diluted solution Nos. 1 to 27 were added to a medium, and cells were cultured in the obtained medium. Specific procedures were as follows.

H716 cells were cultured in RPMI 1640 (manufactured by Thermo Fisher Scientific Inc., model number 61870-036) (10% FBS (manufactured by Gibco, model number SH102770), 1 mM sodium pyruvate) in an incubator at 37°C, 5%$CO_2$. H716 cells were seeded in 96-well plates at 2 x $10^5$ cells/90 μL/well (for dead cell evaluation) and in 48-well plates at 4 x $10^5$ cells/180 μL/well (for morphological observation). The content of FBS in the medium when the cells were seeded in the plate was 0.5%. After 24 hours have passed from the cell seeding, diluted solution Nos. 1 to 27 were added to the wells at an amount of 10 μL/well. The final concentrations of RebA in the medium after the addition of the diluted solutions are shown in the "Final conc. (mM)" column of the above table.

The cell viability was examined after 2 hours and 24 hours from the addition of the diluted solutions. There were no problems with any of the samples.

After 2 hours and 24 hours from the addition of diluted solutions, the 96-well plate was centrifuged at 300 x g for 5 minutes, and the culture supernatant was collected.

GLP-1 in the collected culture supernatant was quantified with Human GLP-1 (7-36 amide) Immunoassay kit (manufactured by PerkinElmer, Inc., model number: AL359C). For quantitative values, a value that was beyond the range of the calibration curve and caused an error was excluded. Furthermore, a box plot was created with a statistical analysis software R, and when a value less than the first quartile or greater than the third quartile was at or beyond the 1.5 quartile range, the value was excluded as an outlier.

The results are shown in Fig. 22.

## Claims

1. A composition for promoting GLP-1 secretion, comprising an emulsified particle comprising an amphiphilic GLP-1 secretion-promoting component and an emulsifier, wherein

the emulsifier is an oil-in-water emulsifier; and
the GLP-1 secretion-promoting component is incorporated into the emulsified particle.

2. The composition according to claim 1, wherein the GLP-1 secretion-promoting component is rebaudioside A.

3. The composition according to claim 1 or 2, wherein an HLB value of the emulsifier is 7 to 18.

4. The composition according to any one of claims 1 to 3, wherein the emulsifier comprises at least one selected from the group consisting of a polyglycerol fatty acid ester, a propylene glycol fatty acid ester, an enzyme-treated lecithin, a sucrose fatty acid ester, and an organic acid monoglyceride.

5. The composition according to any one of the claims 1 to 4, wherein a hydrophilic group of the emulsifier is derived from a sweetness component.

6. The composition according to any one of claims 1 to 5, wherein the emulsifier comprises at least one emulsifier selected from the group consisting of a polyglycerol fatty acid ester and a sucrose fatty acid ester.

7. The composition according to any one of claims 1 to 6, wherein a hydrophilic group of the emulsifier comprises a phosphate group.

8. The composition according to any one of claims 1 to 7, wherein a proportion of the emulsifier based on the GLP-1 secretion-promoting component is 0.02 to 50% by mass.

9. The composition according to any one of claims 1 to 8, wherein the composition comprises the emulsifier in a proportion of 0.1 to 5% by mass.

10. The composition according to any one of claims 1 to 9, wherein the composition comprises the GLP-1 secretion-promoting component in a proportion of 1 to 20% by mass.

11. The composition according to any one of claims 1 to 10, wherein the composition is for use in improving glycometabolism, in suppressing appetite, or in preventing or ameliorating diabetes or obesity.

12. A beverage comprising the composition according to any one of claims 1 to 11.

13. A food comprising the composition according to any one of claims 1 to 11.

14. A pharmaceutical composition comprising the composition according to any one of claims 1 to 11.

15. A method of producing an emulsified particle into which a GLP-1 secretion-promoting component is incorporated, comprising the steps of:

mixing an oil-in-water emulsifier, an amphiphilic GLP-1 secretion-promoting component, and an aqueous medium to prepare a mixture; and
adding a fat and oil to the mixture and stirring.

16. A composition comprising an emulsified particle comprising rebaudioside A and an oil-in-water emulsifier, wherein rebaudioside A is incorporated into the emulsified particle.

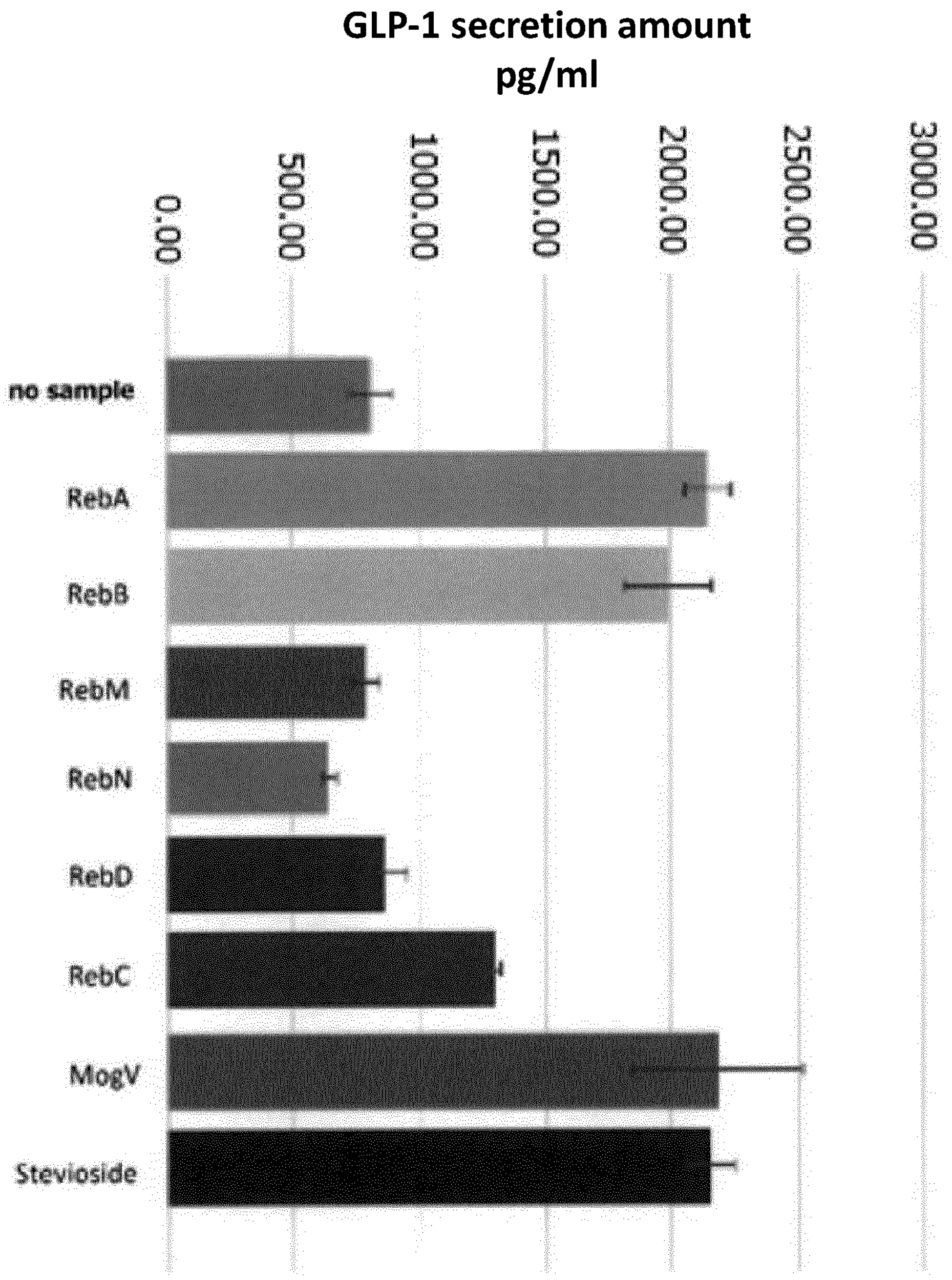
GLP-1 secretion amount
pg/ml
0.00
500.00
1000.00
1500.00
2000.00
2500.00
3000.00
no sample
RebA
RebB
RebM
RebN
RebD
RebC
MogV
Stevioside

FIG. 1

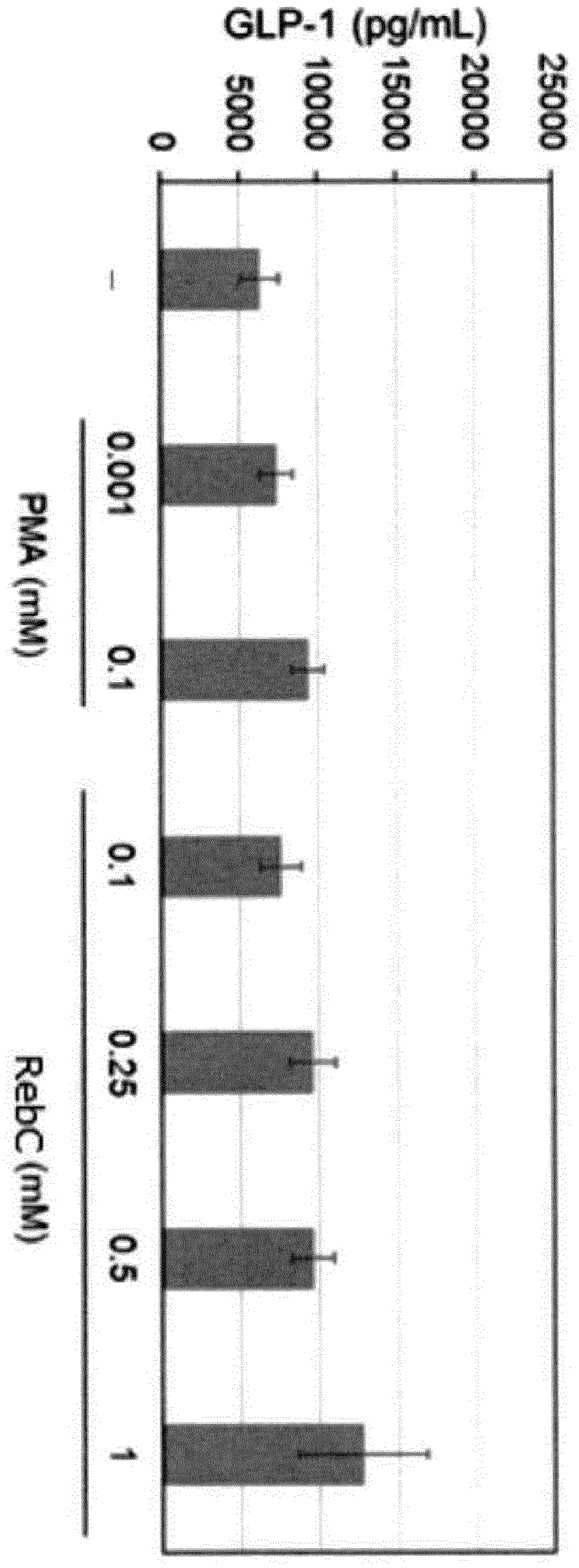
GLP-1 (pg/mL)
0
5000
10000
15000
20000
25000
–
0.001
0.1
PMA (mM)
0.1
0.25
0.5
1
RebC (mM)

FIG. 2

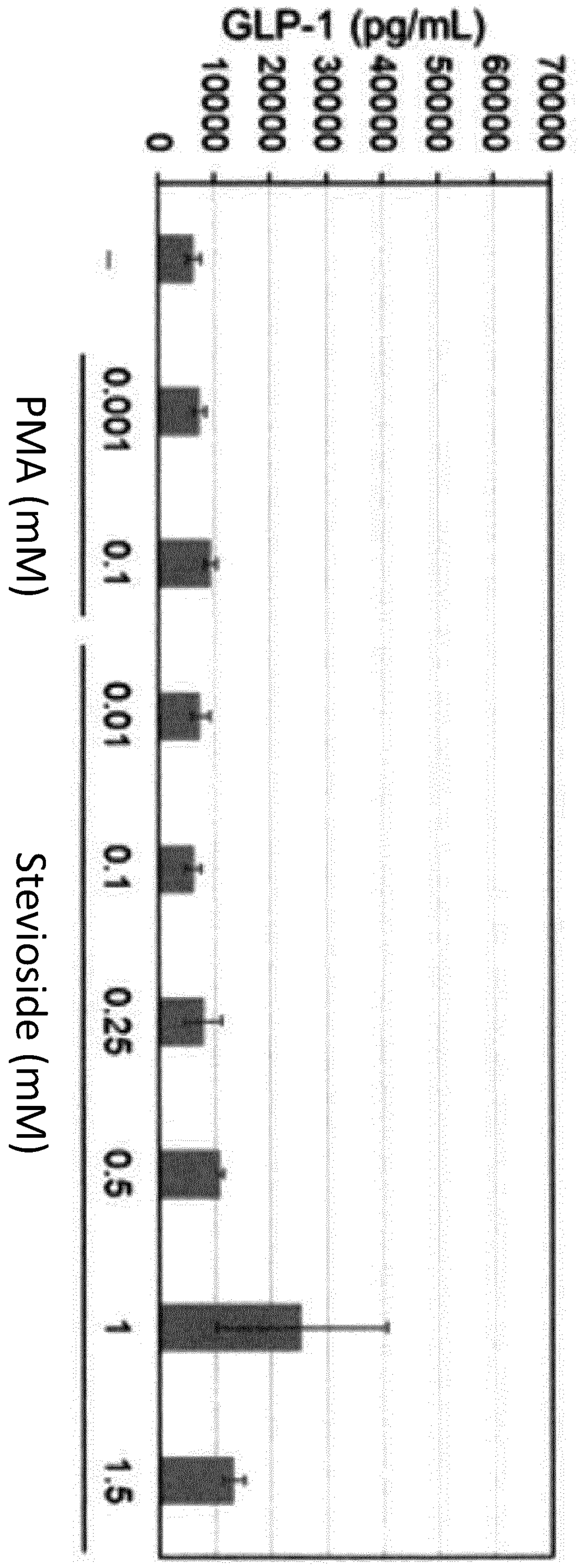
GLP-1 (pg/mL)
0
10000
20000
30000
40000
50000
60000
70000
-
0.001
0.1
PMA (mM)
0.01
0.1
0.25
0.5
1
1.5
Stevioside (mM)

FIG. 3

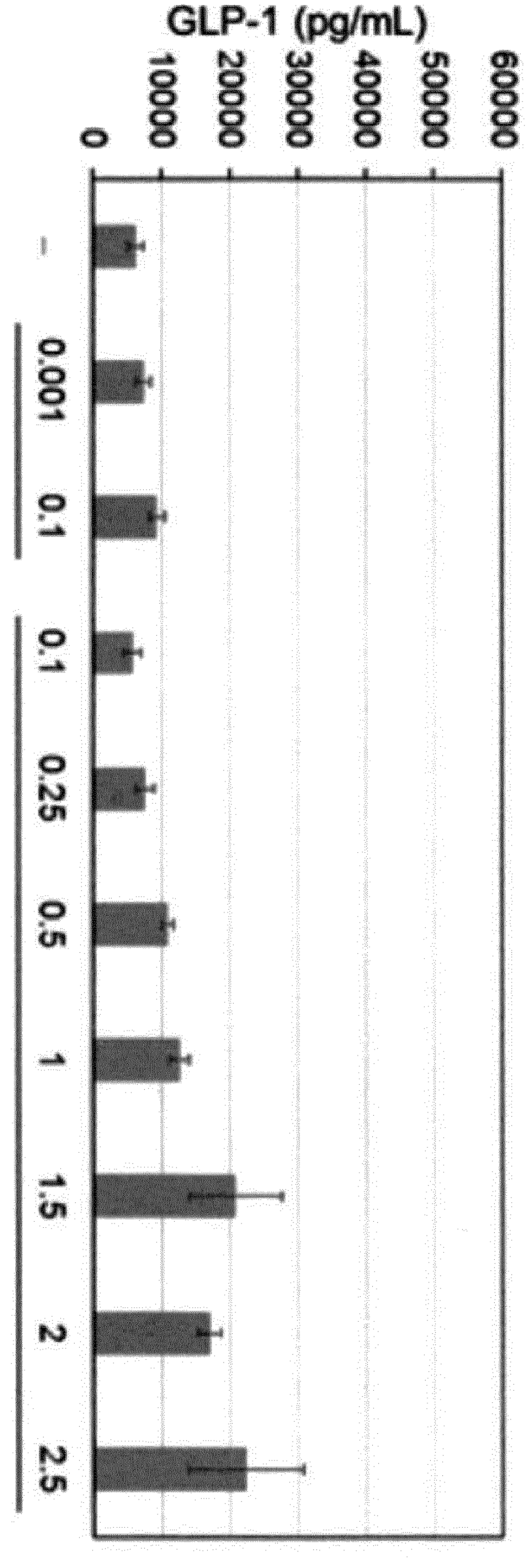
GLP-1 (pg/mL)
0
10000
20000
30000
40000
50000
60000
–
0.001
0.1
PMA (mM)
0.1
0.25
0.5
1
1.5
2
2.5
RebA (mM)

FIG. 4

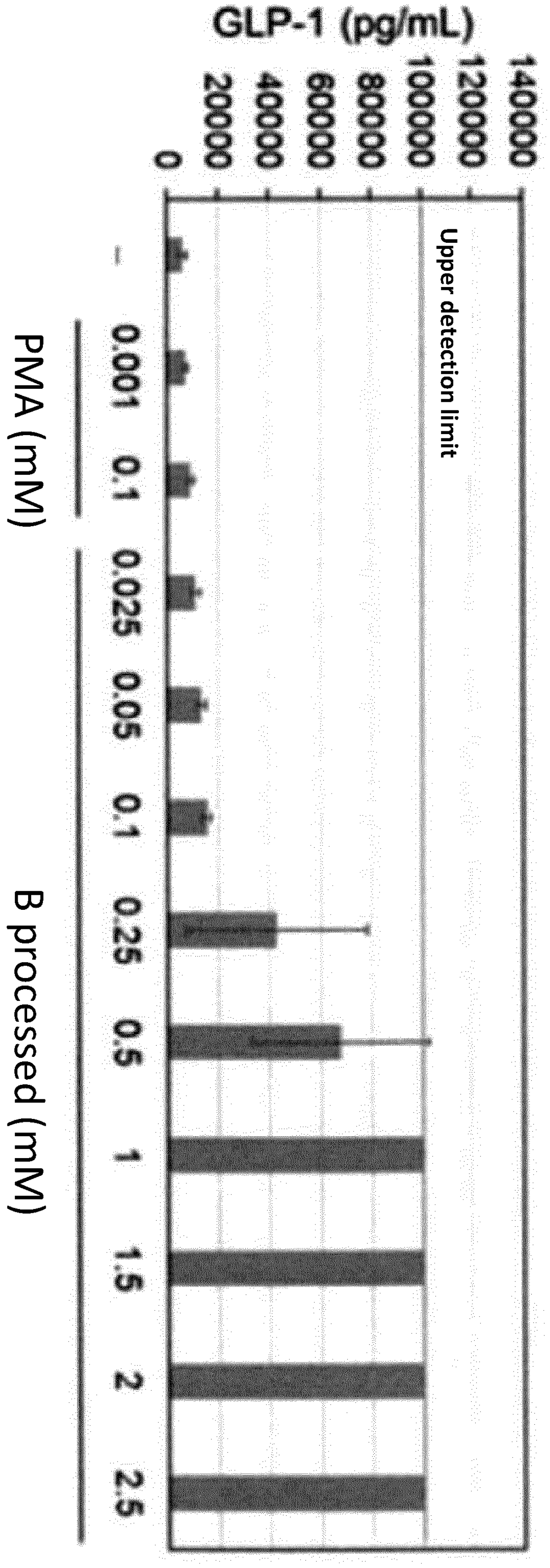
GLP-1 (pg/mL)
0
20000
40000
60000
80000
100000
120000
140000
Upper detection limit
–
0.001
0.1
0.025
0.05
0.1
0.25
0.5
1
1.5
2
2.5
PMA (mM)
B processed (mM)

FIG. 5

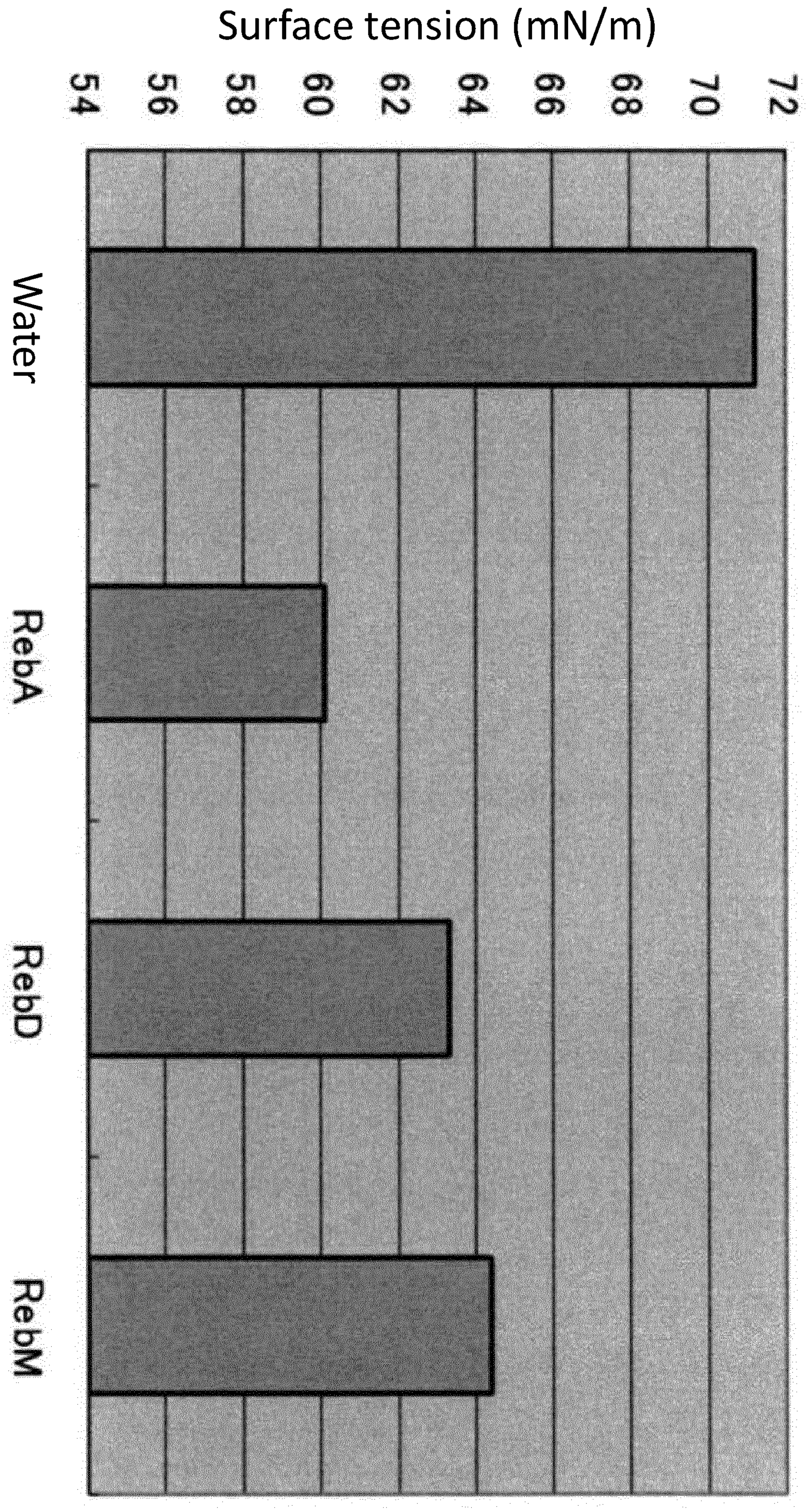
Surface tension (mN/m)
54
56
58
60
62
64
66
68
70
72
Water
RebA
RebD
RebM

FIG. 6

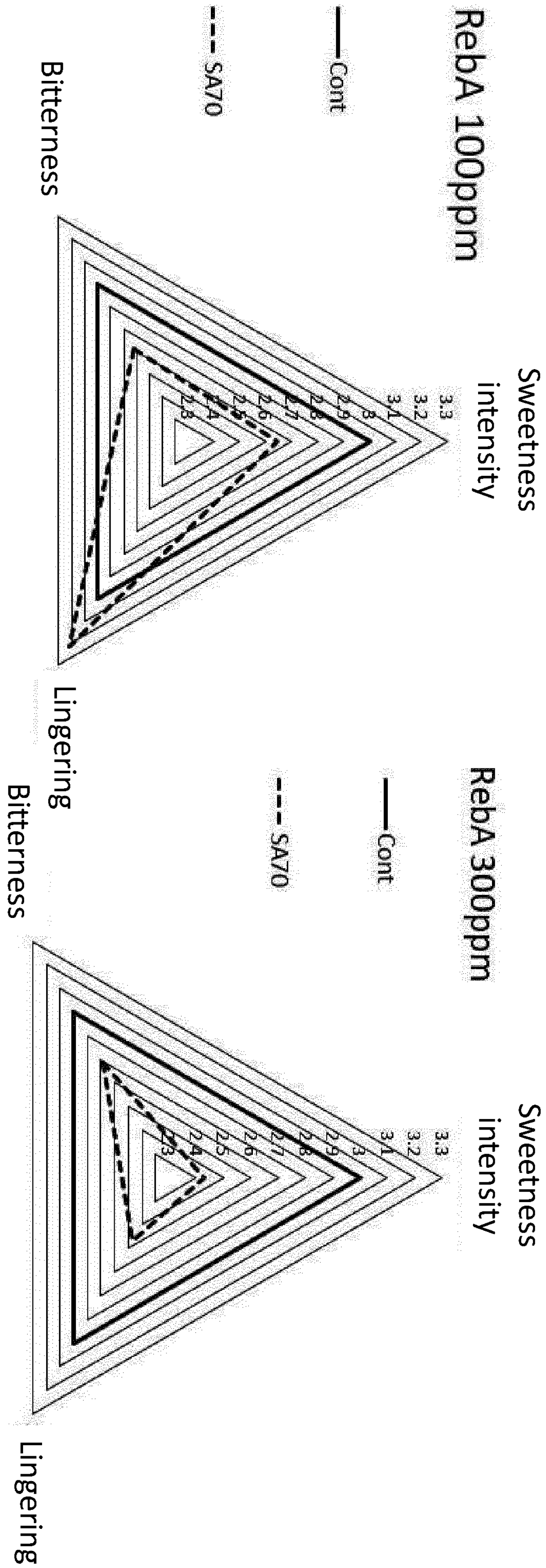
RebA 100ppm
Cont
SA70
Sweetness intensity
Bitterness
Lingering
3.3
3.2
3.1
3
2.9
2.8
2.7
2.6
2.5
2.4
2.3
RebA 300ppm
Cont
SA70
Sweetness intensity
Bitterness
Lingering

FIG. 7

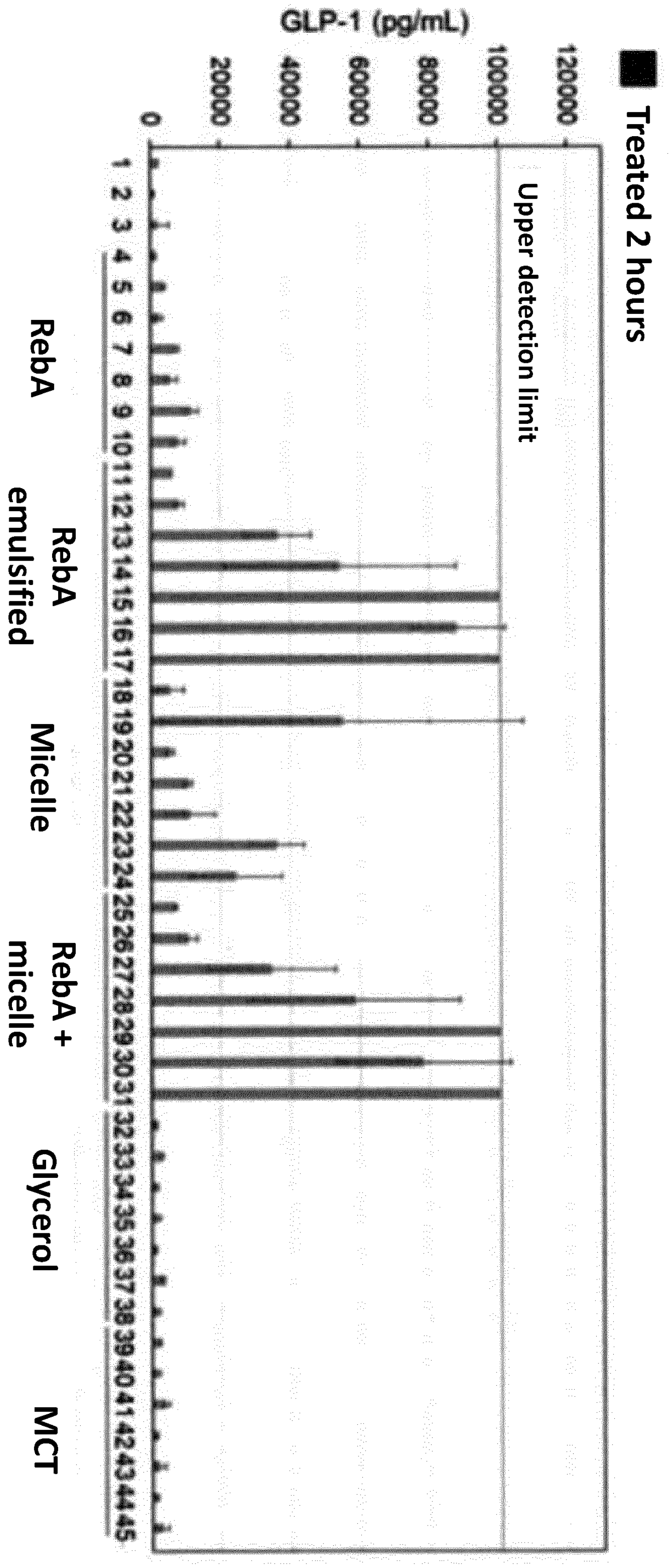
Treated 2 hours
GLP-1 (pg/mL)
0
20000
40000
60000
80000
100000
120000
Upper detection limit
1 2 3 4 5 6 7 8 9 10 11 12 13 14 15 16 17 18 19 20 21 22 23 24 25 26 27 28 29 30 31 32 33 34 35 36 37 38 39 40 41 42 43 44 45
RebA
RebA emulsified
Micelle
RebA + micelle
Glycerol
MCT

FIG. 8

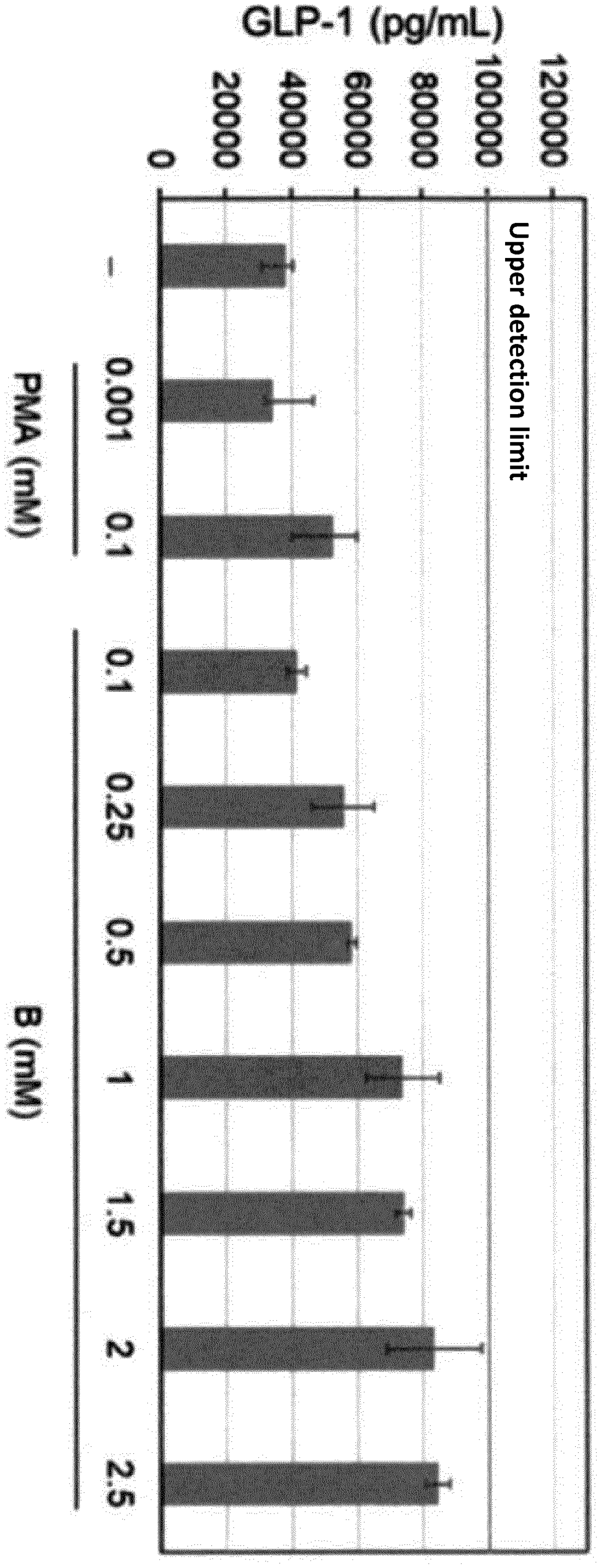
GLP-1 (pg/mL)
0
20000
40000
60000
80000
100000
120000
Upper detection limit
–
0.001
0.1
PMA (mM)
0.1
0.25
0.5
1
1.5
2
2.5
B (mM)

FIG. 9

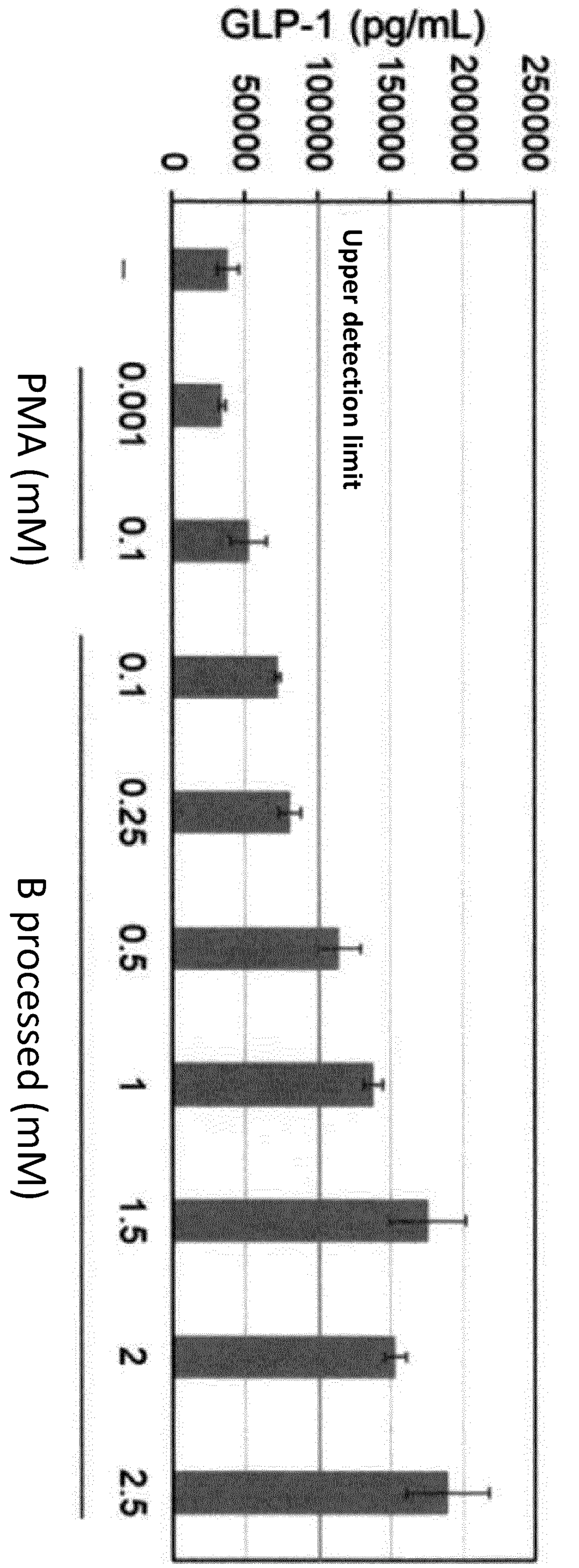
GLP-1 (pg/mL)
250000
200000
150000
100000
50000
0
Upper detection limit
–
0.001
0.1
0.1
0.25
0.5
1
1.5
2
2.5
PMA (mM)
B processed (mM)

FIG. 10

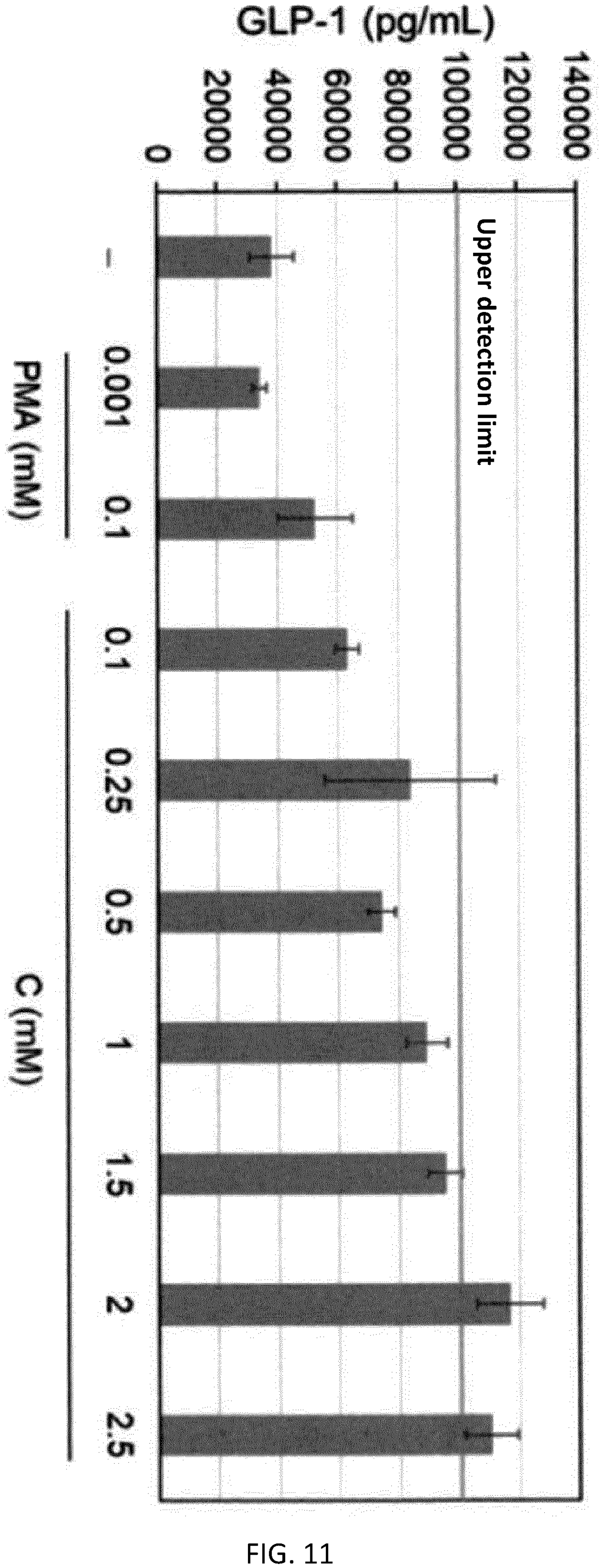
GLP-1 (pg/mL)
0
20000
40000
60000
80000
100000
120000
140000
Upper detection limit
-
0.001
0.1
PMA (mM)
0.1
0.25
0.5
1
1.5
2
2.5
C (mM)

FIG. 11

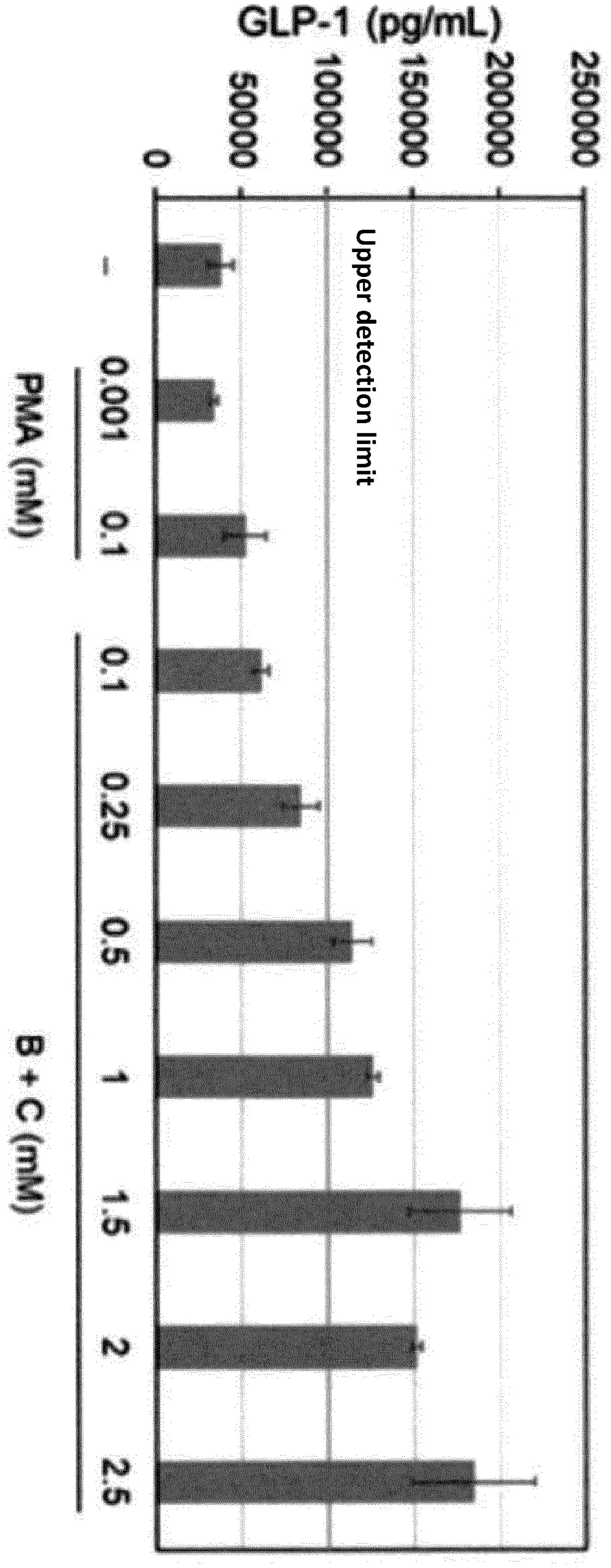
GLP-1 (pg/mL)
0
50000
100000
150000
200000
250000
Upper detection limit
–
0.001
0.1
PMA (mM)
0.1
0.25
0.5
1
1.5
2
2.5
B + C (mM)

FIG. 12

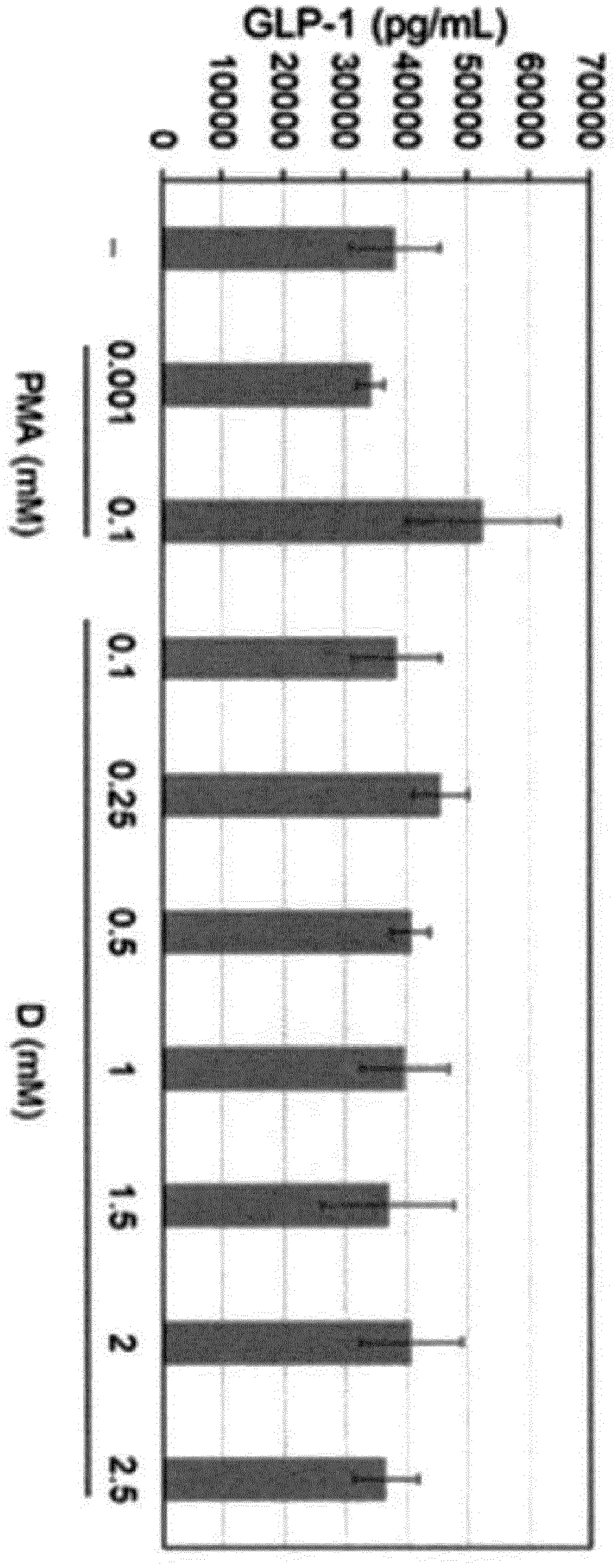
GLP-1 (pg/mL)
0
10000
20000
30000
40000
50000
60000
70000
-
0.001
0.1
PMA (mM)
0.1
0.25
0.5
1
1.5
2
2.5
D (mM)

FIG. 13

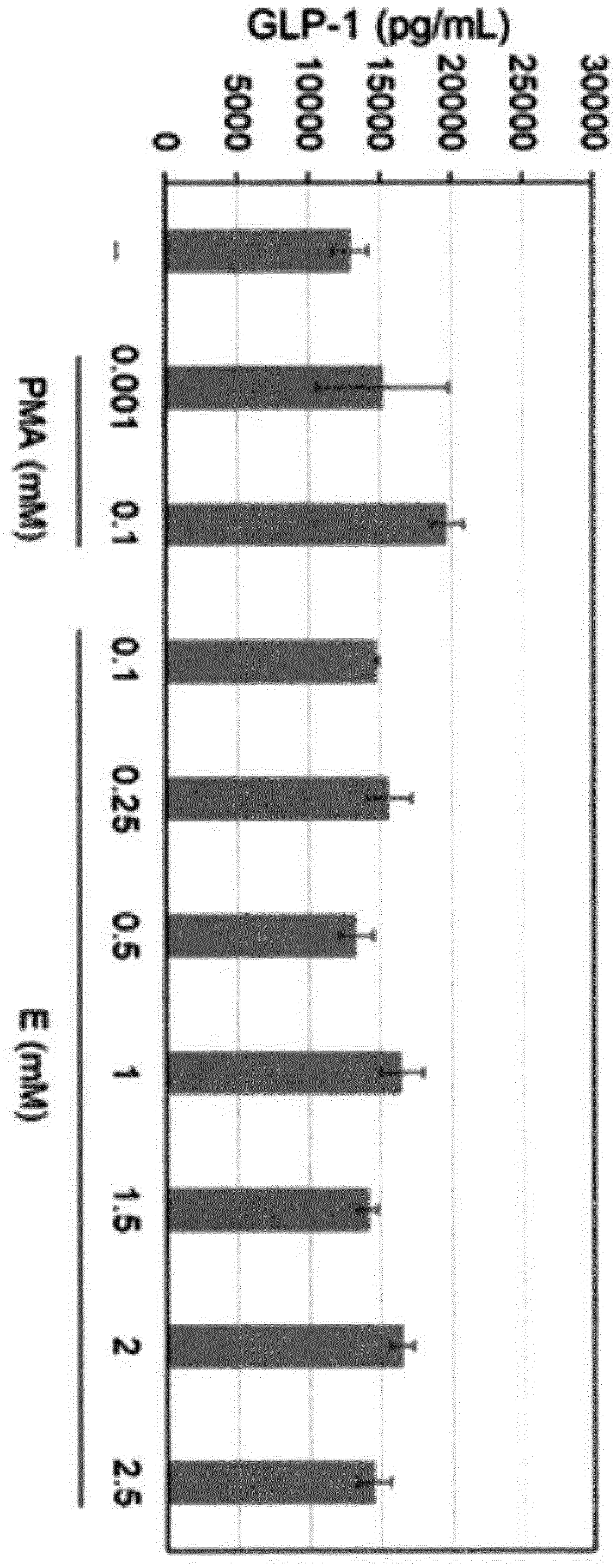
GLP-1 (pg/mL)
0
5000
10000
15000
20000
25000
30000
-
0.001
0.1
PMA (mM)
0.1
0.25
0.5
1
1.5
2
2.5
E (mM)

FIG. 14

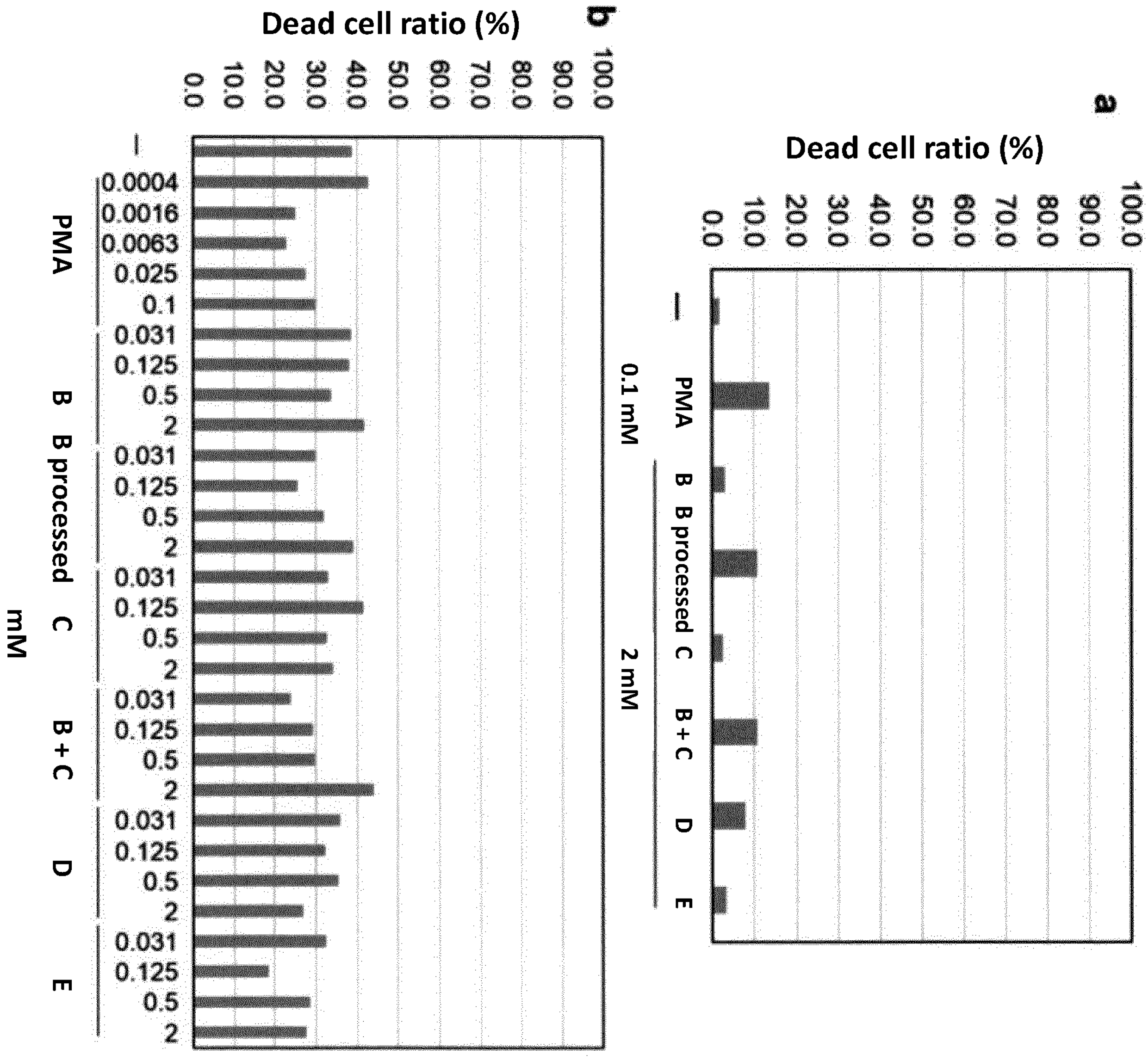
a
Dead cell ratio (%)
0.0
10.0
20.0
30.0
40.0
50.0
60.0
70.0
80.0
90.0
100.0
—
PMA
B
B processed
C
B + C
D
E
0.1 mM
2 mM
b
Dead cell ratio (%)
—
0.0004
0.0016
0.0063
0.025
0.1
0.031
0.125
0.5
2
PMA
B
B processed
C
B + C
D
E
mM

FIG. 15

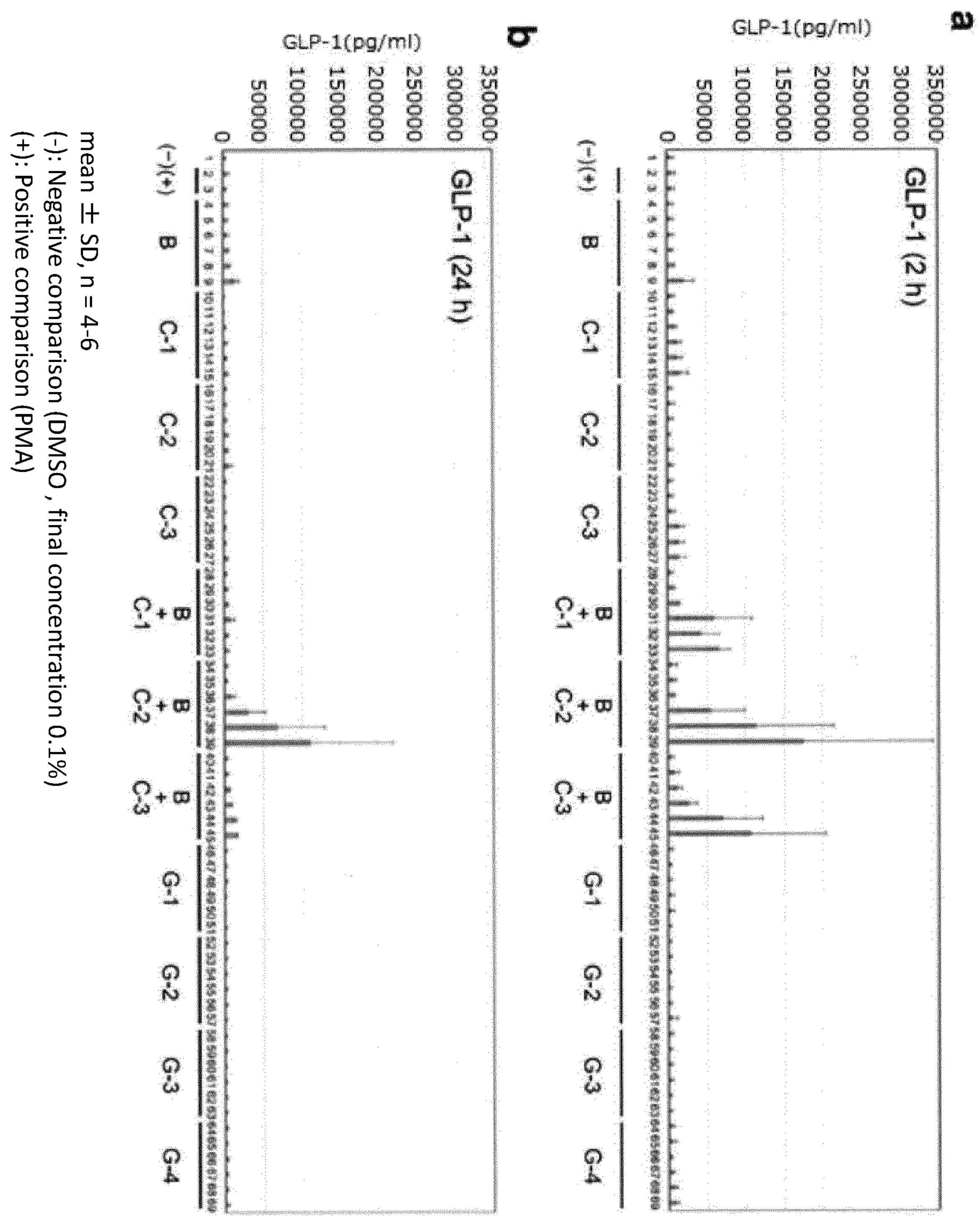
a
GLP-1 (2 h)
GLP-1(pg/ml)
0
50000
100000
150000
200000
250000
300000
350000
(-)(+)
B
C-1
C-2
C-3
B + C-1
B + C-2
B + C-3
G-1
G-2
G-3
G-4
b
GLP-1 (24 h)
GLP-1(pg/ml)
0
50000
100000
150000
200000
250000
300000
350000
(-)(+)
B
C-1
C-2
C-3
B + C-1
B + C-2
B + C-3
G-1
G-2
G-3
G-4
mean ± SD, n = 4-6
(-): Negative comparison (DMSO , final concentration 0.1%)
(+): Positive comparison (PMA)

FIG. 16

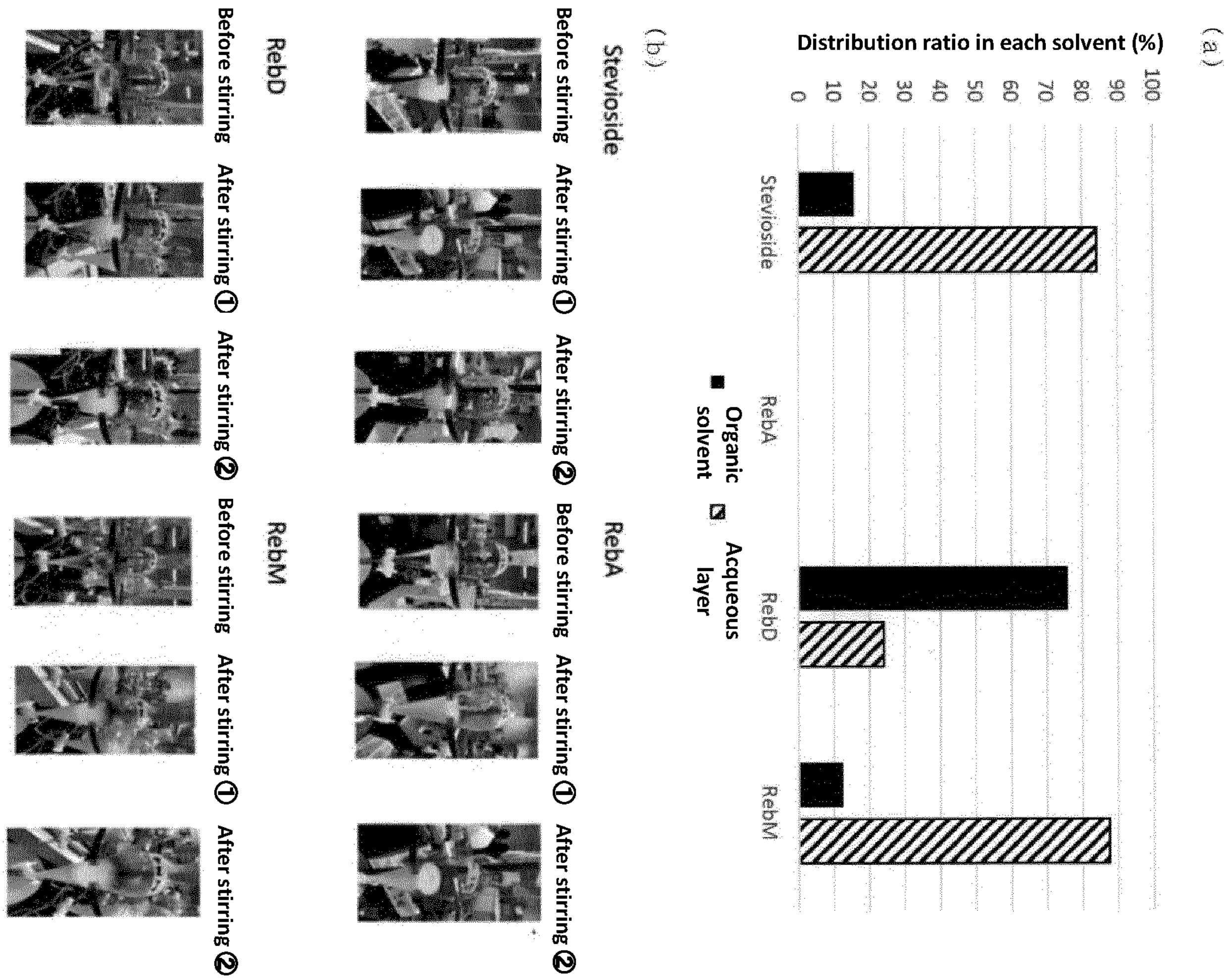
(a)
Distribution ratio in each solvent (%)
0
10
20
30
40
50
60
70
80
90
100
Stevioside
RebA
RebD
RebM
Organic solvent
Acqueous layer
(b)
Stevioside
Before stirring
After stirring ①
After stirring ②
RebA
Before stirring
After stirring ①
After stirring ②
RebD
Before stirring
After stirring ①
After stirring ②
RebM
Before stirring
After stirring ①
After stirring ②

FIG. 17

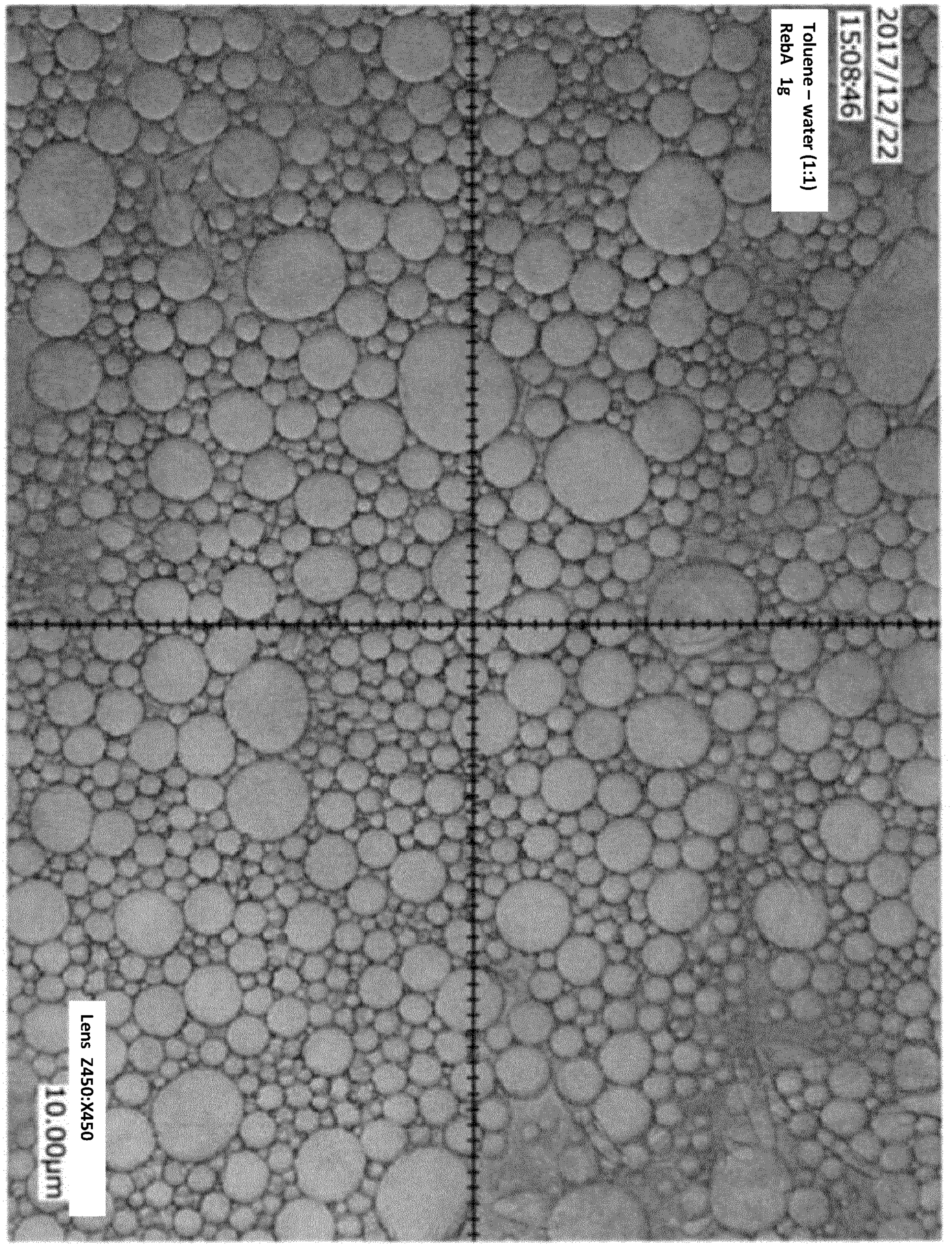
2017/12/22
15:08:46
Toluene – water (1:1)
RebA 1g
Lens Z450:X450
10.00μm

FIG. 18

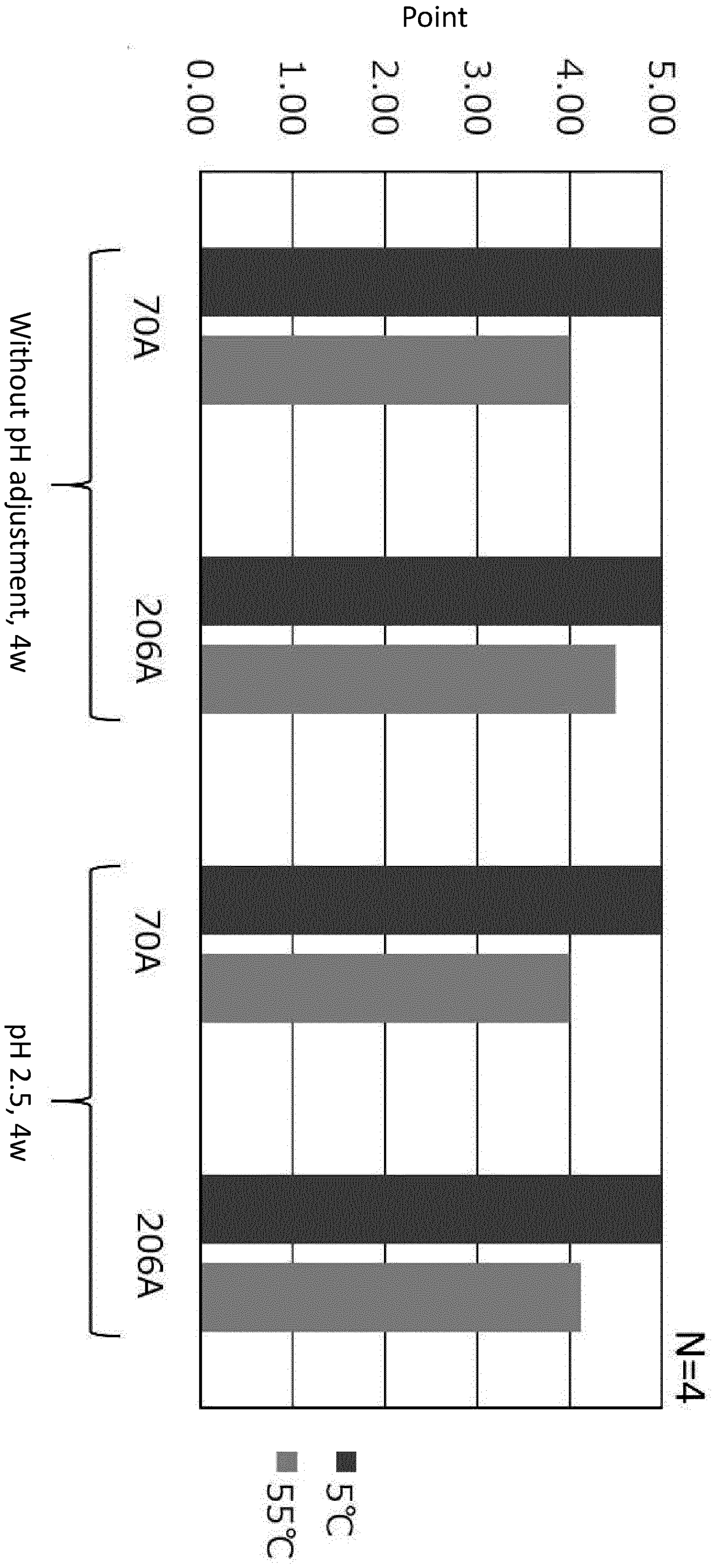
Point
0.00
1.00
2.00
3.00
4.00
5.00
70A
206A
70A
206A
Without pH adjustment, 4w
pH 2.5, 4w
N=4
5°C
55°C

FIG. 19

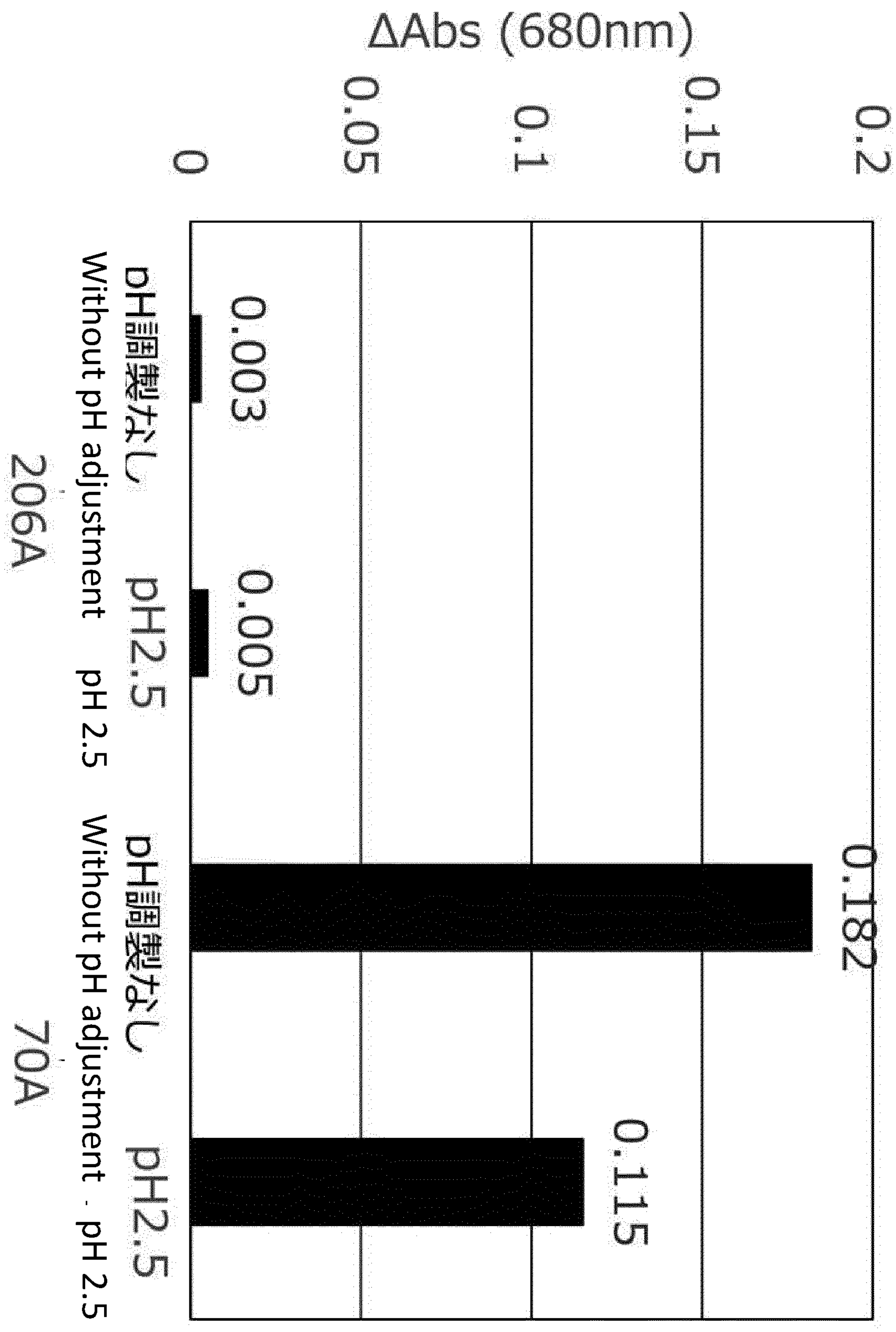
ΔAbs (680nm)
0
0.05
0.1
0.15
0.2
0.003
0.005
0.182
0.115
pH調製なし
Without pH adjustment
pH2.5
pH 2.5
206A
pH調製なし
Without pH adjustment
pH2.5
pH 2.5
70A

FIG. 20

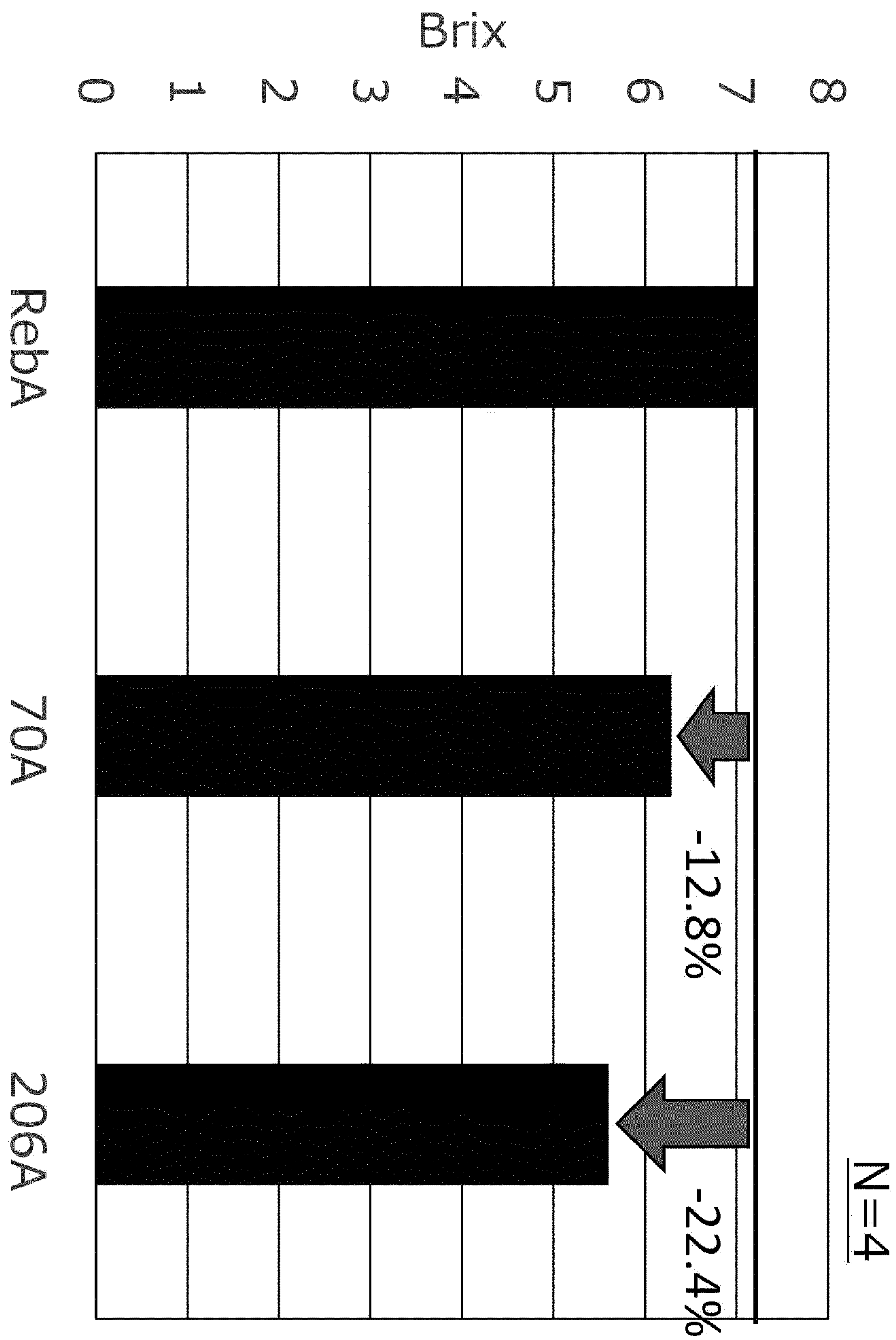


FIG. 21

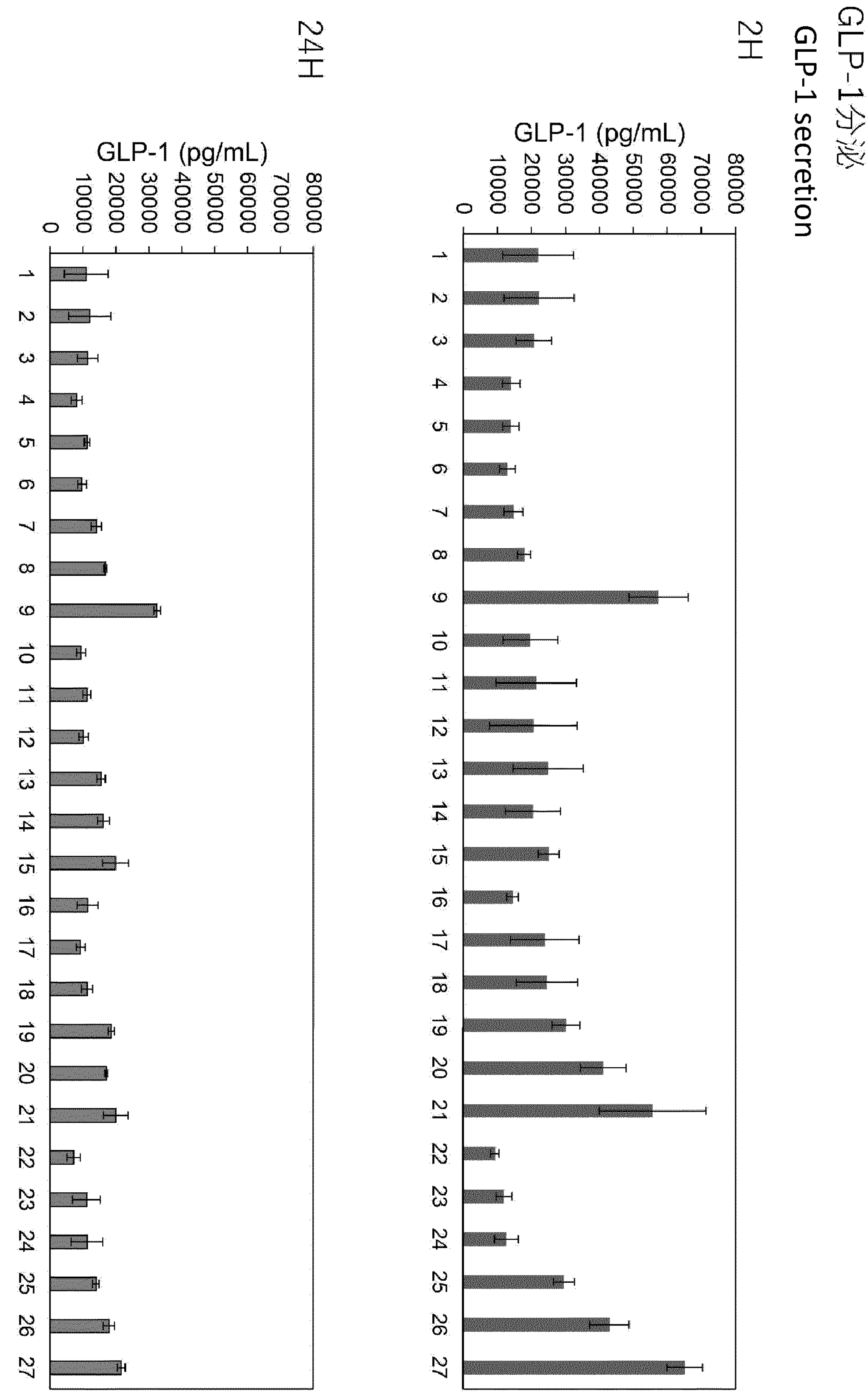
GLP-1分泌
GLP-1 secretion
2H
GLP-1 (pg/mL)
0
10000
20000
30000
40000
50000
60000
70000
80000
1 2 3 4 5 6 7 8 9 10 11 12 13 14 15 16 17 18 19 20 21 22 23 24 25 26 27
24H
GLP-1 (pg/mL)
0
10000
20000
30000
40000
50000
60000
70000
80000
1 2 3 4 5 6 7 8 9 10 11 12 13 14 15 16 17 18 19 20 21 22 23 24 25 26 27

FIG. 22

## INTERNATIONAL SEARCH REPORT

International application No. PCT/JP2020/017399

A. CLASSIFICATION OF SUBJECT MATTER
A61K 45/00(2006.01)i; A61P 3/04(2006.01)i; A61P 3/10(2006.01)i; A61P 43/00(2006.01)i; A61K 9/10(2006.01)i; A23L 33/10(2016.01)i; A61K 47/14(2006.01)i; A61K 47/24(2006.01)i; A61K 47/26(2006.01)i; A61K 47/34(2017.01)i; A23L 2/52(2006.01)i; A61K 31/704(2006.01)i
FI: A23L33/10; A61K45/00; A61P3/04; A61P3/10; A61K9/10; A61K47/34; A61K47/24; A61K47/26; A61K47/14; A61P43/00 105; A23L2/00 F; A61K31/704

According to International Patent Classification (IPC) or to both national classification and IPC

B. FIELDS SEARCHED

Minimum documentation searched (classification system followed by classification symbols)
A61K45/00; A61P3/04; A61P3/10; A61P43/00; A61K9/10; A23L33/10; A61K47/14; A61K47/24; A61K47/26; A61K47/34; A23L2/52; A61K31/704

Documentation searched other than minimum documentation to the extent that such documents are included in the fields searched
JSTPlus/JMEDPlus/JST7580 (JDreamIII); CAplus/WPIDS/MEDLINE/EMBASE/BIOSIS/FSTA (STN)

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | US 2015/0237901 A1 (JAMES AND CAROL MAY FAMILY, LLLP.) 27.08.2015 (2015-08-27) claims, paragraphs [0003]-[0004], [0006], examples | 1-3, 7-14, 16<br>1-3, 7-11 |
| Y<br><br>A | WO 2017/018404 A1 (SUNTORY HOLDINGS LIMITED) 02.02.2017 (2017-02-02) paragraphs [0115]-[0119]<br>paragraphs [0057]-[0061] | 1-3, 7-11<br><br>4-6, 12-16 |
| Y | RIPKEN, D. et al., "Steviol Glycoside Rebaudioside A Induces Glucagon-like Peptide-1 and Peptide YY Release in a Porcine ex Vivo Intestinal Model", J. Agric. Food Chem., 2014, vol. 62, pp. 8365-8370 abstract, table 1 | 1-3, 7-11 |

☒ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 July 2020 (14.07.2020) | 28 July 2020 (28.07.2020) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2020/017399

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 1-176443 A (NONOGAWA SHOJI KK) 12.07.1989 (1989-07-12) claim 8, page 2, lower right column, lines 8-12, page 3, lower left column, line 6 to lower right column, line 11, example 12, table 8 | 12-16<br>1-11 |
| X | JP 59-139309 A (NONOGAWA SHOJI KK) 10.08.1984 (1984-08-10) claims, page 2, upper left column, lines 16-21, page 2, upper right column, line 9 to lower right column, line 16, examples 1-5 | 16 |
| A | JP 2017-504343 A (CARGILL, INCORPORATED) 09.02.2017 (2017-02-09) | 1-16 |
| A | US 2012/0196019 A1 (SHI, Jingang et al.) 02.08.2012 (2012-08-02) | 1-16 |

Form PCT/ISA/210 (continuation of second sheet) (January 2015)

**INTERNATIONAL SEARCH REPORT**
Information on patent family members

International application No.
PCT/JP2020/017399

| Patent Documents referred in the Report | Publication Date | Patent Family | Publication Date |
|---|---|---|---|
| US 2015/0237901 A1 | 27 Aug. 2015 | WO 2015/127297 A1<br>claims, paragraphs [0003]-[0004], [0006], examples | |
| WO 2017/018404 A1 | 02 Feb. 2017 | US 2018/0214508 A1<br>paragraphs [0191]-[0194]<br>EP 3329928 A1<br>CN 107847545 A<br>KR 10-2018-0030915 A<br>TW 201717988 A | |
| JP 1-176443 A | 12 Jul. 1989 | (Family: none) | |
| JP 59-139309 A | 10 Aug. 1984 | (Family: none) | |
| JP 2017-504343 A | 09 Feb. 2017 | US 2017/0006901 A1<br>WO 2015/116785 A1<br>EP 3099185 A1<br>AU 2015210982 A<br>CA 2937368 A<br>CN 106068083 A<br>MX 2016009566 A | |
| US 2012/0196019 A1 | 02 Aug. 2012 | WO 2012/104726 A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017159725 A
- WO 2017018404 A


### Non-patent literature cited in the description


- **TOSHIYUKI SUZUKI.** *J. Soc. Cosmet. Jpn.,* 2010, vol. 44 (2
- **YOUNG-SUN LEE et al.** Anti-Inflammatory Effects of GLP-1-Based Therapies beyond Glucose Control. *Mediators of Inflammation,* vol. 2016, 11